# EUROPEAN PATENT APPLICATION

(11) **EP 4 282 435 A1**
(43) Date of publication of application: **29.11.2023**
(21) Application number: 22174814.8
(22) Date of filing: 23.05.2022
(51) Int. Cl.: A61K 49/00, A61K 9/00, A61K 31/7036, A61K 31/7056, A61K 38/14, A61K 38/29, A61K 38/38, A61K 47/26, A61P 19/10, A61P 31/04

(54) **FORMULATIONS OF ACTIVE PHARMACEUTICAL INGREDIENTS AND EXCIPIENTS IN ICELLS VIA HYDROPHOBIC ION PAIRING**

(71) Applicant: Danmarks Tekniske Universitet, 2800 Kongens Lyngby (DK)
(72) Inventor: Andresen, Thomas Lars, 2800 Kongens Lyngby (DK); Henriksen, Jonas Rosager, 2800 Kongens Lyngby (DK); Hansen, Anders Elias, 2800 Kongens Lyngby (DK); Fuglsang-Madsen, Albert Juan, 2800 Kongens Lyngby (DK); Serrano Chávez, Elizabeth, 2800 Kongens Lyngby (DK)
(74) Representative: AWA Sweden AB

(57) **Abstract**

The present invention refers to a composition forming a hydrophobic ion par (HIP), comprising:
a. an imaging agent with zwitterionic, cationic or anionic charge;
b. a counterion being a hydrophobic or amphiphilic acid or base, or salt thereof;
c. a non-water soluble carbohydrate;
d. a non-aqueous solvent;

wherein at least 50% of the non-water soluble carbohydrates are carbohydrates selected from derivatives of disaccharides or trisaccharides selected from sucrose, lactose, maltose, trehalose, raffinose, or derivatives of disaccharides or trisaccharides, or mixtures thereof;
wherein the composition is a liquid before administration into the human or animal body and increases in viscosity by more than 10,000 centipoise (cP) after administration to transform into a ICell;
and wherein the hydrophobic or amphiphilic acid or base or salt thereof enhances the solubility of the imaging agent in the ICell by forming a hydrophobic ion-pair or multiple hydrophobic ion-pairs with the imaging agent.

## Description

### Field of the invention

Methods are presented for encapsulating water-soluble biologics, imaging agent, and small molecule active pharmaceutical ingredients (APIs) into hydrophobic sustained release carbohydrate ester depots by applying ion-pairing with hydrophobic counterions to produce new salts that exhibit altered solubilities.

### Technical Background

The physicochemical properties of active drug substances, imaging agent or excipients differs, which may affect their route of administration and general application of such compounds. These molecules may be charged, neutral, hydrophilic, hydrophobic, and amphiphilic which impacts their physicochemical compatibility with various formulations or fiducial marker matrices. Several compounds may therefore be excluded from certain routes of administration or applications, which ultimately may lower their applicability, bioavailability and efficacy.

Carbohydrate ester depots also referred to as ICells, are composed of carbohydrate ester scaffolds, organic co-solvents and solvents, which are designed for embedding hydrophobic substances such as imaging agents, colours, or fluorophores and to provide sustained release of hydrophobic drug molecules. Hydrophilic agents or drug molecules are rarely compatible with the ICell vehicle, which has limited its use for a broad range of such compounds. ICells are primarily used as fiducial markers in radiotherapy, surgical procedures, and as sustained release drug depots.

Hydrophobic ion pairing (HIP) is the process of forming ionic interactions between a charged hydrophilic molecule and a hydrophobic counterion. The complexation increases hydrophobicity by two main mechanisms: First, the molecule's natural charge is masked, mitigating solubility in polar solvents such as water. Second, the hydrophobic groups on the counterion, typically nonpolar aliphatic tails or aromatic groups, help to coat the original molecule's surface area with hydrophobic domains that exclude water. HIP thus converts hydrophilic molecules into hydrophobic ion pairs that can be solubilized and released from hydrophobic drug delivery vehicles such as but not limited to oils and ICell compositions. The HIPs possibly remain hydrophobic while solubilized in the ICell. In aqueous media, the HIPs may on the contrary dissociate as the water dipole screening of charges lower the charge-charge interaction energy, thereby releasing the native compound upon exposure to aqueous media.

Engineering of small molecule active pharmaceutical ingredients (API) water solubilities can enable new processing methods by formulation in hydrophobic drug delivery systems, such as but not limited to oils, carbohydrate ester gels (described in patent EP1042339 and US6352722) and ICells. This can be performed by reversible covalent conjugation of APIs with hydrophobic molecules, which results in the production of a hydrophobic prodrug. After encapsulation and delivery, covalent linkages are cleaved, thereby producing the original API for therapeutic activity. However, covalent modification of APIs results in the creation of a new molecular entity, which requires additional comprehensive, costly, and time-consuming testing for Food & Drug Administration (FDA) approval. API salt form engineering is an alternative route to enhance and control API solubility in ICells. In this process, charged functional groups on the API are ion-paired with a counter ion to produce an API with transiently altered solubilities. An advantage of salt form engineering is that resultant products are not considered new molecular entities, and do not require full FDA reapproval. However, in most instances, API salt forms are screened and engineered for enhanced water solubility. The alternative use of hydrophobic ion-pairs to create hydrophobic salt forms is a non-conventional method to increase API hydrophobicity, which is unexplored in the context of hydrophobic depots such as but not limited to oils and ICell compositions.

Previously, ion pairing has been used to formulate nanoparticles from hydrophobic compounds. These are defined by having logP values greater than 1, 2, 3, 4, or 5 at neutral pH [Pinkerton, N.M., et al, Formation of stable nanocarriers by in situ ion pairing during block-copolymer directed rapid precipitation. Molecular pharmaceutics, 2013. 10(1): p.319-328]. For example, Song et.al [Song, Y.H., et al, A novel in situ hydrophobic ion pairing (HIP) formulation strategy for clinical product selection of a nanoparticle drug delivery system. Journal of Controlled Release, 2016. 229: p. 106-119.] used ion pairing for AZD281, which had a logP of 2 at a neutral pH of 7. Another group of researchers has complexed proteins and polymeric charged species with oppositely charged polymeric compounds with high solubility. High solubility could mean logP values less than -2 and/or solubilities in aqueous media of over 10 mg/ml at pH=7. Patel and Guadana [Gaudana, R., et al, Design and evaluation of a novel nanoparticulate-based formulation encapsulating a HIP complex of lysozyme. Pharmaceutical development and technology, 2013. 18(3): p. 752-759; Patel, A., R. Gaudana, and A.K. Mitra, A novel approach for antibody Nanocarriers development through hydrophobic ion-pairing complexation. Journal of Microencapsulation, 2014. 31(6): p. 542-550] showed that soluble antibody or lysozyme proteins could be complexed with oppositely charged dextran sulfate. Dextran sulfate is a water-soluble polymer. The resulting precipitate is not a hydrophobic complex, but rather a "coacervate" as commonly defined in the polymer community [Antonov, M., M. Mazzawi, and P.L. Dubin, Entering and exiting the protein-polyelectrolyte coacervate phase via nonmonotonic salt dependence of critical conditions. Biomacromolecules, 2009. 11(1): p. 51-59; Overbeek, J.T.G. and M. Voorn, Phase separation in polyelectrolyte solutions. Theory of complex coacervation. Journal of Cellular Physiology, 1957. 49(S 1): p. 7-26]. A coacervate is a precipitate resulting from ionic interactions between two soluble polymer species with opposite charges.

Hydrophobic ion paring (HIP) has previously been patented for encapsulation and precipitation of APIs in nanoparticles (WO2019090030).

### Summary of the invention

The present invention concerns active drug substances, biologics, imaging agents, fluorophores and excipients formulated as hydrophobic ion pairs/complexes in ICell fiducial markers or depots for sustained therapeutic or diagnostic release. The present invention describes the enhanced stability, solubility and release of hydrophobic ion pairs from hydrophobic media, such as carbohydrate ester gels, oils and ICells. The size, hydrophobicity and acidity (pKa or pKb) of the hydrophobic co-ion utilized for ion parring may serve as important parameters for tuning the retention or release of the hydrophobic ion pairs (HIPs) of drug, imaging agent or excipient from ICells. Upon release of the HIPs into aqueous media or tissue, the HIPs may dissociate into the native compounds and exert their function/action. Using this procedure, otherwise insoluble biologics, immunotherapeutics, chemotherapeutics, antibiotics, peptides, proteins, antibodies, and the like may be formulated in ICells.

In line with the above, according to a first aspect the present invention provides a composition forming a hydrophobic ion par (HIP), comprising:
a. an imaging agent with zwitterionic, cationic or anionic charge;
b. a counterion being a hydrophobic or amphiphilic acid or base, or salt thereof;
c. a non-water soluble carbohydrate;
d. a non-aqueous solvent;

wherein at least 50% of the non-water soluble carbohydrates are carbohydrates selected from derivatives of disaccharides or trisaccharides selected from sucrose, lactose, maltose, trehalose, raffinose, or derivatives of disaccharides or trisaccharides, or mixtures thereof;
wherein the composition is a liquid before administration into the human or animal body and increases in viscosity by more than 10,000 centipoise (cP) after administration to transform into a ICell;
and wherein the hydrophobic or amphiphilic acid or base or salt thereof enhances the solubility of the imaging agent in the ICell by forming a hydrophobic ion-pair or multiple hydrophobic ion-pairs with the imaging agent.

According to a second aspect, the present invention provides a composition forming hydrophobic ion pairing (HIP) comprising:
a. a pharmaceutical agent with a zwitterionic, cationic or anionic charge;
b. a counterion being a hydrophobic or amphiphilic acid or base, or salt thereof;
c. a non-water soluble carbohydrate;
d. a non-aqueous solvent

wherein at least 30% of the non-water soluble carbohydrates are carbohydrates selected from derivatives of disaccharides or trisaccharides selected from sucrose, lactose, maltose, trehalose, raffinose, or derivatives of disaccharides or trisaccharides, or mixtures thereof;
wherein the composition is a liquid before administration into the human or animal body and increases in viscosity by more than 10,000 centipoise (cP) after administration to transform into a ICell;
and wherein the hydrophobic or amphiphilic acid or base or salt thereof enhances the solubility of the pharmaceutical agent in the ICell by forming a hydrophobic ion-pair or multiple hydrophobic ion-pairs with the pharmaceutical agent.

The present disclosure also provides methods for forming hydrophobic ion pairs (HIPs) of charged hydrophilic, charged amphipathic, or charged hydrophobic molecules and methods for solubilization of these in hydrophobic formulations such as but not limited to ICell compositions. For this purpose, charged molecules are complexed by hydrophobic counterions forming hydrophobic ion pairs that can be solubilized in hydrophobic media such as but not limited to organic solvents, oils, solid lipid nanoparticles, micelles, liposomes, polymersomes, polymeric nanoparticles and ICells.

The HIP complexes of current disclosure do overall not dissociate and cause precipitation in the ICell formulation, but surprisingly allows for incorporation of highly hydrophilic and charged compounds in ICell, such as but not limited to antibiotics, immunotherapeutic, peptides, imaging agents, fluorescent dyes that otherwise would be completely or partially insoluble. Using methods of the current disclosure, such agents may be incorporated and solubilized in complex solvents as such as the ICell in remarkably high concentrations e.g. more than 20 mg/g or higher. The imaging agent may be incorporated and solubilized in the ICell at a concentration of 0.01-1 mg, 20-200 mg/g or 1-10 mg/g. Remarkably, the current disclosure allows for either complete retention or sustained release of charged hydrophilic, charged amphipathic or charged hydrophobic molecules depending on co-ions used for ion-parring. Imaging agents or fluorophores that otherwise would be insoluble in ICell may therefore be solubilized and effectively retained in ICells for use as fiducial marker. Replacing co-ions of the HIP complexes with alternative co-ions having different physicochemical properties provide means for obtaining sustained release of the same compounds, which allows for unique tuning of the ICell release kinetics, which is unexpected. Using methods of the current disclosure charged hydrophilic peptides, antibiotics and fluorophores were embedded in ICells and more than 50% release of the cargo was achieved. The current disclosure thus expands the range of compounds that ICell can deliver, and further offers new possibilities for tuning release rates, even for charged hydrophobic compounds.

The current disclosure enhances compatibility of otherwise hydrophilic and/or charged molecules with organic media by formation of overall hydrophobic ion complexes. This has great importance for compound classes including but not limited to small molecule therapeutics, such as anticancer agents, immunotherapeutic, chemotherapeutics, antimicrobials (antibiotics, antivirals, antifungals, antiprotozoals, and antihelminthics), anti-inflammatory agents, anticoagulant, antidepressant, antiepileptic, antipsychotic, sedatives, antidiabetic, cardiovascular, and analgesic agents, peptides such as, but not limited to, therapeutic peptides, agonistic and antagonistic receptor ligands, peptides with regulatory or enzymatic activity, hormones, peptides with special targeting activities, vaccines, antigens, therapeutic peptides, diagnostic peptides, steroids, proteins such as but not limited to immunotherapeutic agonists and antagonists, growth factors, cytokines, chemokines, hormones, glycoprotein hormones and antibodies, affibodies, peptide and nanobodies and formulation of functional ICell excipients/additives such as but not limited to CT imaging agent, radiographic imaging agent, fluorophores, chromophores, MRI imaging agents, radiotracers, and contrast carrying nanoparticles.

Controlling drug or bioactive agent delivery kinetics and being able to maintain a release of drug(s) that are activate locally, locoregionally or systemically, has important therapeutic indications across several diseases and pathological conditions. Surprisingly, ion-pairing allows for both enhanced solubility and effective control of release rates for several charged species that due to their high hydrophilicity would be incompatible with ICells. The therapeutic indications for the combination of hydrophobic ion-pairing in combination with ICell technology have extensive applications across a broad selection of disease, either as immune stimulating agents, single agent or multiagent drug delivery or as adjuvant drugs for improving therapeutic outcome in combination therapies in human or animal diseases. Indications includes, but are not limited to: vaccination against; cancer (cancer antigens and neoantigens), infectious diseases, cancer therapy; immunotherapy, chemotherapy, adjuvant therapy for radiation therapy, cancer surgery, cryotherapy, ablation therapy, adoptive cell transfer, pre-, intra-, and post-surgical therapy for; improved healing, bone loss, peritendinous adhesions, bone non-union, infections associated with; soft tissue, bones, abscesses, phlegmons, fistulas, infections associated with, or in, catheters, drains, stents, tissue grafts, transplantations, transplanted organs, artificial organs, hearing aids, implanted devices, mechanical devices, electronic devices, bone implants (hip, knee, joint), metal and ceramic internal and external fixation devices, artificial organs, protheses, pain relief; local and systemic analgesia and anaesthesia, local or systemic immune suppression, autoimmune disorders, rheumatoid disease, degenerative disorders, anaemia, myelodysplastic syndrome, endocrine disorders; endocrine gland hyposecretion, endocrine gland hypersecretion, tumours (benign or malignant) of endocrine glands, hormone replacements, hormone supplementation, hormone imbalances, hormone inactivation, adoptive cell therapies; stem cells, chimeric antigen receptor cell products, myeloid and lymphoid cell products.

API HIP complexes in ICell may provide a controlled therapeutic effect locally, loco-regionally, intracavitary or provide a systemic effect following local release. The formulation of small molecule therapeutics, peptides, proteins, glycoproteins, glycopeptides, bioactive agents, antibodies, affibodies, and nanobodies using hydrophobic ion parring is highly important for local drug release and delivery technologies using carbohydrate ester gel, oil or ICell compositions.

The ICell technology is not limited by the type of tissue, region or anatomical position that can be injected with ICell HIPs. For application in cancer therapy injected locations may include primary cancers, metastatic lesions and/or lymph nodes or lymphatic tissues to achieve cancer cell death, antigen release and immune activation. In the case of infectious disease, the injection(s) may be performed directly in or around the infected lesions or in the periphery of the region to be treated. It can further be primary solid cancers, peritumoral tissues, metastatic lymph nodes or metastatic lesions. For application in combination with surgical procedures, tissue grafts, catheters, stents, drains, devices, implants the injection(s) may be performed both in tissue and in the canals or cavities associated with these. When used in combination with cell-based therapies, the ICell may be injected both in the site (e.g., primary solid cancer, metastases, lymph node) where a specific immune activation (e.g., recruitment, engraftment, proliferation) of the cell product is to be achieved. The injection may also be injected at a site where the immune activation will take place e.g., sub cutaneous space, spleen, lymph node, intramuscular, intradermal, intraarticular, in bone and soft tissue. For supporting stem cell-based therapies the ICell may be injected adjacent to the injection of the stem cell product and/or in a tissue or organ of interest and here support engraftment, survival, proliferation and polarization of the stem cell product. In case the ICell is intended to provide systemic controlled dosing of the HIP complexed bioactive(s) (e.g., hormone therapy, sustained release of anti-inflammatory drugs, immunosuppressive drugs, statins, metabolically active agents, vaccine antigens) injection may be flexible in terms of location.

Some HIPs of the current invention have optimized interactions with the drug delivery depot systems described in WO2020/249801A1, which allows for optimized retention or release of excipients, imaging agents or active drugs chosen from a broader class of charged hydrophilic, charged amphipathic, charged hydrophobic compounds.

Biomaterials for use as drug delivery systems have found wide interest for treatment of multiple diseases and conditions in humans and animals, such as pain, inflammation, infection, allergy, and cancer. Other patents and articles have described the use of biomaterials for controlled release of drugs for various applications. EP1212092 and US6413536 describe formulations for drug delivery based on a hydrophobic gel matrix consisting of organic solvent, a saccharide ester based on sucrose derivatives such as SAIB or other poly-ols and one or several drugs. The injectable formulations are based on derivatized carbohydrates. Furthermore, EP1042339 and US6352722 describe isomers of derivatives of sucrose, lactose, cellobiose and trehalose for drug delivery.

The present invention provides HIPs of excipients, imaging agents, active drug species and the like that may be formulated in injectable liquid compositions that forms a gel, semisolid or solid material after administration to human or animal body after which it provides a system with controlled and tuneable HIP release rate. The HIPs of excipients, imaging agents, active drug species and the like may further be administered systemically, intratumorally, intralesional or in any position, tissue or organ in the body.

There may be a range hydrophobic ion pair of drugs, imaging agents, fluorophores or excipients for administration in free form, in drug delivery systems or incorporation in controlled drug delivery systems for direct injection in the tissues that are to be immune modulated or stimulated by the drug included in the drug delivery system.

ICell fiducial markers containing hydrophobic ion pairs of fluorophores, may be injected as surrogate markers in cancerous tissue or lesions of interest. Such fiducial markers may aid surgeons in locating the tissue of interest using NIR fluorescence imaging. The hydrophobic ion pair of the fluorophores may further be released from the ICell, whereafter it may visualize lymphatic drainage and accumulate in draining lymph nodes.

The present invention may provide HIPs of drugs, prodrugs, drug analogues and bioactive agents for systemic or local administration, or formulation in a controlled multidrug release system for injection using thin (down to 30 Ga.) needle technologies. The HIPs of the invention may be prodrugs and can either be targeted to receptors or cell populations, can be activated by intrinsic of extrinsic factors present at the target site, or have optimized interaction with the controlled multidrug release system or a combination thereof. The ICell system may include the possibility to provide controlled release of both single drugs and multiple drugs. ICells may accommodate multiple classes of bioactive agents, including drugs, prodrugs, drug conjugates, and the release kinetics optimized to have synchronous release of drugs or separate release profiles for individual drugs throughout the course of intended therapy.

The overall hydrophobicity or size of HIP complexes of APIs, excipients and the like, may be modulated so that complete retention, complete release or stages in between may be obtained from ICells injected into tissue, or other aqueous filled spaces. Indocyanine green (ICG) may as an example be ion-paired and solubilized in ICell with full or partial retention upon injection into tissues.

In one embodiment of the present invention the composition comprises indocyanine green (ICG), and forms an ICG-HIP complex in ICell after administration.

### Definitions

"Prodrug" refers to drug molecules that have been chemically modified via covalent bonds. Upon activation of the prodrug, one or more covalent bonds are broken, and the native drug is released.

"Drug analogue" refers to a modified version of a drug where new chemical bonds have been created.

"Targeting" of drugs, drug analogues or prodrugs refers to drug molecules that carry a ligand that enhances the molecules affinity for certain tissues or cells.

"Non-water soluble carbohydrates" refers to carbohydrates that are insoluble in water, which is defined as carbohydrates that precipitates when the concentration exceeds 0.1 M at 25 degrees Celsius.

In the context of the present invention, a "gel" is defined as a carrier matrix in which the detectable agent (imaging agent) or active pharmaceutical ingredient is dispersed and/or dissolved within.

The term "gel" as used in the present invention includes systems such as gels or amorphous glass matrices, crystalline solids, amorphous solids, which upon injection into a human or an animal increases viscosity where the composition changes from being liquid like to gel-like in its appearance.

In term "ICell" is used for gel compositions comprising carbohydrate esters that have features of a gel.

With the term "hydrophobicity" we refer to the effect that molecule is seemingly repelled from water, that is a molecule that has a logP > 0.

With the term "gel-like" compound or material, as used herein, we refer to any compound comprising some of the properties of a gel i.e. a material that exhibits limited flow when in the steady-state. By weight, gels are mostly liquid, yet they behave like solids due to a three-dimensional interactions within the liquid. It is the interactions within the fluid that gives a gel its structure (hardness) and contributes to the adhesive stick. In this way gels are a dispersion of molecules of a liquid within a solid in which the solid is the continuous phase and the liquid is the discontinuous phase providing a gel-like material with a higher viscosity than for that of a liquid.

The terms "drug", "medicament", "agent", or "pharmaceutical agent" as used herein include, biologically, physiologically, or pharmacologically active substances that act locally or systemically in the human or animal body.

The terms "treating", "treatment" and "therapy" as used herein refer equally to curative therapy, prophylactic or preventative therapy and ameliorating therapy. The term includes an approach for obtaining beneficial or desired physiological results, which may be established clinically. For purposes of this invention, beneficial or desired clinical results include, but are not limited to, alleviation of symptoms, diminishment of extent of disease, stabilized (i.e., not worsening) condition, delay or slowing of progression or worsening of condition/symptoms, amelioration or palliation of the condition or symptoms, and remission (whether partial or total), whether detectable or undetectable.

The term "hydrophobic ion pair" (HIP) refers to the process and product of forming ionic interactions between a charged hydrophilic, hydrophobic or amphipathic molecule with an oppositely charged counterion. The counterion contains at least one hydrophobic domain such as an alkyl tail or aromatic ring.

The term "counterion", "co-ion", 'ion pair(ing agent) (IP),' or 'salt former, refers to an ion with opposite charge of the hydrophilic molecule.

The term "hydrophobic counterion" is a counterion containing hydrophobic domains.

The term "excipient" refers to pharmaceutically inactive compounds of the formulation, e.g. the carbohydrate esters, imaging agents, fluorophores, dyes, oils or solvents of the ICell composition.

The terms "low dielectric" media refers to a media with low dielectric constant, such as organic solvents, oils and the like.

As used herein, "drug", "therapeutic", "active agent" or "bioactive agent" refers to any compound or mixture of compounds which produces a physiological result, e.g., a beneficial or useful result, upon contact with a living organism, e.g., a mammal, such as a human. Active agents are distinguishable from other components of the delivery compositions, such as carriers, diluents, binders, colorants, etc. The active agent may be any molecule, as well as binding portion or fragment thereof, that can modulate a biological process in a living subject. In certain embodiments, the active agent may be a substance used in the diagnosis, treatment, or prevention of a disease or as a component of a medication. In some embodiments, an active agent may refer to a compound that facilitates obtaining diagnostic information about a targeted site in a body of a living organism, such as a mammal or in a human. For example, imaging agents may be classified as active agents in the present invention as they are substances that provide imaging information required for diagnosis.

"Viscosity" herein refers to the state of being thick, sticky, and semi-fluid in consistency, due to internal friction. Viscosity is a quantity expressing the magnitude of internal friction in a fluid, as measured by the force per unit area resisting uniform flow. Viscosity can be quantified in the unit centipoise (cP) and can be measured using an EMS Viscometer (EMS-1000S).

"Release rate" herein refers to the rate or speed at which a compound such as a HIP complex of an API, excipient, imaging agent and the like are released from the ICell or depot into the aqueous embedding media or tissue.

"Release profile" herein refers to the profile said progression of the accumulated release of a compound as function of time.

"Imaging agent" herein refers to agents that provides contrast or visibility in imaging applications, such as e.g. ultrasound sonography (US), magnetic resonance imaging (MRI), x-ray, fluoroscopy or Computed tomography (CT) contrast imaging, visible colours, fluorophores including dyes visible in the Infrared (IR) and Near Infrared (NIR) region, or radionuclides for PET and SPECT imaging.

### Detailed description of the invention

Compounds eligible for HIP may be small molecule drugs, imaging agents, dyes, fluorophores, peptides, proteins, interleukins, chemoleukins, chemokines, cytokines, antibodies, nanobodies, or the like that has one or more ionizable groups or moieties. The ionizable group may be amines (primary, secondary, tertiary or quaternary), alcohols, carboxylic acids, thiols, sulphonic, sulphenic or sulfinic acids, tetraalkyl phosphonium, phosphonic, phosphenic, or phosphinic acids, phosphorothioates and or conjugated systems with delocalized charges, and the like. Compounds eligible for HIP may contain one or more ionizable groups of the same or opposite charge. Depending on the acidity (pKa) of the molecules ionizable groups, varying charge of the molecule may be obtained as function of pH. Control of pH during preparation of HIP may be important as charge regulation on both the hydrophobic co-ion and the active molecule with ionizable groups may be crucial for a successful HIP formation.

The counterions, also referred to as co-ions, used for hydrophobic ion pairing should contain at least one charged group and at least one hydrophobic domain. The counterions may be either anionic or cationic and typically contain either one, two or multiple charged groups. The ionizable group of the hydrophobic counterions may be amines (primary, secondary, tertiary or quaternary), alcohols, carboxylic acids, thiols, sulphonic, sulphenic or sulfinic acids, tetraalkyl phosphonium, phosphonic, phosphenic, or phosphinic acids, phosphorothioates or conjugated systems with delocalized charges, and the like.

The counterion can have a logP value of 2 or greater at a pH of 7. The counterion can have a logP value of greater than 5. The counterion can be an anionic counterion that has a pKa value of from -2 to 5. The counterion can have a pKb value of greater than 3. The counterion can be a quaternized cationic species, for example, a quaternized cationic species that is permanently cationic.

Some counterions may exert pharmacological or diagnostic activity beyond their actions as hydrophobic counterion.

Examples of counterions suitable for HIP complexation of APIs and ICell excipients with at least one charge and at least one hydrophobic domain includes, but not limited to, the cationic lipids, DC-chol, DMTAP, DPTAP, DSTAP, DOTAP, DOTMA, DOSPA, DDAB, DMDAP, DPDAP, DSDAP, DODAP, DODMA, DOBAQ, DLin-DMA, DLin-KC2-DMA, DLin-MC3-DMA, C12-200, A6, OF-02, A18-Iso5-2DC18, YSK05, 7C1, G0-C14, L319, OF-Deg-Lin, 306-O12B, 3060110, FTT5, 9A1P9, 98N12-5, 304013, cKK-E12, Spermine-chol, and MVL5, such as the carboxylic acids, acetic acid, propanoic acid, butanoic acid, hexanoic acid, octanoic acid, benzoic acid, Myristic acid, Myristoleic acid, Palmitic acid, Palmitoleic acid, Stearic acid, Oleic acid, Elaidic acid, Linoleic acid, Linolenic acid, Linolelaidic acid, and bilesalts such as Cholic Acid, such as Benethamine (N-benzyl-2-phenylethanamine), Dodecylamine (laurylamine), Hexadecyl trimethylammonium(cetrimonium) bromide (CTAB), Maprotiline, Na-Deoxycholyl-L-lysyl-methylester, N,N'-Dibenzyl ethylenediamine(benzathine), N,N-Dimethyl dodecylamine (DDA), N,N-Dimethyl hexylamine, N,N-Dimethyl octadecylamine(dimethyl stearamine), Tetrabutyl ammonium bromide (TBAB), Tetraheptyl ammonium bromide (THA), Tetrahexyl ammonium bromide, Tetraoctyl ammonium bromide (TOAB), Tetrapentyl ammonium bromide (TPA), Triethylamine (TEA), such as but not limited to the multivalent cationic lipids N1 -[2-((1S)-1-[(3-aminopropyl)amino]-4-[di(3-amino-propyl)amino]-butylcarboxamido)ethyl]-3,4-di[oleyloxy]-benzamide or N4-Cholesteryl-Spermine, such as counterions belonging to the group of phosphatidic acid (PA), cyclic PA, lysophosphatidic acid (LPA), cyclic LPA, phosphatidylglycerol (PG), lysophosphatidylglycerol (LPG), phosphoinositides (PI), lysophosphatidylinositol (LPI), phosphatidylserine (PS), or Lysophosphatidylserine (LPS) phospholipids and or ether lipids, or sphingolipids (SP) or sphingolysolipids (LSP), all with mixed or non-mixed, saturated and or unsaturated, aliphatic and or aromatic, C6-C22 hydrocarbon chains. Preferred examples of lipid chains include, but are not limited to propionyl, butyryl, hexanoyl, octanoyl, decanoyl, lauroyl, myristoyl, palmitoyl, stearoyl, arachidoyl, behenoyl, lignoceroyl and the like. Other preferred examples of lipid chains are vaccenoyl, (8Z)octadecenoyl, myristoleoyl, myristelaidoyl, palmitoleoyl, palmitelaidoyl, oleoyl, dielaidoyl and the like.

Examples of counterions suitable for HIP complexation of APIs and ICell excipients with at least one charge and at least one hydrophobic domain includes, but not limited to, hexanoate, decanoate, myristate, oleate, Pamoic acid, deoxycholate, Benezenesulfonic acid monohydrate, 2-naphthalenesulfonate, Camphorsulfonic acid, 1,2-ethanesulfonate, 1-heptanesulfonate, 1-octane-sulfonate monohydrate, 1-decanesulfonate, dodecyl sulfate (SDS), dodecyl-benzenesulfonate (DBS), Linoleic acid, Cis-2-decenoic acid, docusate, Curcumin, Tetraethyl ammonium bromide, Benzyl trimethyl ammonium chloride, Cetyl trimethyl ammonium bromide (CTAB), N-benzyl-2-phenylethanamine (benethamine), Dodecylamine, Tetrahexyl ammonium bromide (THAB), Didodecyl dimethyl ammonium bromide (DDAB), Octadecylamine, Tetraoctyl ammonium bromide (TOAB), Tridodecyl methyl ammonium chloride (TDMAC) and the like.

### Methods for forming HIP complexes

According to one aspect, the present invention provides a method for forming one or more hydrophobic ion pairing (HIP) complexes, wherein the method comprises the steps of:
a) dissolving an imaging agent in a first liquid;
b) dissolving a hydrophobic counterion in a second liquid;
c) mixing the solution of step a) and b);
d) allowing the HIP formation;
e) resuspending the HIPs in an organic solvent, oil or ICell composition; and
f) removal of at least aqueous solvent/phase.

The present invention also embodies a method for forming one or more hydrophobic ion pairing (HIP) complexes, wherein the method comprises the steps of:
a) dissolving a pharmaceutical agent in a first liquid;
b) dissolving a hydrophobic counterion in a second liquid;
c) mixing the solution of step a) and b);
d) allowing the HIP formation;
e) resuspending the HIPs in an organic solvent, oil or ICell composition; and
f) removal of at least aqueous solvent/phase.

It should be noted that step f comprising removal may also include removal of several solvents, such as co-solvents, used in the method according to the present invention.

During HIP formation, an API, excipient, or the like forms a hydrophobic ion pair with a hydrophobic counterion. Depending on the physicochemical properties of the API, excipients or hydrophobic counterion different approaches may be applied. If all species of the complex are individually soluble in water, the organic solvent-free method may be employed. The first liquid may be water.

The hydrophobic counterion may be water soluble and the second liquid may be water. The hydrophobic counterion may be weakly water-soluble and the second liquid is methanol.

For the organic solvent-free method, both API or excipient, and the hydrophobic counterion may be dissolved in water or buffers. The pH of the aqueous buffer may be adjusted so that the charge of the API or excipient and the hydrophobic counterion is non-zero and have opposing charge. Hereafter the API or excipient, and the hydrophobic counterion is mixed and agitated whereafter the HIP forms and precipitates with time. Hereafter the HIP can be spun down by centrifugation, washed and resuspended in organic solvent, oils or ICell compositions. Advantages of the organic solvent-free method is that potentially toxic solvents are avoided.

The hydrophobic counterion may be weakly water-soluble and the second liquid may be chloroform. The hydrophobic counterion may be weakly water-soluble and the second liquid may be an organic solvent being mixable with water.

For counterions that are insoluble or weakly soluble in water, one of the two following approaches may be used.

In the first approach, the API or excipient is dissolved in water, and the counterion in methanol. Next, the API or excipient in water (aq) and the counterion in methanol (MeOH) is mixed with chloroform in the ratio API/excipient (aq) : counterion (MeOH) : chloroform (1:2:1). The mixture forms a monophase which is shaken or stirred for >30min wherein the HIP form. Next, a biphasic system is formed by addition of chloroform and water until reaching the ratio water:MeOH:Chloroform (1:1:1) whereafter the HIP partition into the organic phase. The HIP may be isolated from the organic phase by evaporation of the solvent whereafter it can be resuspended or solubilized in organic solvents, oils or ICell compositions.

Alternatively, the API or excipient is dissolved in water and the hydrophobic counterion in chloroform. Equal volumes of the API or excipient and the counterion is mixed forming a biphasic system. The solution is agitated or stirred for 2-4 hours whereafter the solutions is centrifuged promoting phase separation. Following phase separation, the HIP partitions into the organic phase wherefrom it can be isolated.

Alternatively, the API or excipients is dissolved in water and the hydrophobic counterion in an organic solvent which is mixable with water. The water and organic phase are mixed, and the solution is agitated or stirred for 1-4 hours. Following, the aqueous fraction of the mixture may be removed by vacuum distillation at low temperature if the organic solvent has a higher boiling point than water and does not form an azeotrope with water. After distillation, the remaining phase is comprised of the organic solvent and the HIP complex. Relevant solvents for vacuum transfer are but not limited to DMSO, PC, EtOH, iso-propanol and BnOH.

Depending on solvent sensitivity of the API or excipient, variations of the previous described methods using alternative solvent may be applied to minimize denaturation or deterioration of peptides, proteins, antibodies and the like.

The formed HIP complex may have an increased hydrophobicity and size depending on the HIP complex stoichiometry and choice of co-ions.

The method may have an additional step a'), performed between step a) and b). The step a') may be to adjust the pH of the solution such that the charge of the imaging agent or pharmaceutical agent and the hydrophobic counterion is non-zero and have opposing charge.

The mixing of step c) may be performed in the presence of chloroform or DCM. The formation of step d) may be performed through HIP partition into an organic phase. The formation of the one or more HIP complexes may be performed through agitation and precipitation.

### Controlled release of HIPs from ICells

ICell is a drug delivery platform composed of a hydrophobic matrix. The hydrophobic matrix may be made from gel-forming carbohydrate esters, oils of the ICell solution (also referred to as co-solvent), and solvents of the ICell solution. Overall, the ICell composition is hydrophobic, but the carbohydrate backbone of the carbohydrate esters also provides options for hydrogen bonding via oxygen.

HIPs may be dispersed in ICell compositions as powder, crystals, nanoparticles and the like or be fully solubilized in the ICell composition. HIPs in the ICell formulation or depots formed upon injection into tissues may therefore interact with the ICell constituents via hydrogen bonding, dipole or van der Wahls interactions. Enhancing the interaction of the HIPs and ICell may reduce release rate of HIPs from the ICell, whereas reduction of the HIPs interaction with the ICell oppositely may increase the release rate of API.

HIPs are hydrophobic salts of APIs, imaging agents, fluorophores or other excipients, and interaction of these with the ICell may be regulated via the type of hydrophobic co-ion used for complexing. Using more hydrophobic co-ions with a larger logP may enhance the interaction of the HIP with the ICell via enhanced van der Wahls interaction. Using bulkier co-ions may increase the size of the HIP complex, which increases the steric interaction of the HIP and ICell. Both effects of using more hydrophobic or bulkier co-ions may enhance the interaction of the HIP and the ICell and may lead to altered release rate of the HIP. The overall hydrophobicity of the HIP complex may additionally be reduced or increased by the co-ion type and hydrophobicity, the stoichiometry of the HIP complex, e.g., the ratio of co-ions to API, excipient and the like can be increased from 1:1 to 2:1 or higher depending on the number of ionizable groups on the API, excipient, and the like.

HIP complexation of APIs may in addition to improving solubility in the ICell composition also allow for improving control and tailoring of drug release profiles through embedding multiple different HIP complexes of the same API in a single ICell. Enhanced solubility of ICG in ICell is demonstrated in example 5, and enhanced solubility of Gentamycin, Clindamycin and Abaloparatide in ICell is shown in example 15, 17 and 27 respectively. In example 11 and 18 it is illustrated how the selection of the counterion in the HIP complex can be used to control release profile and release rate from ICells. A rational selection of HIPs with differing counterions can therefore be used to create bi-, tri- or multiphased release profile of a specific API. This can secure a rapid saturation or loading dose followed by a long-term drug maintenance dose locally or systemically. As example this could be used to release 25-50% within the first 3 days followed by complete release of the slower release HIP complex over the following 2-3 weeks. In a therapeutic setting this may be of particular interest for cancer immunotherapy and vacinology. In these cases, the coordinated release of APIs that modulate, manipulate or optimize various stages of immune response generation. In cancer this may be particularly interesting for intratumoral delivery of ICells that have rapid release of a cancer cell death inducing agent, e.g., doxorubicin followed by long term immune activation of innate and adaptive APIs e.g., TLR agonists and compounds that polarize the tumor microenvironment, e.g., TGFb inhibitors. In the case of vaccines that ability to provide long term immune stimulation may counteract the development of immunological exhaustion and thereby secure optimal memory formation.

### ICell forming component

**ICell comprising components:** The ICell solution, is the solution of the composition before injection of the ICell solution into human or animal tissues or aqueous media, after which it forms the ICell as a gel or gel-like depot within the tissue or aqueous media. The ICell solution may comprise organic solvents, oils (co-solvent), gel-forming carbohydrates ester and polynucleotide complexes.lt is a fluid with viscosities that increases after administration to the body by more than 10,000 centipose (cP) after administration. Typically, but not limited to, the viscosity may be in the range of 50-5000cP. Upon administration of the ICell solution into tissues, the solution is in contact with aqeuous fluids which causes phase separation to occur. In this process, the organic solvent of the ICell solution diffuses into the aqueous phase (interstitial fluids), and the oil (co-solvent) and gel-forming carbohydrate esters forms a high viscosity fluid, solidify or precipitate or a combination thereof forming a hydrophobic depot (the ICell) at the site of injection. The hydrophobic depot provides controlled release over time from hours, days, weeks or months of the polynucleotide complex. The viscous fluid, solid, precipitate or combinations thereof is referred to as a gel or gel depot or the ICell.

**Solvents of the ICell solution:** Solvents of the ICell solution are soluble or mixable in hydrophobic substances. Solvents may be the oil (co-solvent) and gel-forming carbohydrates, as well as in hydrophilic substances such as water. This partial hydrophilic / hydrophobic property of the solvents drives the non-solvent induced phase separation since the solvents of the ICell solution readily diffuses out of the ICell solution or ICell when exposed to an aqueous environment.

The chemical composition of the solvent (dispersion medium) should not be particularly limited, and examples include biocompatible organic solvents such as ethanol, ethyl lactate, propylene carbonate, glycofurol, N-methylpyrrolidone, 2-pyrrolidone, propylene glycol, acetone, methyl acetate, ethyl acetate, methyl ethyl ketone, benzyl alcohol, triacetin, dimethylformamide, dimethylsulfoxide, tetrahydrofuran, caprolactam, decylmethylsulfoxide, such as but not limited to N-methyl-2-pyrrolidone, glycofurol, polyethylene glycol (PEG), benzyl benzoate, triglycerides, acetone, benzyl alcohol, V-(betahydromethyl) lactamide, butylene glycol, caprolactam, caprolactone, corn oil, decylmethylsulfoxide, dimethyl ether, dimethyl sulfoxide, 1-dodecylazacycloheptan-2-one, ethanol, ethyl acetate, ethyl lactate, ethyl oleate, glycerol, glycofurol (tetraglycol), isopropyl myristate, methyl acetate, methyl ethyl ketone, esters of caprylic and/or capric acids with glycerol or alkylene glycols, oleic acid, peanut oil, polyethylene glycol, propylene carbonate, 2-pyrrolidone, sesame oil, [±]-2,2-dimethyl-1 ,3-dioxolane-4-methanol, tetrahydrofuran, diethylene glycol monoethyl ether, carbitol, triacetin, triethyl citrate, and combinations thereof; or desirably from trichlorofluoromethane, dichlorofluoromethane, tetrafluoroethane, dimethyl ether, propane, butane, and combinations thereof; or specifically from caprylic/capric triglyceride, oleic acid, 1-dodecylazacycloheptan-2-one and the like. Although the gel formulation can be stably dispersed in these solvents (dispersion media), the solvents may be further added with a saccharide derivatives of for example, triglycerides such as tri-pentanoyl glycerol, tri-octanoyl glycerol, tri-dodecanoyl glycerol, a monosaccharide such as glucose, galactose, mannose, fructose, inositol, ribose and xylose, disaccharide such as lactose, sucrose, cellobiose, trehalose and maltose, trisaccharide such as raffinose and melezitose, and polysaccharide such as α-, β-, or γ-cyclodextrin, sugar alcohol such as erythritol, xylitol, sorbitol, mannitol, and maltitol, or a polyhydric alcohol such as glycerin, diglycerin, polyglycerin, propylene glycol, polypropylene glycol, ethylene glycol, diethylene glycol, triethylene glycol, polyethylene glycol, ethylene glycol mono-alkyl ether, diethylene glycol mono-alkyl ether and 1,3-butylene glycol.

Examples of more preferable solvents are polyhydric alcohol such as glycerin, diglycerin, polyglycerin, propylene glycol, polypropylene glycol, ethylene glycol, diethylene glycol, triethylene glycol, polyethylene glycol, polyethylene glycol (PEG), benzyl benzoate, triglycerides, acetone, benzyl alcohol, ethanol, ethyl lactate, propylene carbonate and Dimethyl Sulfoxide, 1-butanol, 2-butanol, Tert-butylmethyl ether, Ethyl ether, Ethyl formate, Heptane, 3-Methyl-1-butanol, Methylisobutylketone, 2-Methylisobutylketone, 2-Methyl-l-propanol, Pentane, 1-Pentanol, 1-Propanol, 2-Propanol.

**Oils of the ICell solution (also referred to as co-solvent):** The composition of the invention may further comprise a co-solvent. The co-solvent may be selected from one or more lipids, monoglycerides, diglycerides or triglycerides. The oils optionally included in the ICell solution are hydrophobic substances that mix poorly with aqueous media. Upon injection of a ICell solution into tissue or aqueous media, the oil (co-solvent) and gel-forming carbohydrate ester are separated from the solvent due to NIPS. During this phase separation, the oil (co-solvent) carbohydrate ester mixture forms a gel, gel depot or ICell with properties governed by the oil (co-solvent) and carbohydrate ester. The co-solvent is important for modulating and controlling the release rate of APIs from ICell.

Examples of oils (co-solvents) include, but are not limited to glycerol trivalerate, glycerol trihexanoate (GTH), glycerol trioctanoate (GTO), glycerol tridecanoate (GTD), ethyl octanoate, ethyl hexanoate, ethyl decanoate, Ethyl myristate, ethyl laurate, ethyl oleate, ethyl palmitate, ethyl myristate, corn oil, peanut oil, coconut oil, sesame oil, cinnamon oil, soybean oil, and poppyseed oil, or Lipiodol and aliphatic alkyl acyl esters.

**Gel-forming carbohydrate esters of the ICell solution:** Carbohydrate esters comprise the ICell forming constituents of the ICell solution. Upon solvent efflux caused by Non-solvent Induced Phase Separation (NIPS), the carbohydrate esters alone form viscous fluid depots, amorphous solid depots, crystal solid depots or mixtures thereof.

The gel-forming carbohydrate esters are suitable based on any of the following structures:

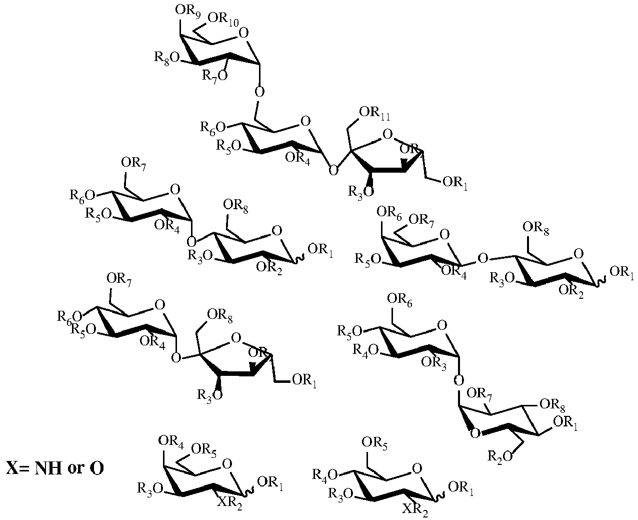

These are schematic views of chemical structures of exemplary carbohydrate esters, wherein R₁, R₂, R₃, R₄, R₅, R₆, R₇, R₈, R₉, R₁₀, and R₁₁ are selected collectively from the group consisting of hydrogen, methyl, alkanoyl, hydroxyl-substituted alkanoyl, and acyloxy-substituted alkanoyl, alkanyl, hydroxy-substituted alkanyl, acyloxy substituted alkanyl, benzoyl, and substituted benzyol; or wherein R₁, R₂, R₃, R₄, R₅, R₆, R₇, R₈, R₉, R₁₀, and R₁₁ are independently selected from the group consisting of hydrogen methyl alkanoyl, hydroxyl-substituted alkanoyl, cyloxy-substituted alkanoyl, alkanyl, hydroxysubstituted alkanyl, acyloxy substituted alkanyl, benzoyl and substituted benzoyl;or wherein all groups of R₁, R₂, R₃, R₄, R₅, R₆, R₇, R₈, R₉, R₁₀, and R₁₁ are selected collectively from the group consisting of methyl, acetyl, isobutyryl, propionyl or benzoyl; or wherein R₁, R₂, R₃, R₄, R₅, R₆, R₇, R₈, R₉, R₁₀, and R₁₁ are independently selected from the group consisting methyl, acetyl, isobutyryl, propionyl or benzoyl;
and wherein both pure anomers and mixtures of α- and β- anomers of the above mentioned structural variations are claimed.

Examples of gel forming carbohydrate esters are carbohydrate ester analogues based on mono-, di-, and tri-saccharides such as but not limited to Glucose (dextrose), Fructose (levulose), Galactose, Sibose, Xylose, such as but not limited to Sucrose, Lactulose, Maltose, Trehalose, Cellobiose, Chitobiose, Isomaltise, such as but not limited to Nigerotriose, Maltotrios, Melezitose, Maltotriulose, Raffinose and Kestose.

Examples of carbohydrate esters include but are not limited to acetate, propionate, butyrate, iso-butyrate and benzoate esters and Sucrose, Lactose, Trehalose, Raffinose, and Maltose, such as Sucrose octaacetate, Sucrose octapropionate (SOP), Sucrose acetate isobutyrate (SAIB), Sucrose octaisobutyrate (SOIB), Sucrose octabenzoate (SuBen), Lactose octapropionate (LOP), lactose octaisobutyrate (LOIB), Lactose octabenzoate (LacBen), Rafinose undecapropionate (RUP), Rafinose undecaisobutyrate (RUIB), Rafinose undecabenzoate (RaBen), Trehalose octapropionate (TOP), Trehalose octaisobutyrate (TOIB), Trehalose octabenzoate (TreBen), Maltose octapropionate (MOP), Maltose octaisobutyrate (MOIB), Maltose octabenzoate (MaBen), MeLOIB (Methyl hepta-O-isobutyryl-α,β-lactoside), and the like.

The carbohydrates may be fully or partially functionalized/esterified with small organic acids such as but not limited to actetate, propanoic acid, butyrate, isobutyrate, valerate, iso-valerate, benzoic acid or mixtures thereof.

Most preferred carbohydrate esters are Lactose octapropionate (LOP), lactose octaisobutyrate (LOIB), Lactose octabenzoate (LacBen), methoxy-LOIB (meLOIB), Raffinose undecabenzoat (RaBen), Raffinose undecaisobutyrate (ROIB), Sucrose octaisobutyrate (SOIB), Trehalose octaisobutyrate (TOIB) and Sucrose octabenzoate (SuBen).

### Computed Tomography (CT) imaging agents and fluorescence

ICell formulations may contain lipiodol as a CT contrast oil (co-solvent) or CLA-8 (α-, β-Lactose octa para-iodobenzoate) or xSAIB (6,6'-(2,4,6-triiodophenoxy)acetoxy-isobutyric-sucrose) ca. This may serve as radiographic imaging agents and may be used as fiducial markers for guiding therapeutic interventions, including, but not limited to, external beam radiotherapy or surgical procedures. Radiographic contrast in ICell may generally be used for CT, radiography or fluoroscopy and guide injection, installation, administration, and smearing of the ICell. Alternative CT imaging agent are lactose or sucrose octa para or meta iodobenzoate. In addition to the optional radiographic contrast the ICell has inherent magnetic resonance contrast and can also be appreciated during ultrasound imaging following the absence of water in the ICell composition. Inclusion of fluorescent molecules or nucleotides encoding the transcription of peptide-based fluorophores can furthermore allow for identification of the ICell position and transcriptional activity by fluorescence imaging e.g., near infrared imaging (NIR) which is becoming increasingly applied for diagnostic and therapeutic interventions in clinical procedures.

### Other constituents of the ICell solution

In one embodiment a polymer may be used to work as a stabilizer between the ICell and biological surrounding and therefore, the composition may also comprise a molecule that increase ICell stability in the human or animal body, such as an amphiphilic molecule, such as an emulsifier. Therefore, in one embodiment the composition comprises poly(ethylene glycol-b-caprolactone) (PEG-PCL), sucrose acetate isobutyrate (SAIB), poly(*D,L*-lactic acid) (PLA), or poly(lactic-co-glycolic acid) (PGLA), or a combination thereof. In one embodiment of the present invention poly(*D,L*-lactic acid) (PLA) is added to the non-water-soluble carbohydrate causing a reduction of burst release of said encapsulated contents e.g., drugs, particles, imaging agents, etc. The formulation may further include other constituents, such as α-, β-, and/or γ-cyclodextrins and any derivate hereof.

The formulation may further comprise compounds or polymers, which are visible in imaging modalities other than radiography-based modalities.The ICell formulation that as such has a comparable low viscosity is intended for injection in the body of a human or animal, where after the formulation becomes more viscous, i.e. it goes through a sol-gel transition (liquid to gel) transition, due to the presence of the gel-forming system. It is preferred that the viscosity of the formulation after injection in the body of a human or animal increases by at least 50 %, such as at least 80 %, such as at least 100 %, or at least 150 %, or at least 200 %, or at least 300 %, or at least 500 %, or at least 750 %, or at least 1000 %, or at least 10,000%, or that the formulation becomes essentially solid (non-viscous). The formulation is preferably adapted for injection via a thin needle used for injection into a body or surgical related procedures, such as but not limited to biopsy. The viscosity of the gel-forming formulation before injection can be any suitable viscosity such that the formulation can be parenterally administered to a patient. Exemplary formulations include, but are not limited to, those having a viscosity (prior to administration/injection) lower than 10,000 centipoise (cP), e.g. lower than 2,000 cP, such as 10 to 2,000 cP, such as 20 to 1,000 cP, such as 150 to 350 cP, such as 400 to 600 cP, such as 600 to 1,200 cP or such as 1,000 to 2,000 cP, or 10 to 600 cP, or 20 to 350 cP, at 20 °C. Alternative formulations include, but are not limited to, those having a viscosity (prior to administration/injection) lower than 10,000 centipoise (cP), e.g. lower than 2,000 cP, such as 10 to 2,000 cP, such as 20 to 1,000 cP, such as 150 to 350 cP, such as 400 to 600 cP, such as 600 to 1,200 cP or such as 1,000 to 2,000 cP, or 10 to 600 cP, or 20 to 350 cP, at 5 °C. When referred to herein, the (dynamic) viscosity is measured at the specified temperature in accordance with the method described in ASTM D7483.

### Pharmaceutical agent of the ICell solution

The gel forming ICell composition may further comprise pharmaceutical agents including prodrugs (in short "drugs"; broadly interpreted as agents which are able to modulate the biological processes of a human or animal). These drugs, prodrugs, HIPs or the like can be formulated as a single drug or as a combination of two or more in the ICell composition.

The ability to HIP complex a broad range of therapeutic drug classes for incorporation and release from ICell technology has important clinical applications and indications.

Antimicrobial agents represent attractive agents to have a controlled and sustained loco-regional release at the sites of injection. This may improve therapeutic efficacy, maintain therapeutic drug concentration at infection sites and reduce risks for developments of drug resistance. HIP complexing of antimicrobial agents in the ICell may improve release properties and provide an improved stability of the antimicrobial agent(s). The combination of multiple antimicrobial agents may further allow for optimizations and/or synchronization of release characteristics and for achieving synergistic therapeutic efficacy. The HIP complexing in ICell may furthermore provide optional combination of antimicrobial agents and therapeutics that have synergistic or additive effects.

Cancer immunotherapy is demonstrating increasing therapeutic potential with novel targets and agent constantly arising. However, some of the most important immune activating compounds and technologies are very poorly tolerated and as such they should optimally be targeted only to the site where immune activation is intended to take place. Considering that the anticancer immune response is generated in tumors, peritumoral and lymphatic tissue makes technologies for providing sustained intratumoral drug release highly attractive. HIP complexing of immunotherapeutic drugs and drug combinations is highly promising to achieve optimal and tolerated anti-cancer immune activation.

Vaccine technologies generally suffers from the requirement of repeated administration to generate a sufficiently strong and durable immune response. The HIP complexing of vaccine antigens in the ICell may improve release properties and provide an improved stability of the antigens in order for improving immune stimulation and duration. The ICell technology furthermore allow for sustained release of optimal adjuvant combinations which can further enhance the vaccine response. The HIP complexing of vaccines in the ICell technology may therefore provide more effective single injection vaccinations with the booster injections needed for current vaccine technologies.

The ability to provide sustained release and stimulation is attractive across a range of disease and disorders, including, hormonal diseases, regenerative disorders, cardiovascular, and metabolic diseases. Across several diseases the therapeutic intervention requires a continuous stimulation or inhibition of biological activites. In these cases, HIP complexing of suitable therapeutics or therapeutic combinations in the ICell formulation may provide a possibility to achieve more stable dosing, minimize dose fluctuation, reduce administration intervals and reduce adverse events by securing biologically adapted, systemic or localized activity. The HIP complexing may furthermore stabilise biologics and secure optimal tailoring of ICell drug release kinetics. This may be indicated for, but not limited to, hormonal supplementation, metabolic diseases, endocrine disorders, transplantions, autoimmunity, enzyme replacement, storage diseases and tissue regeneration.

CT and radiographic imaging agents, such as but not limited to the CT imaging agent Diatrizoate, Acetrizoic acid, iodamide and the like may form hydrophobic complexes via hydrophobic ion parring rendering these soluble in organic media such as but not limited to oils, carbohydrate ester gels and ICell compositions.

MRI imaging agents such as Gadoteric acid, gadopentetate, gadobenate, gadoxentate and the like may form hydrophobic complexes via hydrophobic ion parring rendering these soluble in organic media such as but not limited to oils, carbohydrate ester gels and ICell compositions.

Chemical variants of fluorophores, chromophores and coloured agents such as methylene blue (MB), indocyanine green (ICG) may form hydrophobic complexes via hydrophobic ion parring rendering these soluble in organic media such as but not limited to carbohydrate ester gels and ICell compositions.

Compositions containing CT or MR contrast, fluorophores or chromophores are important for image guided injection, or localization of ICells used as fiducial markers or as drug delivery depots or for guiding interventional procedures. Hydrophobic ion parring of such agents expands the range of modalities available for imaging of ICells.

### Embodiments of a first aspect of the present invention

With reference to an imaging application, below there is provided some embodiments of a first aspect of the present invention.

According to one embodiment, the composition further comprises a co-solvent selected from one or more lipids, monoglycerides, diglycerides or triglycerides.

According to yet another embodiment, the composition further comprises one or more polymers or copolymers.

According to one specific embodiment, the counterion reduces or enhances the release rate of the imaging agent from the ICell compared to a ICell composition without the counterion.

Furthermore, according to yet another embodiment, the imaging agent is a fluorescent imaging agent. It should of course be noted that all other type of imaging agents are possible to include according to the present invention.

Moreover, according to one embodiment, the imaging agent is a fluorescent imaging agent or an imaging agent, which has one or more ionizable groups or moieties, preferably the ionized group is one or more amines (primary, secondary, tertiary or quaternary), alcohols, carboxylic acids, thiols, sulphonic, sulphenic or sulfinic acids, tetraalkyl phosphonium, phosphonic, phosphenic, or phosphinic acids.

According to one specific embodiment, the fluorescent imaging agent is methylene blue (MB), indocyanine green (ICG), or the imaging agent is an MRI imaging agent, preferably being Gadoteric acid, gadopentetate, gadobenate or gadoxentate.

Furthermore, according to one embodiment, the fluorescent imaging agent is methylene blue (MB), indocyanine green (ICG) and wherein the counterion is selected from Tetraethyl ammonium bromide, Dodecylamine, Cetyl trimethyl ammonium bromide (CTAB), Tetraoctyl ammonium bromide (TOAB), N-benzyl-2-phenylethanamine (benethamine, BENA).

Moreover, according to one embodiment, the imaging agent and the hydrophobic or amphiphilic acid or base, or salt thereof have different charge ratios.

Furthermore, according to yet another embodiment, the counterion is selected from any of the class of ionic detergents, ionic lipids and lysolipids, fatty acids and ionic polymers; or wherein the counterion is selected from any of one or multiple ionizable groups belonging to the group of amines (primary, secondary, tertiary or quaternary), alcohols, carboxylic acids, thiols, sulphonic, sulphenic or sulfinic acids, tetraalkyl phosphonium, phosphonic, phosphenic, or phosphinic acids, and conjugated systems with delocalized charges.

The present invention may also provide a combined composition comprising both an imaging agent and a pharmaceutical agent. In line with this, according to one embodiment, the composition further comprises a pharmaceutical agent.

Moreover, for the method according to the first aspect, according to one embodiment of the present invention, the HIP is formulated in an ICell and/or as a powder.

According to yet another embodiment, the first liquid is water; wherein the hydrophobic counterion is water soluble and the second liquid is water; or wherein the hydrophobic counterion is weakly water-soluble and the second liquid is chosen from methanol, Ethanol, chloroform, DCM, DMSO, PC (propylene carbonate), iso-propanol, BnOH (benzyl alcohol) or an organic solvent being mixable with water.

Furthermore, according to yet another embodiment, the method has an additional step a') adjusting the pH of the solution such that the charge of the imaging agent or pharmaceutical agent and the hydrophobic counterion is non-zero and have opposing charge, wherein a') is performed between step a) and b).

Moreover, according to one embodiment, the mixing of step c) is performed in the presence of chloroform or DCM and wherein the formation of step d) is performed through HIP partition into an organic phase.

### Embodiments of a second aspect of the present invention

Below there is provided embodiments of the second aspect of the present invention.

According to one embodiment of the second aspect, the hydrophobic or amphiphilic acid or base or salt thereof reduces or enhances the release rate of the pharmaceutical agent from the ICell compared to a ICell composition without the hydrophobic or amphiphilic acid or base or salt thereof.

According to yet another embodiment, the composition further comprises a co-solvent selected from one or more lipids, monoglycerides, diglycerides or triglycerides.

Moreover, also in this case the composition may further comprise one or more polymers or copolymers.

Furthermore, according to yet another embodiment, the pharmaceutical agent is a small molecule drug, peptide, or protein. Moreover, according to one embodiment the small molecule drug is one or more anticancer agents, immunotherapeutic, chemotherapeutics, antimicrobials (antibiotics, antivirals, antifungals, antiprotozoals, and antihelminthics), anti-inflammatory agents, anticoagulant, antidepressant, antiepileptic, antipsychotic, sedatives, antidiabetic, cardiovascular, and analgesic agents; wherein the peptide is one or more therapeutic peptides, agonistic and antagonistic receptor ligands, peptides with regulatory or enzymatic activity, hormones, peptides with special targeting activities, vaccines, antigens, therapeutic peptides, diagnostic peptides, steroids; wherein the protein is one or more immunotherapeutic agonists and antagonists, growth factors, cytokines, chemokines, hormones, glycoprotein hormones and antibodies, affibodies, peptide and nanobodies.

According to one embodiment of the present invention, the pharmaceutical agent and the hydrophobic or amphiphilic acid or base, or salt thereof have different charge ratios.

Moreover, according to one embodiment of this second aspect, the hydrophobic or amphiphilic acid or base, or salt thereof is selected from any of the class of ionic detergents, ionic lipids and lysolipids, fatty acids and ionic polymers.

Furthermore, according to one embodiment the hydrophobic or amphiphilic acid or base, or salt thereof is selected from one or multiple ionizable groups belonging to the group of one or more amines (primary, secondary, tertiary or quaternary), alcohols, carboxylic acids, thiols, sulphonic, sulphenic or sulfinic acids, tetraalkyl phosphonium, phosphonic, phosphenic, or phosphinic acids, and conjugated systems with delocalized charges.

Also in this case the same type of method embodiments are relevant as mentioned above.

### Further embodiments of the present invention and both aspects thereof HIP Physicochemical properties

In one embodiment, a hydrophobic ion-pair (HIP) is formed from an ionizable said cationic, anionic or zwitterionically charged API, excipient, imaging agent, or combinations thereof and a hydrophobic or amphipathic counterion, and the HIP is solubilized or dispersed in a ICell composition.

In one embodiment of the disclosure, a HIP is formed using counterions/co-ions that are hydrophobic or amphiphilic acids or bases, or salts thereof, and the HIP is solubilized or dispersed in a ICell composition.

In one embodiment, a HIP is formed, and the HIP is solubilized or dispersed in a ICell composition.

In one embodiment, a HIP is formed from an API, excipient, imaging agent, or combinations thereof containing one or more ionizable groups and a counterion, and the HIP is formulated said solubilized in a ICell composition.

In one embodiment of the disclosure, a HIP is formed from an API, excipient, imaging agent, or combinations thereof and a counterion containing one or more ionizable groups and multiple counterions, and the HIP is formulated said solubilized in a ICell composition.

In one embodiment, a HIP is formed from a charged, such as but no limited to cationic, anionic or zwitterionic, API, excipient, imaging agent, or combinations thereof and a hydrophobic counterion comprising one or multiple hydrophobic domains, and the HIP is formulated said solubilized in a ICell composition.

In one embodiment, a HIP is formed from a charged hydrophobic, charged amphipathic or charged hydrophilic API, excipient, imaging agent, or combinations thereof and a counterion, and the HIP is formulated said solubilized in a ICell composition.

In one embodiment, a HIP is formed from a charged API, excipient, imaging agent, or combinations thereof and one or multiple counterions, and the HIP is formulated said solubilized in a ICell composition.

In one embodiment, a HIP is formed from a charged API, excipient, imaging agent, or combinations thereof and one or multiple counterion combinations, and the HIP is formulated said solubilized in a ICell composition.

In one embodiment, a HIP is formed from a charged API, excipient, imaging agent, or combinations thereof and one or multiple counterions at different charge ratios, and the HIP is formulated said solubilized in a ICell composition.

In one embodiment, HIP complexes formulated in ICells comprises hydrophobic counterions selected from the class of ionic detergents, ionic lipids and lysolipids, fatty acids and ionic polymers.

In one embodiment of the disclosure, HIP complexes formulated in ICells comprises hydrophobic counterions with one or multiple ionizable groups belonging to the group of amines (primary, secondary, tertiary or quaternary), alcohols, carboxylic acids, thiols, sulphonic, sulphenic or sulfinic acids, tetraalkyl phosphonium, phosphonic, phosphenic, or phosphinic acids, phosphorothioates and or conjugated systems with delocalized charges, and the like.

In one embodiment of the disclosure, HIP complexes formulated in ICells comprises hydrophobic counterions with one or multiple ionizable groups belonging to the group of alcohols, carboxylic acids, amines, organophosphorus compounds, or organosulfur compounds functionalized with one or multiple aliphatic or aromatic C4-C22 hydrocarbon chains, or preferable functionalized with one or multiple aliphatic or aromatic C8-C16 hydrocarbon chains, or yet more preferable functionalized with one or multiple aliphatic C8-C12 hydrocarbon chains.

In one embodiment of the disclosure, HIP complexes formulated in ICells comprises hydrophobic counterions with one or multiple ionizable groups belonging to the group of alcohols, carboxylic acids, amines, organophosphorus compounds, or organosulfur compounds functionalized with one or multiple aliphatic or aromatic C4-C22 hydrocarbon chains, such as but not limited to methanol, ethanol, propanol, butanol, hexanol, octanol, decanol, dodecanol, phenol, hydroquinone, catechol, resorcinol, and the like. acid Acetic acid, propanoic acid, butanoic acid, hexanoic acid, octanoic acid, benzoic acid, Myristic acid, Myristoleic acid, Palmitic acid, Palmitoleic acid, Stearic acid, Oleic acid, Elaidic acid, Linoleic acid, Linolenic acid, Linolelaidic acid, Arachidonic acid, Elauricacid acid, Mycolic Acids, α-mycolic acid (C80), α-mycolic acid, methoxy cis, α-mycolic acid, keto cis, Cyclopropyl Lipids, Cis-9,10-methylenehexadecanoic acid, 1-palmitoyl-2-cis-9,10-methylenehexadecanoyl-sn-glycero-3-phosphocholine, 1-palmitoyl-2-cis-9,10-methylenehexadecanoyl -sn-glycero-3-phosphoethanolamine, (s)-12-methyltetradecanoic acid, 13-methyltetra-decanoic acid, cis-9-oleicacid, cis-9- octadecenoicacid, trans-9-octadecenoicacid, (2-16)-octadecenoicacid, , 2-tridecenoic acid, 11-tridecenoic acid, 12 tridecenoicacid,2-dodecenoicacid, 5-dodecenoicacid, 6-dodecenoic acid, 7-dodecenoic acid, 9-dodecenoic, 10-dodecenoic acid, 11-dodecenoic acid, 11-eicosenoic acid, 14-eicosenoic acid, 2-undecenoic acid, 6-undecenoic acid, 9-undecenoic acid, 10 undecenoicacid, 2-decenoicacid, 3-decenoicacid, 8-decenoicacid, 9-decenoicacid, acrylic acid, A-methylacrylic acid, vinyl acetic acid, b-b-dimethylacrylic acid, beta-pentenoic acid, alylacetic acid, angelic acid, tiglic acid, 2-heptadecenoic, 9-heptadecenoic acid, 2-hexadecenoic acid, 9-hexadecenoicacid(cis form);2-tetradecenoicacid, 4-tetradecenoicacid, radecenoicacid, 8-tetradecenoicacid;9-tetradecenoic acid; 2-nonenoicacid, 3-nonenoicacid, 8-nonenoicacid, 2-octenoicacid, 3-octenoicacid, 7-octenoicacid, 2-hep tenoicacid, 3-heptenoicacid;4-heptenoicacid, 5-hep tenoicacid, 6-heptenoicacid, 2-hexenoicacid, 3-hexenoicacid, 4-hexenoicacid, 5-hexenoicacid,15-tetracosenoicacid;17-hexacosenicacid, 21-triacentenoic acid, 2- to 8-nonynoic acid, Octadecadienoic acid 8:10, oleic acids (i.e.,cis-9-oleicacidorcis-9-octadecenoicacid), octadecadienoicacid,(8-trans and 10-transforms), 8:11 acidsiselaidicacid(i.e.,trans-9-octadecenoicacid), octadecadienoicacid,(8-cis and 11-cis forms), 9:11 octadecadienoicacid,(9-cis and 11-cis and I-trans forms), 5:12-octadecadienoic acid, (5-cis, 5-trans, 12 trans and 12-cisforms), 9:12-octadecadienoic acid,(9- cis, 9-trans, 12-trans and 12-cis forms), 10:12 octadecadienoic acid, (10-cis, 10-trans, 12-cis and 12 trans forms), 10:13-octadecadienoic acid, (10-cis and 13-cisforms), 11:14-octadecadienoicacid,(11-cis and 14-cisforms), beta-vinylacrylic acid, sorbic acid, geranicacid, tetra-triethenoidfaty acids, triethenoidfaty acid, clupandoic acid, moroctic acid, arachidonic acid, alpha and beta parinaric acids, oleicacid, linoleicacid,licanic acid, eleostearic acid, ricinoleicacid, clupanodonicacid , palmitoleic acid, eleostearic acid, cis-11-methyl-2-dodecenoic acid, trans-2-decanoic acid (T2DA), heptylcyclopropane-1-carboxylic acid (2CP), palmitic acid, Cis-2-decanoic acid (C2DA), palmitoleic acid, Palmitelaidic acid, 7(Z),10(Z)-Hexadecadienoic acid, Linoleic acid, γ-Linolenic acid, Arachidonic acid, chenodeoxycholic acid, deoxycholic acid, formic acid or hexanoic acid, and the like. 1-Hydroxy-2-naphthoic acid (xinafoic acid), 2-Naphthalene sulfonic acid (NSA), Brilliant blue FCF, Carboxy methyl polyethylene glycol (CM-PEG), Cholesteryl hemisuccinate, Cholic acid (sodium cholate), Decanoic acid (sodium decanoate/sodium caprate), Docosahexaenoic acid, Hexadecylphosphate, Linoleic acid, N,N-Dipalmitoyl-L-lysine, Oleic acid (sodium oleate also used), Pamoic (disodium pamoate also used), acetate, cholesteryl sulfate, Sodium decanesulfonate (SDES), deoxycholate, docusate (AOT, dioctyl sulfosuccinic acid, bis-2- ethylhexylsulfosuccinate), dodecyl benzenesulfonate (SDBS), dodecyl sulfate (sodium lauryl sulfate), laurate (sodium dodecanoate), n-octadecyl sulfate (sodium stearyl sulfate), stearate (stearic acid also used), stearoyl glutamate (SSG), taurodeoxycholate (STDC), tetradecyl sulfate, tripolyphosphate, Taurocholic acid (sodium taurocholate also used), Vitamin E (a-tocopherol) succinate, Arginine-hexadecanoyl ester (AHE), Arginine-nonyl ester (ANE), Benethamine (N-benzyl-2-phenylethanamine), Dodecylamine (laurylamine), Hexadecyl trimethylammonium(cetrimonium) bromide (CTAB), Maprotiline, Na-Deoxycholyl-L-lysyl-methylester, N,N'-Dibenzyl ethylenediamine(benzathine), N,N-Dimethyl dodecylamine (DDA), N,N-Dimethyl hexylamine, N,N-Dimethyl octadecylamine(dimethyl stearamine), Stearylamine-(octadecylamine), Tetrabutyl ammonium bromide (TBAB), Tetraheptyl ammonium bromide (THA), Tetrahexyl ammonium bromide, Tetraoctyl ammonium bromide (TOAB), Tetrapentyl ammonium bromide (TPA), Triethylamine (TEA), Cholesteryl, hemisuccinate, Sulfonated Cholesterol, 25-hydroxycholesterol-3-sulfate, cholesterol 3-sulfate, Dehydroepiandrosterone sulfate and the like.

In one embodiment of the disclosure, HIP complexes formulated in ICells comprises hydrophobic counterions with one or multiple ionizable groups belonging to the group of bile-acids, such as but not limited to Cholic Acids, 3α,6α,7α-trihydroxy-5ß-cholanic acid, 3α,6ß,7α,12α-tetrahydroxy-5β-cholan-24-oic acid, 5β-cholanic acid-3β-ol, 5α-cholanic acid-3β-ol, 5β-cholanic acid-3α,12α-diol, 5β-cholanic acid-3α,7α-diol, 5β-cholanic acid-3α,7β-diol, 5β-cholanic acid-3α,7α,12α-triol, 5α-cholanic acid-3α,7α,12α-triol, 5β-cholanic acid-3α,6α-diol, 5β-cholanic acid-3,7,12-trione, 5β-cholanic acid-3α,12α-diol-7-one, 5β-cholanic acid-3-one, 5β-cholanic acid-3α-ol, 5ß-cholanic acid-7α,12α-diol, 3α,6α,7α,12α-tetrahydroxy-5ß-cholan-24-oic acid, 3α,7ß,2α-trihydroxy-5ß-cholan-24-oic acid, 5β-cholanic acid-3α-ol-7,12-dione, 5ß-cholanic acid-3α,6α,7ß-triol, 5β-cholanic acid-3α,6β,7α-triol, 5β-cholanic acid-3α,6β,7β-triol, 5ß-cholanic acid-3α-ol-6,7-dione, 5β-cholanoic acid-3α,7α-diol-12-one, 5β-cholanoic acid-7α,12α-diol-3-one, 5ß-cholanoic acid-3α-ol-7-one, 5β-cholanoic acid-3α-ol-12-one, 23-nor-5β-cholanoic acid-3α, 12α-diol, 8(14),5β-cholenoic acid-3α,12α-diol, 5β-cholanic acid-12α-ol-3-one, 5β-cholanic acid-7α-ol-3-one, 5ß-cholanic acid-3α,6ß-diol, 5ß-chol-14-enoic acid-3α,12α-diol, Including, but not limited to, cholic acid sulfates, glycocholic acids, taurocholic acids and functionalized bile acids.

In one embodiment, HIP complexes formulated in ICells comprises hydrophobic counterions with one or multiple ionizable groups belonging to the group of lipids comprising phosphatidic acid (PA), cyclic PA, lysophosphatidic acid (LPA), cyclic LPA, phosphatidylglycerol (PG), lysophosphatidylglycerol (LPG), phosphoinositides (PI), lysophosphatidylinositol (LPI), phosphatidylserine (PS), or Lysophosphatidylserine (LPS) phospholipids and or ether lipids, or sphingolipids (SP) or sphingolysolipids (LSP), all with mixed or non-mixed, saturated and or unsaturated, aliphatic and or aromatic, C6-C22 hydrocarbon chains. Preferred examples of hydrocarbon chains are but not limited to propionyl, butyryl, hexanoyl, octanoyl, decanoyl, lauroyl, myristoyl, palmitoyl, stearoyl, arachidoyl, behenoyl, lignoceroyl and the like. Other preferred examples of hydrocarbon chains are vaccenoyl, (8Z)octadecenoyl, myristoleoyl, myristelaidoyl, palmitoleoyl, palmitelaidoyl, oleoyl, dielaidoyl and the like.

In one embodiment, HIP complexes formulated in ICells comprises hydrophobic counterions with one or multiple ionizable groups belonging to the group of lipids comprising PA, LPA, PG, LPG, PI, LPI, PS, or LPS phospholipids, or SP or LSP all with mixed or non-mixed, saturated or unsaturated, sterol modified, fatty acid modified, or headgroup modified such as but not limited to PEGylated, fluorophore or chelator functionalized. Functionalized here includes but is not limited to deuteration, fluorescence, biotinylation, diphytanoylation, bromination, oxidization, diacetylenation, fluorination, and modifications with bioactive molecules.

In one embodiment, HIP complexes formulated in ICells comprises hydrophobic counterions with one or multiple ionizable groups belonging to the group of multivalent cationic lipids such as but not limited to N1-[2-((1S)-1-[(3-aminopropyl)amino]-4-[di(3-amino-propyl)amino]butylcarboxamido)ethyl]-3,4-di[oleyloxy]-benzamide or N4-Cholesteryl-Spermine.

In one embodiment, HIP complexes formulated in ICells comprises hydrophobic counterions with one or multiple ionizable groups belonging to the group of ionizable cationic lipids such as but not limited to DOBAQ (N-(4-carboxybenzyl)-N,N-dimethyl-2,3-bis(oleoyloxy)propan-1-aminium), 1,2-distearoyl-3-dimethylammonium-propane, 1,2-dipalmitoyl-3-dimethylammonium-propane, 1,2-dimyristoyl-3-dimethylammonium-propane, 1,2-dioleoyl-3-dimethylammonium-propane (DODAP), 1,2-dioleyloxy-3-dimethylaminopropane (DODMA), DLin-DMA, DLin-KC2-DMA, DLin-KC3-DMA, C12-200, A6, OF-02, A18-Iso5-2DC18, YSK05, 7C1, G0-C14, L319, OF-Deg-Lin, 306-012B, 306O₁₁₀, FTT5, 9A1P9, 98N₁₂-5, 304O₁₃, cKK-E12 and the like.

In one embodiment, HIP complexes formulated in ICells comprises hydrophobic counterions with one or multiple ionizable groups belonging to the group of fixed cationic lipids such as but not limited to 3ß-[N-(N',N'-dimethylaminoethane)-carbamoyl]cholesterol (DC-Cholesterol), N-(2-hydroxyethyl)-N,N-dimethyl-2,3-bis(oleoyloxy)propan-1-aminium (DORI), O,O'-ditetradecanoyl-N-(α-trimethylammonioacetyl)diethanolamine, ethylphosphocholines (EPCs) of lauroyl, myristoyl, palmitoyl, stearoyl, and oleoyl. Such as Dimethyldioctadecylammonium, 1,2-dimyristoyl-3-trimethylammonium-propane, 1,2-dipalmitoyl-3-trimethylammonium-propane, 1,2-stearoyl-3-trimethylammonium-propane and 1,2-dioleoyl-3-trimethylammonium-propane. Such as 1,2-di-O-octadecenyl-3-trimethylammonium propane.

In one embodiment, HIP complexes formulated in ICells comprises hydrophobic counterions from the list hexanoate, decanoate, myristate, oleate, Pamoic acid, deoxycholate, Benezenesulfonic acid monohydrate, 2-naphthalenesulfonate, Camphorsulfonic acid, 1,2-ethanesulfonate, 1-heptanesulfonate, 1-octane-sulfonate monohydrate, 1-decanesulfonate, dodecyl sulfate (SDS), dodecyl-benzenesulfonate (DBS), Linoleic acid, Cis-2-decenoic acid, docusate, Curcumin, Tetraethyl ammonium bromide, Benzyl trimethyl ammonium chloride, Cetyl trimethyl ammonium bromide (CTAB), N-benzyl-2-phenylethanamine (benethamine), Dodecylamine, Tetrahexyl ammonium bromide (THAB), Didodecyl dimethyl ammonium bromide (DDAB), Octadecylamine, Tetraoctyl ammonium bromide (TOAB), Tridodecyl methyl ammonium chloride (TDMAC) and the like.

In one embodiment, HIP complexes formulated in ICells comprises charged polymers from the class such as but not limited to polyethyleneimine (PEI), polylysine (PLL), polyarginine (PAA), Chitosans, Poly(2-ethyl-2-oxazoline) (ULTROXA), Diethylaminoethyl-dextran (DEAE-dextran), dendritic polyamidoamine (PAMAM), Poly-beta-amino-esters (PBAE) and PDMAEMA [poly(N,N-dimethylaminoethyl methacrylate].

### HIP formation and formulation in ICell

In one embodiment, a HIP is formed as a powder from an API, excipient, imaging agent, or combinations thereof, and the HIP powder is formulated said solubilized in a ICell composition.

In one embodiment, a HIP is formed from an API, excipient, imaging agent, or combinations thereof and a counterion and is isolated from an organic or aqueous phase, and the HIP is formulated said solubilized in a ICell composition.

In one embodiment, a HIP is formed from an API, excipient, imaging agent, or combinations thereof and a counterion, and the HIP is added to a ICell composition from an organic phase.

### Controlled release or retention

In one embodiment, a HIP with high hydrophobicity or molecular size is formed from a charged API, excipient, imaging agent, or combinations thereof and a counterion, that provides high retention of the HIP in the ICell composition upon injection into tissue or aqueous media.

In one embodiment, a HIP with high hydrophobicity is formed from a charged API, excipient, imaging agent, or combinations thereof and a counterion which provides high retention of the HIP in the ICell composition upon injection into tissue or aqueous media.

In one embodiment, ICell compositions comprising fluorophores such as but not limited to ICG or MB HIP complexes have high retention in ICell upon injection into tissue or aqueous media.

In one embodiment, ICell compositions comprising ICG or MB HIP complexes are used for image guided surgical intervention.

In one embodiment, SAIB:xSAIB:EtOH, LOIB:xSAIB:EtOH, LOIB:LacBen:xSAIB:EtOH or LOIB:CAB:xSAIB:EtOH, SuBen:SAIB:xSAIB:EtOH, SuBen:Ethyl-palmitate:EtOH ICell compositions comprising ICG or MB HIP complexes have high retention of ICG or MB in ICell upon injection into tissue or aqueous media.

In one embodiment, ICell compositions comprising ICG or MB HIP complexes comprising counterions such as but not limited to CTAB, BENA, DDA, and TOAB have high retention of ICG or MB in ICell upon injection into tissue or aqueous media.

In one embodiment, controlled release of HIPs from ICell compositions injected into tissue or aqueous media is obtained by varying the size, charge ratio or hydrophobicity or combination thereof of the counterion of the HIP.

In one embodiment, the release rate of HIPs from ICell compositions injected into tissue or aqueous media is regulated by varying the size, charge ratio or hydrophobicity of the counterion of the HIP.

In one embodiment, slower release of HIPs from ICell compositions injected into tissue or aqueous media is obtained by increasing the size, hydrophobicity, or combinations thereof of the counterion of the HIP.

In one embodiment, ICG or MB HIP complexes are released from ICells, and release rate is governed by the counterion.

In one embodiment of the disclosure, ICG or MB HIP complexes are released from ICells upon administration and ICG or MB accumulates in draining lymph nodes.

In one embodiment, ICG Benethamine (BENA) HIP complexes are released from ICells (SuBen:GTO:EtOH) upon administration and ICG accumulates in the draining lymph nodes.

In one embodiment, faster release of HIPs from ICell compositions injected into tissue or aqueous media is obtained by decreasing the size, hydrophobicity, or combinations thereof of the counterion of the HIP.

In one embodiment, faster release of ICG-BENA (logP BENA 3.6) compared to ICG-TOAB (logP TOAB 8.4) from ICell (SuBen:GTH:DMSO) compositions injected into aqueous media is obtained.

In one embodiment, said release profile for a charged API, excipient, imaging agent or combinations thereof is controlled said modified by embedding two or more HIP complexes of the same charged API, excipient, imaging agent or combinations thereof produced from two or multiple counterions/co-ion with differing release rates.

In one embodiment, two or multiple HIP complexes, formed from differing counterions/co-ions and a single charged API, excipient, imaging agent or combinations thereof, are formulated in a single ICell composition.

In one embodiment, two or multiple HIP complexes, formed from differing counterions/co-ions and a single charged API, excipient, imaging agent or combinations thereof, have different release rates from a ICell.

In one embodiment, two or multiple different HIP complexes of a single charged API, excipient, imaging agent or combinations thereof are formulated in a single ICell composition, and the ratio of HIP complexes with different release rate determines the overall release rate of the charged API, excipient, imaging agent or combinations thereof.

### Dissociation of HIP upon release

In one embodiment of the disclosure, HIPs release from ICell compositions are dissociated upon release.

In one embodiment of the disclosure, HIPs release from ICell compositions remain assembled as HIP complexes.

In one embodiment of the disclosure, the dissociation rate of HIPs release from ICell compositions is governed by the acidity of the ionizable groups on species forming the HIP.

In one embodiment of the disclosure, the dissociation rate of HIPs release from ICell compositions is governed by the pH of the environment where into the HIP is released.

### Achieving diagnostic, additive, or synergistic therapeutic activity through selection of counterion

In one embodiment of the disclosure, ICell comprises a HIP formed from an API, excipient, or imaging agent and a counterion, where the counterion exerts an additive or synergistic pharmacological effect.

In one embodiment of the disclosure, the HIP formulated in ICell comprises an API, excipient, or imaging agent and a counterion that may have, but not limited to, anti-cancer activity, cytotoxic, hormonal effect, anti-microbial activity, tissue regenerative effect (e.g., pro-angiogenic, pro-fibrogenic, pro-osteogenic factors).

In one embodiment of the disclosure, the HIP formulated in ICell comprises an API, excipient, or imaging agent and counterions that are agents with but not limited to radiographic contrast, MRI contrast, NIR or standard fluorescence, chromophores or radiometal chelators.

### Relevant HIP concentrations

In one embodiment, one or multiple HIP complexes are formulated in a single ICell composition.

In one embodiment, one or multiple HIP complexes, and one or multiple native APIs, excipients, imaging agents or combinations thereof are formulated in a single ICell composition.

In one embodiment, one or multiple HIP complexes of APIs are formulated in a single ICell composition at a concentration of 0.01-200 mg per gram ICell, such as 0.1-50 mg per ICell, such as 1-10 mg per ICell.

In one embodiment, one or multiple HIP complexes of excipients are formulated in a single ICell composition at a concentration of 1-300 mg per gram ICell, such as 10-100 mg per ICell, such as 25-50 mg per ICell.

In one embodiment, one or multiple HIP complexes of imaging agents such as fluorophores are formulated in a single ICell composition at a concentration of 0.001-10 mg per gram ICell, such as 0.01-5 mg per ICell, such as 0.1-1 mg per ICell.

In one embodiment, one or multiple HIP complexes of fluorophores are formulated in a single ICell composition at concentration where fluorescent quenching is minimal.

### ICell composition embodiment

In one embodiment, a ICell comprises a HIP complex, a carbohydrate ester and a solvent

In one embodiment, a ICell comprises a HIP complex, a carbohydrate ester, a co-solvent, and a solvent.

In one embodiment, a ICell comprises a HIP complex, a carbohydrate ester, a co-solvent, an imaging agent, and a solvent.

In one embodiment, carbohydrate esters of the ICell are selected from Lactose octapropionate (LOP), lactose octaisobutyrate (LOIB), Lactose octabenzoate (LacBen), methoxy-LOIB (meLOIB), Raffinose undecabenzoat (RaBen), Raffinose undecaisobutyrate (ROIB), Sucrose octaisobutyrate (SOIB), Sucrose acetate isobutyrate (SAIB), Trehalose octaisobutyrate (TOIB) and Sucrose octabenzoate (SuBen) and the like.

In one embodiment, co-solvents of the ICell are selected from glycerol trivalerate, glycerol trihexanoate (GTH), glycerol trioctanoate (GTO), glycerol tridecanoate (GTD), ethyl octanoate, ethyl hexanoate, ethyl decanoate, Ethyl myristate, ethyl laurate, ethyl oleate, ethyl palmitate, ethyl myristate, corn oil, peanut oil, coconut oil, sesame oil, cinnamon oil, soybean oil, and poppyseed oil, or Lipiodol and aliphatic alkyl acyl esters and the like.

In one embodiment, solvents of the ICell are selected from glycerin, diglycerin, polyglycerin, propylene glycol, polypropylene glycol, ethylene glycol, diethylene glycol, triethylene glycol, polyethylene glycol, polyethylene glycol (PEG), benzyl benzoate, triglycerides, acetone, benzyl alcohol, ethanol, ethyl lactate, propylene carbonate and Dimethyl Sulfoxide, 1-butanol, 2-butanol, Tert-butylmethyl ether, Ethyl ether, Ethyl formate, Heptane, 3-Methyl-1-butanol, Methylisobutylketone, 2-Methylisobutylketone, 2-Methyl-l-propanol, Pentane, 1-Pentanol, 1-Propanol, 2-Propanol and the like.

In one embodiment, imaging agents of the ICell are selected from xSAIB, CLA-8, lipiodol and the like.

### ICells for surgical use

In one embodiment one or more HIPs are formulated in ICells comprising SAIB, xSAIB and EtOH or DMSO

In a preferred embodiment one or more HIPs are formulated in ICells with the composition; 30-80% SAIB, 10-40% xSAIB and EtOH or DMSO as solvent.

In yet a preferred embodiment one or more HIPs are formulated in ICells with the composition; 30-80% SAIB, and EtOH or DMSO as solvent.

In one embodiment one or more HIPs are formulated in ICells comprising LOIB, xSAIB and EtOH or DMSO

In one embodiment one or more HIPs are formulated in ICells with the composition: 30-80% LOIB, 10-40% xSAIB and EtOH or DMSO as solvent.

In one embodiment one or more HIPs are formulated in ICells with the composition: 30-80% LOIB, and EtOH or DMSO as solvent.

In one embodiment one or more HIPs are formulated in ICells comprising SuBen, CLA-8 and EtOH or DMSO.

In one embodiment of the disclosure, one or more HIPs are formulated in ICells comprising 30-80% SuBen, 5-20% CLA-8 and EtOH or DMSO.

In one embodiment of the disclosure, one or more HIPs are formulated in ICells comprising SuBen, LOIB, xSAIB and EtOH or DMSO.

In one embodiment of the disclosure, one or more HIPs are formulated in ICells comprising 10-40% SuBen, 10-40% LOIB, 10-40% xSAIB and EtOH or DMSO.

In one embodiment of the disclosure, one or more HIPs are formulated in ICells comprising LacBen, LOIB, xSAIB and EtOH or DMSO.

In one embodiment of the disclosure, one or more HIPs are formulated in ICells comprising 10-40% LacBen, 10-40% LOIB, 10-40% xSAIB and EtOH or DMSO.

In one embodiment of the disclosure, one or more HIPs are formulated in ICells comprising LOIB, CAB, xSAIB and EtOH or DMSO.

In one embodiment of the disclosure, one or more HIPs are formulated in ICells comprising 30-80% LOIB, 1-10% CAB, 10-30% xSAIB and EtOH or DMSO.

In one embodiment of the disclosure, one or more HIPs are formulated in ICells comprising SuBen, Ethyl-palmitate and EtOH or DMSO.

In one embodiment of the disclosure, one or more HIPs are formulated in ICells comprising 25-60% SuBen, 10-30% Ethyl-palmitate and EtOH or DMSO.

In one embodiment of the disclosure, one or more HIPs are formulated in ICells comprising SuBen, CLA-8, Ethyl-palmitate and EtOH or DMSO.

In one embodiment of the disclosure, one or more HIPs are formulated in ICells comprising 15-60% SuBen, 5-30% CLA-8, 10-30% Ethyl-palmitate and EtOH or DMSO.

In one embodiment the HIP formulated in ICell comprises a fluorophore for injection or administration in a human or animal to allow for identification by fluorescence imaging.

In on embodiment the HIP formulated in ICell comprises a NIR fluorophore as example, but not limited to ICG, for NIR fluorescence imaging.

In one embodiment the HIP formulated in ICell comprises a NIR fluorophore for injection or administration in a tissue identified by diagnostic imaging e.g., CT, MR, PET, SPECT, ultrasound, radiography for subsequent identification by NIR fluorescence imaging.

In one embodiment the ICell composition is injected or administered using image guidance, including both not limited to CT-, ultrasound-, MR-, PET-, SPECT-, fluoroscopy-, endoscopy-guided imaging.

In one embodiment the injected or administered tissue comprises a cancer lesion e.g., primary malignant or benign tumor, a metastasis, a lymph node, a surgical bed for identification by NIR imaging in connection with treatment e.g., surgery.

In one embodiment the injected or administered site contains an object of interest, e.g., foreign body, polyp, tissue defect for identification by NIR imaging in connection with treatment e.g., surgery, including NIR guided and / or robotic surgery.

In one embodiment the injected or administered site is of interest for localization contains an object or tissue that is of interest to localize during therapeutic procedures, non-limiting examples include fragile tissue, tissue margins, nerves and vessels and ducts.

In one embodiment the injected or administered to form positional waypoints that can guide dissections and navigation during NIR guided surgery. In one embodiment multiple fluorescent markers are placed in the optimal path from the skin surface to e.g., a deeply located lymph, foreign body, cancerous lesion or vascular defect to guide and minimize trauma and improve accuracy during NIR guided surgery.

In one embodiment the injected tissue is a lung lesions of interest for accurate localization by NIR imaging or palpation during surgical procedures e.g., during thoracotomy, robotic surgery and video assisted thoracic surgery (VATS).

In one embodiment the injected or administered lesions is a primary, recurrent, metastatic cancerous lesion or a lymph node, metastatic lymph node or a surgical bed after cancer surgery.

In one embodiment, the ICell contains HIP complexes of NIR fluorophores such as but not limited to ICG or MB, that upon injection or administration in primary, recurrent, metastatic cancerous lesion or a lymph node, metastatic lymph node or a surgical bed after cancer surgery release fluorophores that travel to draining lymph node e.g., sentinel lymph nodes.

In one embodiment the fluorophore releasing ICell composition is used to identify lymphatic disease, dysregulation, lymphangiogenesis, traumatic lesions in connection with therapy, including but not limited to surgery, diagnostic, or interventional radiological procedures.

### ICell compositions for sustained release of HIPs

In one embodiment of the disclosure, one or more HIPs are formulated in ICells comprising a carbohydrate ester, a co-solvent and a solvent.

In one embodiment of the disclosure, one or more HIPs are formulated in ICells comprising a carbohydrate ester, a CT imaging agent, a co-solvent and a solvent.

In one embodiment of the disclosure, one or more HIPs are formulated in ICells comprising 30-80% carbohydrate ester, 5-20% CT imaging agent, 5-30% co-solvent and 10-30% solvent.

In one embodiment of the disclosure, one or more HIPs are formulated in ICells comprising a carbohydrate ester such SOP, SAIB, SOIB, SuBen, LOP, LOIB, LacBen, MeLOIB and the like, a CT imaging agent such as xSAIB, CLA-8 and the like, a co-solvent such as glycerol trihexanoate (GTH), glycerol trioctanoate (GTO) and glycerol tridecanoate (GTD) or Lipiodol and the like, and a solvent such as EtOH, DMSO and the like.

In one embodiment of the disclosure, one or more HIPs are formulated in ICells comprising 30-80% carbohydrate ester such SOP, SAIB, SOIB, SuBen, LOP, LOIB, LacBen, MeLOIB and the like, 5-30% CT imaging agent such as xSAIB, CLA-8 and the line, 5-30% co-solvent such as glycerol trihexanoate (GTH), glycerol trioctanoate (GTO) and glycerol tridecanoate (GTD) or Lipiodol and the like, and 10-30% solvent such as EtOH, DMSO and the like.

In one embodiment, one or multiple HIP complexes are formulated in SuBen:GTO:EtOH (60:25:15), SuBen:GTH:DMSO (60:20:20), SuBen:CLA-8:GTH:DMSO (50:10:20:20), SuBen:GTO:EtOH (63:17:20), or SuBen:CLA-8:GTO:EtOH (60.5:2.5:17:20) ICell compositions.

In one embodiment, one or multiple HIP complexes are formulated in SuBen:GTO:EtOH (60:25:15), SuBen:GTH:DMSO (55:25:20), SuBen:GTH:DMSO (60:15:25), SuBen:GTH:DMSO (60:20:20), SuBen:GTH:DMSO (64:16:20), SuBen:GTH:EtOH (55:25:20), SuBen:GTH:EtOH (60:15:25), SuBen:GTH:EtOH (64:16:20), SuBen:GTH:EtOH (60:20:20) ICell compositions.

### HIP of excipients

In one embodiment, the fluorescent imaging agent is methylene blue (MB), indocyanine green (ICG), an MRI imaging agent or a CT imaging agent.

In one embodiment of the disclosure, HIP complexes of CT imaging agents such as Diatrizoate, Acetrizoic acid, iodamide, lodipamide, ioxaglate, iothalamate and the like are formulated in ICell compositions.

In one embodiment of the disclosure, HIP complexes of MRI imaging agents. Exemplary magnetic resonance agents include but are not limited to paramagnetic agents, superparamagnetic agents, and the like. Exemplary agents can include but are not limited to gadopentetic acid, gadolinium, gadoteridol, gadoxetic acid, superparamagnetic iron oxide and ferristene. gadoteric acid, gadopentetate, gadobenate, gadoxentate, magnevist, multihance, dotarem, eovist and the like are formulated in ICell compositions.

In one embodiment of the disclosure, HIP complexes of Chromophores, colour agents, fluorophore and the like, such as methylene blue (MB), indocyanine green (ICG), FD&D Blue (No. 1, No.2), D&C Blue No. 4, FD&C Green No. 3, D&C Green (No. 5, No. 6, No. 8), D&C Orange (No. 4, No.5, No.10, No. 11), FD&C Red (No. 3, No. 4), D&C Red No. 6, D&C Red (No. 7, No. 17, No. 21, No. 22, No. 27, No. 28, No. 28, No. 30, No. 31, No. 33, No. 34, No. 36, No. 39), FD&C Red No.40, D&C Violet No. 2, FD&C Yellow (No. 5, No.6), D&C Yellow (No. 7, No.8, No. 10, No. 11), Carbazole violet, C.I. Vat Orange 1, Poly(hydroxyethyl methacrylate) -dye copolymers(3): one or more of (1) Reactive Black 5, Reactive Blue 21, reactive orange 78, Reactive yellow 15, reactive blue (No. 19, No. 4), C.I. reactive red 11, C.I. Reactive Yellow No. 86, C.I. Reactive blue 163, C.I. reactive red 180, D&C Blue No. 9, D&C Green No. 5, [Phthalocyaninato(2-)] copper, FD&C Blue No. 2, D&C Blue No. 6, D&C Green No. 6, D&C Red No. 17, D&C Violet No. 2, D&C Yellow No. 10 and the like, are formulated in ICell compositions.

In one embodiment of the disclosure, HIP complexes of ICell forming agents such as carbohydrate esters or polymers are formulated in ICell.

### API classes

In one embodiment combinations of HIP complexed APIs and native (not HIP complexed) APIs are formulated in a ICell composition.

In one embodiment combinations of HIP complexed APIs and native APIs and HIP complexed or native imaging imaging agents are formulated in a ICell composition.

In one embodiment the suitable therapeutic agents for HIP complexes formulated in a ICell compositions can also be, without limitation, immunosuppressive agents, immune activating agents, immune modulating agents, cytokines, cytotoxic agents, nucleolytic compounds, radioactive isotopes, receptors, and pro-drug activating enzymes. The therapeutic agents of the present invention may be naturally secreted or produced by synthetic or recombinant methods, or any combination thereof.

A wide spectrum of therapeutic agents may be used for HIP complexing and formulation in a ICell composition. Non-limiting examples of such therapeutic agents include antineoplastic agents, anti-infective agents, antimicrobials, antibiotics, local anesthetics, anti-allergics, anti-anemics, beta-adrenergic blockers, calcium channel antagonists, anti-hypertensive agents, glucagon like peptides, insulins, antidepressants, angiogenic, anti-angiogenic, growth factors, anticonvulsants, antibacterial, anti-fungal, anti-viral, anti-rheumatics, anthelminithics, anti-parasitic agents, corticosteroids, hormones, hormone antagonists, immunomodulators, neurotransmitter antagonists, anti-diabetic agents, statins, lipid-lowering medications that reduce illness and mortality in those who are at high risk of cardiovascular disease (e.g., HMG-CoA reductase inhibitors), anti-epileptics, anti-haemorrhagic, anti-hypertonics, antiglaucoma agents, immunomodulatory cytokines, sedatives, chemokines, vitamins, toxins, narcotics, plant derived agents, wound repair agents, tissue repair agents, thermal therapy agents, and combinations thereof prepared and formulated in ICell compositions. The ICell composition may be injected, smeared, administered or installed in both soft tissues and bone, non-limiting examples includes sub-cutaneous, intramuscular, intradermal, intranodal, intraosseous, in organs, intratumoral, in infected tissues, in tissue defects, in scars, in wounds, smearing in surgical sites and surgical beds, in and around athroplastics, in and around implants, peritendinous, peri- and intraarticular, on mucosal and serosal surfaces, in wounds and on wound surfaces, in abscesses, phlegmons and body cavities e.g., intraperitoneal, intrathoracic, intravesical, intrauterine, intranasal, in sinuses, in the inner, middles and outer ear and on the skin and body surface.

In one embodiment, the active pharmaceutical ingredient is an innate immune activating compound which is a ligand for intracellular proteins and/or receptors; or a ligand for cell surface proteins and/or receptors.

The compounds can include the following drug as single therapeutic agents or as combinations of; immune activating compounds, including; Toll-like-receptor (TLR) family; TLR1, TLR2 TLR3, TLR4, TLR5, TLR6, TLR7, TLR8, TLR9, TLR10, TLR11, TLR12, TLR13. Examples of TLR agonists includes; polyinosinic:polycytidylic acid (poly I:C), Polyadenylic-polyuridylic acid (poly A:U), poly I:C-poly-L-lysine (poly-ICLC), poly-ICR, 3p'dsRNA, 3p'dsDNA, 2p'dsRNA, 2p'dsDNA, p'dsRNA, p'dsDNA, dsRNA, dsDNA, ssDNA, ssRNA, Imiquimod (R837), Resiquimod (R848), TMX-1 0 , TMX-201, TMX-202, DSR6434, Gardiquimod, R850, R851, 852A, Isatoribine, S-2761 0 , 3M- 002 (CL075), 3M-003, 3M-005, 3M-006, 3M-007, 3M-012 , 3M-13 , 3M-031 ,3M-854, CL075, CL097, CL264, IC-31 , Loxoribine and other imidazoquinolines, ssPolyU, ANA975, SM360320, SM3244405, RWJ 21757 , R 1354, single stranded or double stranded RNA, ORN 02 (5'- UUAUUAUUAUUAUUAUUAUU-3'), ORN 06 5'-UUGUUGUUGUUGUUGUUGUU-3', CpG-ODN DSLIM, AVE 0675, CpG B oligodeoxynucleotide, 1018, AZD 1419, ODN 1982, CpG B ODN 2006, IMO 2125, CpG A ODN 221 6 , CpG A ODN 2336, CpG 2395, CpG ODN 7909, CpG 10101, CpG ODN AVE0675, CpG ODN HYB2093, CpG ODN HYB2055, CpG-ODN IMO-21 25, CpG C ODN M362, Tolamba (Amb a 1 ragweed allergen with covalently linked CpG B class ODN 10 18), Heplisav, 10 181SS, IM02055, IRS954, (flagellin, muramyl dipeptide, saponins such as QS21 , Leishmania elongation factor, SB-AS4, threonyl-muramyl dipeptide, L 18-MDP, mifamurtid, A83-01 , A4476, GW788383, LY364947, R26871 2, RepSox, SB431542, SB505124, SB525334, SD208, FAK inhibitor 14, PF431 396, PF573228, Y 11 and OM- 174, nickel and the like, but not limited to those.

Inhibitors of Toll-like-receptors (TLR) include; AT791, E6446, COV08-0064, COV08-0055 and COV08-0064 and the like, but not limited to those.

STING agonists; compounds include; ADU-S100, C11, Cridanimod, MK-1454, PO-424, H-151, C-176, diABZI compound 3 (2138299-34-8 and 2138299-33-7), diABZI compound2 (2138300-40-8), 3'3'-cGAMP, 2'3'-cGAMP, ChX0306710, ML RR-S2 CDA ammonium salt, ML RR-S2 CDA, sodium Cridanimod, G10 and the like, but not limited to those.

STING inhibitors, compounds include; H-151, C-176, C-178, STING inhibitor 18, Astin C and the like, but not limited to those. RIG-1-like receptor agonists, examples include; KIN1148, KIN131A, KIN126X, KIN150X, KIN1000, KIN1408, SLR14, MK4621, RGT100, KIN1400 and the like, but not limited to those.

In yet another embodiment the active pharmaceutical ingredient is an immune activating/modulating drug which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The compounds include; Tumor necrosis factor alpha (TNF-a) agonists, TNF-a receptor blocking molecules, Interferon (IFN) agonists, examples include; RO8191, RO8181 and the like, but not limited to those. T-Cell Immunoreceptor with Ig and ITIM Domains (TIGIT) agonists.

In yet another embodiment the active pharmaceutical ingredient is a transcription factor modulator, post translational enzyme modulator, immune cell polarizing and immune modulating drug which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition.

The compounds include, as single APIs or in combination: including transforming growth factor beta signaling inhibitors (TGF-ß inhibitors) and transforming growth factor beta receptor inhibitors (TGF-ß receptor inhibitors) and including ALK signaling inhibitors and Smad singnaling inhibitors; RepSox, Galunisertib (LY2157299), LY550410, LY580276, TEW-7197, SB 505124, SB 431542, A 83-01, SD 208, LY 364947, SB 525334, SB 505124, D 4476, GW 788388, R 268712, IN 1130, SM 16, A 77-01, SB 431542, LY 364947, R268712, ITD 1, SIS3, LY2109761, LY 3200882, Pirfenidone, LDN-193189, LDN-193189 HCL, K02288, LDN-214117, TEW-7179, DMH1, LDN-212854, ML347, sotirimod, Kartogenin, Hespertin, Alantolacton, Suramin sodium, BML-275 dihydrochloride, BML-275, ALK2-IN-1, Vactosertib, LSKL, Inhibitor of Thrombospondin (TSP-1), SJ000291942, K02288, LDN-212854, ML347, SM 16, TGFβRI-IN-1 and the like, but not limited to those.

The compounds include, as single APIs or in combination: including tyrosine phosphatase SHP2 inhibitors; SHP099, PC61275 and the like, but not limited to those.

The compounds include, as single APIs or in combination: including Glycogen Synthase Kinase 3 (GSK-3) inhibitors; SB415286, Lithium, Valproic acid, lodotubercidin, Naproxen, Cromolyn, Famotidin, Curcumin, Olanzapine, Pyrimidine derivater, ARA014418, CHIR 99021, CHIR 99021 trihydrochloride, SB 216763, BIO, Kenpaullone, , 10Z-Hymenialdisine, SB 415286, Indirubin, Indirubin-3prime-monoxime-5-sulphonic Acid, Indirubin-3'-oxime, 5-lodo-indirubin-3prime-monoxime, Indirubin-5-sulfonic acid sodium salt, NSC 693868, TWS 119, TWS 119 ditrifluoroacetate, TCS 2002, MeBIO, BIO, BIO-acetoxime, Bisindolylmaleimide I, Bisindolylmaleimide I hydrochloride, 3F8, TCS 21311, TCS2002, TC-G 24, A 1070722, Lithium Carbonate, TDZD 8, AlsterPaullone, CHIR 99021, CHIR 99021 trihydrochloride, CHIR 98014, tideglusib, AZD2858, AZD1080, LY2090314, 2-D08, IM-12, 1-Azakenpaullone, Bikinin, L807mts, Staurosporine, KT5720, GSK-3 inhibitor IX, Ro 31-8220, CID 755673, GSK-3 Inhibitor XVI, 10Z-Hymenialdisine, GSK-3beta Inhibitor VI, Manzamine A, GSK-3 Inhibitor X, GSK-3 Inhibitor XV, GSK-3beta Inhibitor I, GSK-3beta Inhibitor VII, GSK-3beta Inhibitor II, GSK-3beta Inhibitor VIII, Hymenidin, Bisindolylmaleimide X hydrochloride, 3F8, Isogranulatimide, CR8, L779,450, Aloisine A, GSK-3beta Inhibitor XI, Ro-31-8220, Ro 31-8220 methanesulfonate, Enzastaurin, PHA 767491 hydrochloride, AR-AO 14418-d3, Indole-3-acetamide, Hymenialdisine Analogue 1, CP21R7, Necrosulfonamide, IM12, Leucettine, LY-2090314, Tideglusib and the like, but not limited to those.

The compounds include, as single APIs or in combination: including signal transducer and activator of transcription (STAT) inhibitors; Stattic, Cucurbitacin I, Niclosamide, NSC 74859, SD 1008, Cryptotanshinone, Napabucasin, Galiellalactone, S3I-201, Nifuroxazide, SH-4-54, AS1517499, Artesunate, BP-1-102, SH5-07, STA-21, HJC0152, APTSTAT3-9R, C188-9- HO-3867, RSVA 405, and the like, but not limited to those.

The compounds include, as single APIs or in combination: including Wnt/β-catenin signaling targeting therapy; WAY-316606, IWP, IWP-L6, LGK974, WNT-C59, ETC-159, Ant1.4Br/Ant 1.4CI, (hetero)arylpyrimidines, Niclosamide, apicularen, bafilomycin, XAV939, IWR, G007-LK, G244-LM, IQ1, pyrvinium, QS11, NSC668036, SB-216763, CHIR99021, BIO(6-bromoindirubin-3'-oxime), LY2090314, DCA, 2-amino-4-[3,4-(methylenedioxy)benzyl-amino]-6-(3-methoxyphenyl)pyrimidine, 2,4-diamino-quinazoline, Quercetin, ICG-001, PKF115-584, BC2059, Shizokaol D, 3289-8625, J01-017a, Derricin, Derricidin, Carnosic acid, Windorphen, TMEM88, KY-02061, KY-02327, BMD4702, DK-520, Sulindac, ICG-001, PNU-74654, E7449 and the like, but not limited to those.

The compounds include, as single APIs or in combination: including Phosphoinositide 3-kinases (PI3Ks) inhibitors; Wortmannin, LY294002, PX-866, XL-147, SF1126, GDC0941, PI-103, NCPBEZ235, XL765, GSK2126458, PKI-587, MK2206, PF-04691502, IC187114, CAL-101, Rapamycin, Torin1, AZD-8055, OSI-027, GDC-0032, NVP-BKM120, ZSTK474, BAY 80-6946, BYL719, GDC0326, SAR260301, GDC0980, GNE-317, GNE-477, PF-0491502, PKI-179, PKI-587, INK-1117, CH5132799, AZD8186, IPI-145 Buparlisib, Idelasilib, IPI-549, Pictillisib and the like, but not limited to those.

The compounds include, as single APIs or in combination: including tyrosine kinase receptor inibitors, including but not limited to c-KIT (SCFR), PDGFR, FGFR, VEGFR, and FA; Axitinib, Dasatinib, TKI-258, ST1571, AMG-706, GW786034 HCL, Sunitinib malate, AB1010, PTK787, XL184, BMS-907351, AV-951, OSI-930, MP-470, Ki8751, Telatinib, Pazopanib, TKI-258, CHIR-258, Thyrpostin AG 1296, PKC-412, ISCK03, AP 24534, KRN633, SU6668, Sorafinib, ABT-869, Divitinib, Pazopanib, 4,4prime-Bis(4-aminophenoxy)biphenyl, ISCK03, Tandutinib, SU1652, AGL 2043, PLX9486, BLU-285, AZD2932, PLX3397, MGCD516 and the like, but not limited to those.

The compounds include, as single APIs or in combination: including tyrosine kinase receptor agonists, including but not limited to c-KIT (SCFR), PDGFR, FGFR, VEGFR, and FA; DRM/gremlin, and the like, but not limited to those.

The compounds include, as single APIs or in combination: including Bruton's Tyrosine Kinase (BTK) and Interleukin-2-Inducible Kinase (ITK) Inhibitors: Ibrutinib, Acalabrutinib and the like, but not limited to those.

The compounds include, as single APIs or in combination: including Hedgehog pathway modulators, including Smoothened (Smo), Sonic hedgehog pro-tein (Shh), and Gli1 inhibitors and agonists: SAG, compound 10c, Mercaptobenzoimidazole, cyclopamine, HhAntag, IPI926, GDC-0449, Cur61414, GANT61, IPI-269609 BMS-833923, PF-04449913, HPI1, HPI2, HPI3, HPI4, JK-184, NMDA298-1, robotnikinin, Purmorphamine, 22(S)-hydroxycholesterol, 20(S)-hydroxycholesterol, GANT58, GANT61 and the like, but not limited to those.

The compounds include, as single APIs or in combination: including Mammalian target of rapamycin (mTOR) inhibitors; Temsirolimus, Everolimus, Ridaforolimus (AP23573 and MK-8669), Dactolisib, Omipalisib, Niclosamide and the like, but not limited to those.

The compounds include, as single APIs or in combination: including C-Myc inhibitors; JQ1, I-BET151, 10058-F4, 10074-G5, 7594-0035, KJ Pyr 9, ML327, Mycro3, IZCZ-3, KSI-3716, 403811-55-2, Apto-253 and the like, but not limited to those.

The compounds include, as single APIs or in combination: including c-Met inhibitors; PHA665752, INC280, SU 11274, AMG208, Golvatinib, PF 02341066, LY 2801653, ARQ 197, PF 04217903, Fortinib, Crizotinib, PHA-665752, SAR125844, Pulsatilla saponin D, SGX-523, BMS-777607, JNJ-38877605, MGCD-265, INCB28060, BMS-794833, BMS-754807, AMG-208, MK-208, MK-2461, E7050, AMG-458, NVP-BVU972, EMD 1214063, AMG-337, LY12801653, S49076, Norcantharidin, NPS-1034, AZD6094 and the like, but not limited to those.

The compounds include, as single APIs or in combination: including BRAF inhibitors; BMS-9086662, LGX818, PLX3603, RAF265, RO5185426, GSK2118436, PLX4032, Sorafenib, PLX-4720, GDC-0879, AZ304, PLX-8394, LXH254, Dabrafenib mesylate, RAF265, AZ 628, NVP-BHG712, SB590885, ZM 336372, GW5074, TAK-632, CEP-32496, LGX818, BAW2881, CCT196969, RAF709, BGB-283, PLX7904, LY3009120, RO5126766, MLN2480, Regorafenib and the like, but not limited to those.

The compounds include, as single APIs or in combination: including MEK inhibitors; BIX02188, PD0325901, U0126-ETOH, GSK1120212, AZD6244, PD0325901, CI-1040, PD98059, AS-703026, TAK-733, AZD8330, MEK162, PD318088, Honokoil, SL-327, RDEA119, Myricetin, BI-847325, GDC-0973, GDC-0623, APS-2-79 and the like, but not limited to those.

In yet another embodiment the active pharmaceutical ingredient is an epigenetic modulating drug, which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The APIs include, as single agents or in combination: including DNA methyltransferase inhibitors (DNMTi), histone deacetylase inhibitors (HDACi), histone methyltransferases inhibitors (HMTi), histone acetyltranferases inhibitors (HATi), histone demethylases inhibitors (HDMi), proteins binding to methylated and acetylated histones inhibitors (PAHi and PMHi); decitabine, azacitidine, EGCG, zebularine, hydralazine, procainamide, Vorinostat, givinostat, panobinostat, TSA, belinostat, entinostat, CG-1521, romidepsin, ITF-A, ITF-B, valproic acid, OSU-HDAC-44, HC-toxin, magnesium valproate, plitidepsin, tasquinimod, sodium butyrate, mocetinostat, carbamazepine, SB939, CHR-2845, CHR-3996, JNJ-26481585, sodium phenylbutyrate, pivanex, resveratrol, abexinostat, resminostat, dacinostat, droxinostat, Pargyline, clorgyline, bizine, GSK2879552, GSK-J4, KDM5-C70, JIB-04, tranylcypromine, EPZ-6438, GSK126, CPI360, DZNep, GSK343, EI1, BIX-01294, UNC0638, EPZ004777, UNC0224, JQ1, CPI203, RVX-208, I-BET151, I-BET762, i-BET-726, UNC669, UNC1215 and the like, but not limited to those.

In yet another embodiment the API is a lymphocyte activating and modulating drugs which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The compounds include, as single APIs or in combination: Programmed death-ligand 1 and 2 (PD-L1 and PD-L2) inhibitors, programmed cell death protein 1 (PD1) inihibitors and PD-L1/PD-L1 checkpoint inhibitors; BMS-8, BMS-37, BMS-57, BMS-71, BMS-105, BMS-202, BMS-230, BMS-242, BMS-1001, BMS-1166, BMS-1165, BMS-2007, BMS-1016, BMS-40210, BMS-8, CA-170, CA-327, SB415286, INCB086550, INCMGA00012, CX072, CCX4503, MAX-10129, vorinostat, panobinostat, azacitidine, decitabine, entitostat, JQ1, I-BET151, GSK503, WO2015/034820, WO2015/033301 and the like, but not limited to those.

The compounds include, as single APIs or in combination: including Anti-cytotoxic T-lymphocyte-associated protein-4 (anti-CTLA-4) inhibitors/modulators; Compounds "8 and 9", ACY- 241 and the like, but not limited to those.

The compounds include, as single APIs or in combination: including T-cell immunoglobulin and mucin domain-3 (TIM-3) inhibitors; TSR-022, Sym023, ATIK2a, CA-327 and the like, but not limited to those.

The compounds include, as single APIs or in combination: including Lymphocyte activating gene 3 (LAG3) inhibitors; IMP32, BMS986016 and the like, but not limited to those.

The compounds include, as single APIs or in combination: including OX40 (CD124) activators/modulators, examples include; DB36, DB71, DB15, CVN, MGCD0103, SNDX-275 and the like, but not limited to those. Tumor necrosis factor receptor super family (TNFRSF) agonist (e.g. OX40 (CD124) agonists, CD40 agonists, CD27 agonists, 4-1BB (CD137) agonists, Glucocorticoid-induced tumor necrosis factor receptor-related protein (GITR, CD357) agonists, inducible T-cell costimulator (ICOS) agonists and tumor necrosis factor related apoptosis-inducing ligand (TRAIL, CD253, TNFSF10) receptor agonist, examples include; Acrp30-CD40L, DB36, DB71, DB15, CVN, MGCD0103, SNDX-275, dulanermin, and the like, but not limited to those.

In yet another embodiment the APIs is an immunemodulating and/or inflammation modifying enzyme inhibitor or activator, a cellular receptor of metabolites, which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The compounds include, as single agents or in combination: including Indoleamine 2,3-dioxygenase-1 (IDO1) inhibitors; methyl-tryptophan, D-1MT, L-1MT, tryptophan, epacadostat, GDC-0919, Indoximod, EOS-200271, NLG919, BMS-986205 and the like, but not limited to those.

The compounds include, as single APIs or in combination: including Arginase inhibitors; INCB001158 and the like, but not limited to those.

The compounds include, as single APIs or in combination: including Adenosine receptor inhibitors; caffeine, AZD4635, Vipedenant, Preladenant, CPI-444 and the like, but not limited to those.

The compounds include, as single APIs or in combination: including cyclooxygenase (COX) 1 and/or 2 inhibitors; Cyclooxygenase (COX) 1 and/or 2 inhibitors; celecoxib, rofecoxib, DuP-697, valdecoxib, etoricoxib, lumiracoxib, indomethacin, 6-methoxy-α-methyl-2-naphthylacetic acid, meclofenamic acid, diclofenac, flufenamic acid, niflumic acid, mefenamic acid, sulindac, tolmetin, suprofen , ketorolac, flurbiprofen, ibuprofen, aceloferac, alcofenac, amfenac, benoxaprofen, bromfenac, carprofen, clidanac, diflunisal, Efenamine, Etodol, fenbufen, fenclofenac, fenclorac, fenoprofen, piroxicam, fleclozic, indoprofen, isofezolac, ketoprofen, loxoprofen, meclofenamate, naproxen, Organoxin , pirprofen, pranoprofen, tolfenamic acid, zaltoprofen, zomepirac and the like, but not limited to those.

The compounds include, as single agents or in combination: including hypoxia inducible factor 1 (HIF-1) inhibitors and hypoxia inducible factor 2 (HIF-2) inhibitors; Chemotomin, Chrysin, Dimethyl-bisphenol, Echinomycin, PX 12, YC-1, Vitexin and the like, but not limited to those.

In yet another embodiment the APIs is a chemokine receptor signal and chemokine receptor modifying drug which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The compounds include, as single APIs or in combination: chemokine receptor signaling modifiers; AZD5069, SX-682, AMD3100, X4P-001, PF-4136309, Maraviroc, LMT-28, madindoline-5 (MDL-5), MDL-16 and MDL-101, SPD-304, C87 ((E)-4-(2-(4-chloro-3-nitrophenyl)), tamatinib fodium (R788), ZINC09609430, ZINC49467549, ZINC13113075, ZINC39907639, ZINC25251930, ZINC02968981, ZINC09544246, ZINC58047088, ZINC72021182, ZINC08704414, ZINC05462670, ZINC35681945, ZINC23553920, ZINC05328058, and ZINC17206695 and the like, but not limited to those.

In yet another embodiment the APIs is a cell cycle checkpoint inhibitor, activator or modulator which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The compounds include, as single APIs or in combination: including Checkpoint kinase 1 and/or 2 (CHK1/2) inhibitors; AZD7762, LY 2603618, CCT 241533, NSC 109555, PD 407824, PF 477736, SB 218078, UCN-01, CHIR-124, SAR-020106, CCT244747, SCH900776, V158411, TCS 2312, Hymenialdisine, ABI, NSC1095555, PV1019, VRX0466617, CCT241533, Aminopyridine 7 and the like, but not limited to those.

The compounds include, as single APIs or in combination: including Ataxia telangiectasia mutated (ATM) inhibitors; Wortmannin, Caffeine, KU55933, KU55403, KU60019, CP-466722, CGK733, NVP-BEZ235, Torin-2, Methoxyquinazoline 1, Fluoroquinoline 2, SJ573017 and the like, but not limited to those.

The compounds include, as single APIs or in combination: including WEE1 inhibitors; PD0166285, PD407824, WEE1 inhibitor II, MK1775, Pyrimidopyrimidinone 8 and the like, but not limited to those.

The compounds include, as single APIs or in combination: including Ataxia telangiectasia and Rad3-related (ATR) inhibitors; Schisandrin B, ETP-46464, NU6027, VE-821, VE-822, AZ20, AZD6738 and the like, but not limited to those.

In yet another embodiment the API is a cell death inducing chemotherapeutic and immunogenic cell death inducing chemotherapeutics which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The compounds include, as single APIs or in combination: irinotecan hydrochloride, nogitecan hydrochloride, exatecan, RFS-2000, lurtotecan, BNP-1350, Bay-383441, PNU-166148, IDEC-132, BN-80915, DB-38, DB-81, DB-90, DB-91, CKD-620, T-0128, ST-1480, ST-1481, DRF-1042 and DE-310, taxane derivatives such as docetaxel hydrate, IND-5109, BMS-184476, BMS-188797, T-3782, TAX-1011, SB-RA-31012, SBT-1514 and DJ-927, ifosfamide, nimustine hydrochloride, carboquone, cyclophosphamide, dacarbazine, thiotepa, busulfan, melphalan, ranimustine, estramustine phosphate sodium, 6-mercaptopurine riboside, enocitabine, gemcitabine hydrochloride, carmofur, cytarabine, cytarabine ocphosphate, tegafur, doxifluridine, hydroxycarbamide, fluorouracil, methotrexate, mercaptopurine, fludarabine phosphate, actinomycin D, aclarubicin hydrochloride, idarubicin hydrochloride, epirubicin hydrochloride, daunorubicin hydrochloride, pirarubicin hydrochloride, bleomycin hydrochloride, zinostatin stimalamer, neocarzinostatin, mytomycin C, bleomycin sulfate, peplomycin sulfate, vinorelbine tartrate, vincristine sulfate, vindesine sulfate, vinblastine sulfate, amrubicin hydrochloride, gefitinib, exemestan, capecitabine, TNP-470, TAK-165, KW-2401, KW-2170, KW-2871, KT-5555, KT-8391, TZT-1027, S-3304, CS-682, YM-511, YM-598, TAT-59, TAS-101, TAS-102, TA-106, FK-228, FK-317, E7070, E7389, KRN-700, KRN-5500, J-107088, HMN-214, SM-11355, ZD-0473, magnesium 5,10,15,20-tetrakis(4-sulphophenyl)-porphine dodecahydrate, PYROA protein (EmerICella nidulans), photosan III, lomefloxacin, cyamemazine, tiaprofenic acid, doxorubicin, mitomycin, paclitaxel, nitrogen mustards, etoposide, camptothecin, 5-fluorouracil, nicotinamide, metronidazole, doxorubicine, Lomeguatrib, Temozolomide, tamoxifen, bleomycin, 5-fluorouracil, cyclophosphamide, methotrexate, gemcitabine, oxaliplatin, cisplatin, carboplatin, camptothecin, CPT-11 (SN-38), Etanidazole, Nimorazole, Mitomycin C, Tirapazamine, procaine, lidocaine, chlorpromazine, Fluordeoxyuridine, bromodeoxyuridine, iododeoxyuridine, hydroxyurea, fludarabine, Texaphyrins (motexafin gadolinium), N-ethylmalemide, paclitaxel, docetaxel, irinotecan, Mechtorethamine, Cyclophosphamide, Ifosfamide, Melphalan, Chlorambucil, Procarbazine (N-methylhydrazine, MIH), Busulfan, Camustine (BCNU), Streptozocin (streptozotocin), Bendamustine, Dacarbazine (DTIC; dimethyttriazenol midazole carboxamide), Temozolomide, Cisplatin, carboplatin, oxaliplatin, Methotrexate (Amethopterin), Pemetrexed, Fluorouracil (5-fluorouracil; 5-FU), capecitabine, Cytarabine (cytosine arabinoside), Gemcitabine, 5-aza-cytidine, Deoxy-5-aza-cytidine, Mercaptoptirine (6-mercaptopurine; 6-MP), Pentostatin (2'-deoxycoformycin), camptothecin, SN-38 (CPT-11), Rudarabine, Clofarabine, Nelarabine, Tirapazamine, Vinblastine, Vinorelbine, Vincristine, Paclitaxel, docetaxel, Etoposide, Teniposide, Topotecan, Irinotecan, Dactinomycin, (actinomycin D). Daunorubicin (daunomycin, rubidomycin), Doxorubicin, Yondelis, Mitoxantrone, Bleomycin, Mitomycin C, L-Asparaginase, Mitotane (o.pDDD) Prednisone, Hydroxyprogesterone caproate, medroxyprogesterone acetate, megestrol acetate, Dietyhlstilbestrol, ethinyl estradiol, Tamoxifen, toremifene, Anastrozole, Gefitinib, letrozole, exemestane, Testosterone propionate, fluoxymesterone, Flutamide, casodex, Leuprolide. Hydroxyurea, Tretinoin, arsenic trioxide, Vorinostat, Imatinib, Dasatinib, nilotinib, Gefrtinib, ertoinib, Sorafenib, Sunitinib, Lapatinib, Bortezomib, Thalidomide, Lenaiidomide, Temsiroiimus, Everolimus, and the like, but not limited to those.

In yet another embodiment the API is an osteoinductive or osteogenic drug which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The compounds include, as single APIs or in combination: including tiludronate, alendronate, pamidronate, risedronate, ibandronate, zoledronic acid, etidronate, BPH-675, BPH-715, bone morphogenic protein (BMP e.g. BMP-2, BMP-7 and osteogenic protein 1 (OP-1)) and the like, but not limited to those.

In yet another embodiment the API is a 3-hydroxy-3-methylglutaryl coenzyme A (HMG-CoA) reductase suppressants (statin) which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The compounds include, as single APIs or in combination: including compactin, simvastin and the like, but not limited to those.

In yet another embodiment the active pharmaceutical ingredient is an inducer of osteogenic commitment of stem cells, including BMP, ERK, WNT, AMPK signalling pathways modulators which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The compounds include, as single APIs or in combination: including Purmorphamine, Mevinolin, Resveratrol, Icariin, Metformin and the like, but not limited to those.

In yet another embodiment the active pharmaceutical ingredient is an inducer of chondrogenic commitment of stem cells modulators which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The compounds include, as single APIs or in combination: including Kartogenin, TD-198.946, Prostaglandin E2 and the like, but not limited to those.

In yet another embodiment the active pharmaceutical ingredient is an inhibitor of matrix metalloproteinases (MMPs) which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The compounds include, as single APIs or in combination: including ab142180, ab145190, ab141579, Actinonin, SB-3CT, A4336, Marimastat, TAPI-0, TAPI-1, TAPI-2, Luteolin, Collagenase Inhibitor I, GM 6001, PD166793, Ro 32-3555, CP 471474, UK 356618, NNGH, ND-322, ND-336, RXP470.1 and the like, but not limited to those.

In yet another embodiment the API is an interleukin (IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IL-36 and IL-37) modulator which is a ligand for intracellular proteins and/or receptors; or a ligand for cell surface proteins and/or receptors. The drug may serve as a single therapeutic API or serve as part of a combination with additional APIs in HIP complexes or as native APIs in a ICell composition. The compounds include, as single APIs or in combination: including LMT-28, rapamycin, FK506, A-552, tofacitinib, GSK650394, LTV-1, apilimod, HG-9-91-01, GNE-7915, GSK-J4, I-BET762, SR1001, digoxin, VX-765, ONX0914, SP4206, PD225002, SB265610, PD0210293, PD0220245, NSC201631, NSC61610, LMT-28, rilonacept, anakinra, and NSC80734 and the like, but not limited to those.

In yet another embodiment the composition could further comprise a formulation where one or more of the active pharmaceutical ingredients induces an anti-bacterial effect, or an anti-infectious effect in a human or animal body. The drug may serve as a single therapeutic API or serve as part of a combination with additional APIs in HIP complexes or as native APIs in a ICell composition. The compounds include, as single APIs or in combination: Aminoglycosides, Amikacin, Gentamicin, Kanamycin, Neomycin, Netilmicin, Tobramycin, Paromomycin, Streptomycin, Spectinomycin(Bs), Ansamycins, Geldanamycin, Herbimycin, Rifaximin, Carbacephem, Loracarbef, Carbapenems, Ertapenem, Doripenem, Meropenem, Cefadroxil, Cefazolin, Cephradine, Cephapirin, Cephalothin, Cefalexin, Cefaclor, Cefoxitin, Cefotetan, Cefamandole, Cefmetazole, Cefonicid, Loracarbef, Cefprozil, Cefuroxime, Cefdinir, Cefditoren, Cefotaxime, Cefpodoxime, Ceftibuten, Ceftizoxime, Moxalactam, Ceftriaxone, Cefepime, Ceftaroline fosamil, Ceftobiprole, Glycopeptides, Teicoplanin, Vancomycin, Telavancin, Dalbavancin, Oritavancin, Lincosamides(Bs), Clindamycin, Lincomycin, Lipopeptide, Daptomycin, Macrolides(Bs), Azithromycin, Clarithromycin, Erythromycin, Roxithromycin, Telithromycin, Spiramycin, Fidaxomicin, Monobactams, Aztreonam, Nitrofurans, Furazolidone, Nitrofurantoin(Bs), Oxazolidinones(Bs), Linezolid, Posizolid, Radezolid, Torezolid, Penicillins, Amoxicillin, Ampicillin, Azlocillin, Dicloxacillin, Flucloxacillin, Mezlocillin, Methicillin,Nafcillin,Oxacillin,Penicillin G,Penicillin V,Piperacillin, Temocillin, Ticarcillin, Amoxicillin/clavulanate, Ampicillin/sulbactam, Piperacillin/tazobactam, Ticarcillin/clavulanate, Polypeptides, Bacitracin, Colistin, Polymyxin B, Polymyxin E, Ciprofloxacin, Enoxacin, Gatifloxacin, Gemifloxacin, Levofloxacin, Lomefloxacin, Enrofloxacin, Moxifloxacin, Nadifloxacin, Nalidixic acid, Norfloxacin, Ofloxacin, Trovafloxacin, Grepafloxacin, Sparfloxacin, Temafloxacin, Mafenide, Sulfacetamide, Sulfadiazine, Silver sulfadiazine, Sulfadimethoxine, Sulfamethizole, Sulfamethoxazole, Sulfanilimide (archaic), Sulfasalazine, Sulfisoxazole, Sulfonamidochrysoidine (archaic), Tetracyclines(Bs), Demeclocycline, Doxycycline, Metacycline, Minocycline, Oxytetracycline,Tetracycline, Clofazimine, Dapsone, Capreomycin, Cycloserine, Ethambutol(Bs), Ethionamide, Isoniazid, Pyrazinamide, Rifampicin, Rifabutin, Rifapentine, Streptomycin, Arsphenamine, Chloramphenicol(Bs), Fosfomycin, Fusidic acid, Metronidazole, Mupirocin, Platensimycin, Quinupristin/Dalfopristin, Thiamphenicol, Tigecycline(Bs), Tinidazole, Trimethoprim(Bs), Vancomycin, Doxycyline, Ceftobiprole[, Ceftaroline, Dalbavancin, Fusidic acid, Mupirocin, Omadacycline, Oritavancin, Tedizolid, Telavancin, Tigecycline, Ceftolozane/tazobactam, etambutol, isoniazid, pyrazinamide, aciclovir, Valaciclovir, efavirenz, emtricitabin, tenofovirdisoproxil, Rilpivirine, penicillin, Trimethoprim-sulfamethoxazole, rifampicin, etambutol, isoniazid, pyrazinamide, voriconazole, amphotericin B, caspofungin, flucytosine, itraconazole, and the like, but not limited to those.

In yet another embodiment, the API is an immuno suppressive compound comprising a steroid selected from the group consisting of 21-Acetoxyprefnenolone, Aalclometasone, Algestone, Amicinonide, Beclomethasone, Betamethasone, Betamethasone dipropionate, Betamethasone hemisuccinate, Budesonide, Chloroprednisone, Clobetasol, Blovetasone, Clocortolone, Cloprednol, Corticosterone, Cortisone, Cortivazol, Deflazacort, Desonide, Desoximethasone, dexamethason, Dexamethasone palmitate, Dexamethasone phosphate, Diflorasone, Diflucortolone, Difluprednate, Enoxolone, Fluazacort, Flucloronide, Flumethasone, Flunisolide, Fluocinolone Acetonide, Fludrocortisone, Fluocinonide, Fluocortin Butyl, Fluocortolone, Fluorometholone, Fluperolone, Fluprednidine, Fluprednisolone, Flurandrenolide, Formocortal, Halcinonide, Glucocorticoids, Halomethasone, Halopredone, Hydrocortamate, Hydrocortisone, Limethasone, Mazipredone, Medrysone, Meprednisone, Methyolprednisolone, Methyolprednisolone hemisuccinate, Mometasone Furoate, Paramethasone, Prednicarbate, Prednisolone, Prednisolone palmitate, Prednisolone phosphate, Prednisone, Prednival, Prednylidene, Tixocortal, and Triamcinolone, azathioprine, ciclosporine, 6-mercaptopurine, mycophenolate and the like, but not limited to those.

The HIP complexed drug(s), active agent(s) or bioactive agent(s) formulated in the ICell composition of the present invention may be used for treating, monitoring, preventing and/or diagnosing several diseases and conditions (e.g., cancer, inflammation, hormonal diseases and deficiency, metabolic disorders and diseases, regenerative disease, chronic wounds and inflammation, autoimmune disease, or infections). Certain embodiments can involve delivery of the same, multiple or possibly different therapeutic agents to a site or region affected by a disease or condition. In some embodiments, the HIP complexes in the ICell technology of the present invention may be particularly useful for oncological applications, such as for the treatment, monitoring, prevention and/or diagnosis of a cancerous condition (e.g., malignant tumor). In such embodiments, the HIP complexes in the ICell of the present invention may be used for delivering an active agent (e.g., a therapeutic and/or a diagnostic agent) to a site affected with cancer (e.g., a tumor). The ICell may injected intratumorally, adjacent to malignant tumors, in tissues harbouring the malignant cells (e.g., bone marrow or lymph nodes) in lymph nodes including metastatic nodes, on non-cancerous tissue, including intramuscular and subcutaneous. Non-limiting examples of cancerous conditions that may be treated, monitored, prevented and/or diagnosed include, without limitation, leukaemia, lymphoma, skin cancers (including melanomas, basal cell carcinomas, and squamous cell carcinomas), epithelial carcinomas of the head and neck, lung cancers (including squamous or epidermoid carcinoma, small cell carcinoma, adenocarcinoma, and large cell carcinoma), breast cancer, gastrointestinal tract cancers, malignant tumors of the thyroid, sarcomas of the bone and soft tissue, ovarian cancer, carcinoma of the fallopian tube, uterine cancer, cervical cancer, prostatic carcinoma, testicular cancer, bladder cancer, renal cell carcinoma, pancreatic cancer, and hepatocellular cancer. In some embodiments, the present invention provides a method for treating a subject with a cancer characterized by solid cancers/tumors.

In one embodiment the HIP complexed APIs in the ICell can be used in conjunction or concurrently with other known methods of disease treatment, including, but not limited to, chemotherapy, radiotherapy, thermal ablation, surgery, electroporation, laser therapy, oncolytic viruses, adoptive cell therapies, chimeric antigen receptor cell therapy, stem cell therapies, xenogenic, allogenic and autogenic transplants and radiotherapy.

In one embodiment the HIP complexed API in the ICell can be used to stimulate immunological recognition, immune activation, and immune mediated rejection of cancer in a human or animal. The ICell technology can provide release of single or multiple HIP complexed immune activating APIs, including co-formulation with native APIs in the ICell composition. The ICell delivered combination of APIs may support innate and adaptive anti-cancer immune activation and/or immune cell recruitment, agility, polarization, engraftment, and proliferation.

In one embodiment the therapeutic the HIP complexed API in ICell compositions may be one or more anti-cancer agents, including but not limited to cancer chemotherapy agents. Examples of suitable cancer chemotherapy agents include, without limitation: nitrogen mustards, nitrosorueas, ethyleneimine, alkane sulfonates, tetrazine, platinum compounds, pyrimidine analogs, purine analogs, nucleoside analogs, epigenetic modifying agents, nucleic acid synthesis inhibitiors, antimetabolites, folate analogs, anthracyclines, TPI-1, NSC-87877, taxanes, vinca alkaloids, and topoisomerase inhibitors, hormonal agents, alkylating agents, such as cyclosphosphamide; alkyl sulfonates; aziridines; ethylenimines and methylamelamines; anti-metabolites; nucleic acid synthesis inhibitors, pyrimidine analogs; anti-adrenals; folic acid replenisher; retinoic acid; and pharmaceutically acceptable salts, acids or derivatives of any of the above. Additional therapeutic agents for HIP complexes in ICell compositions may include, without limitation, anti-hormonal agents that act to regulate or inhibit hormone action on tumors. Non-limiting examples of such anti-hormonal agents include anti-estrogens, including for example Tamoxifen and Toremifene; anti-androgens, such as Leuprolide and pharmaceutically acceptable salts, acids or derivatives of any of the above.

In one embodiment APIs that are affected by classical multidrug resistance can have utility for HIP complexing and formulation in a ICell composition. Such drugs include, without limitation: vinca alkaloids (e.g., vinblastine), the antimetabolite and pyrimidine analogs (e.g., 5FU, gemcitabine) the anthracyclines (e.g., doxorubicin) and RNA transcription inhibitors.

In one embodiment doxorubicin is HIP complexed with a counterion in the ICell composition. In one embodiment cisplatin is HIP complexed with a counterion in the ICell composition.

In one embodiment HIP complexes of immunotherapeutics APIs such as, but are not limited to, innate immune stimulating drugs (e.g. toll like receptor (TLR) agonists, RIG-1-like receptor agonists and Stimulator of Interferon Receptor (STING) agonists, Nucleotide-binding oligomerization domain-like (NOD-like) receptor agonists), immune activation pathway inhibitors and activators (e.g. Tumor necrosis factor alpha (TNF-a), TNF-a receptor blocking molecules, OX40 (CD124) agonists, TNFSFR agonists (e.g., CD40 agonists), SHP-1 inhibitors, SHP-2 inhibitors, SHP1/2 inhibitors, NSC87877, TPI-1, NSC-87877 SHP-1 agonists, T-Cell Immunoreceptor With Ig and ITIM Domains (TIGIT) agonists), transcription factor modulators, immune cell polarizing and immune modulating drugs (e.g. transforming growth factor beta inhibitors (TGF-ßi), T box-containing protein expressed in T cells stimulators, glycogen synthase kinase 3 inhibitors, signal transducer and activator of transcription (STAT) inhibitors, Wnt/β-catenin signaling targeting therapy, Phosphoinositide 3-kinases (PI3Ks) inhibitors, c-KIT inhibitors, mammalian target of rapamycin (mTOR) inhibitors, C-Myc inhibitors, MET inhibitors, BRAF inhibitors, MEK inhibitors), lymphocyte activating and modulating therapeutics (e.g. programmed death-ligand 1 (PD-L1) inhibitors, programmed death-ligand 2 (PD-L2) inhibitors, programmed cell death protein 1 (PD-1) inhibitors, anti-cytotoxic T-lymphocyte-associated protein-4 (CTLA-4) inhibitors, KIR, NOX2, T-cell immunoglobulin and mucin domain-3 (TIM-3) inhibitors, lymphocyte activating gene 3 (LAG3) inhibitors, Tyrosine phosphatase SHP2 inhibitors), , immune metabolism and inflammatory programming therapeutics (e.g. indoleamine 2,3-dioxygenase-1 (IDO1) inhibitors, arginase (Arg) inhibitors, Adenosine A2A Receptor (A2AR) antagonists, hypoxia inducible factor 1 (HIF-1) inhibitors, hypoxia inducible factor 2 (HIF-2) inhibitors, V-domain Ig suppressor of T cell activation (VISTA) inhibitors, cyclooxygenase (COX) 1 and/or 2 inhibitors), IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IL-36 and IL-37) inhibitors and activators, immunosuppressive agents (e.g. synthetic glycocorticoids, ciclosporine, Azathioprine, ketoconazole), acute phase protein inhibiting drugs (e.g. inhibitors of: C-reactive protein, Serum amyloid P,Serum amyloid A, Complement factors, Mannan-binding lectin, Fibrinogen, prothrombin, Plasminogen activator inhibitor-1 (PAI-1), tissue Plasminogen Activator (tPA), Alpha 2-macroglobulin, Ferritin , Ceruloplasmin, Haptoglobin, (Alpha-1-acid glycoprotein, AGP), Alpha 1-antitrypsin, and Alpha 1antichymotrypsin), drugs modulating extracellular matrix composition (e.g. matrix metalloproteinases (MMP) including (MMP1, MMP2, MMP3, MMP7, MMP8, MMP9, MMP10, MMP11, MMP12, MMP13, MMP14, MMP15, MMP16, MMP17, MMP18, MMP19, MMP20, MMP21, MMP23A, MMP23B, MMP24, MMP25, MMP26, MMP27, MMP28), Osteoconductive and osteoinductive agents (e.g. osteoprotegerin agonists and/or Glycogen synthase kinase 3b inhibitors, transforming growth factor beta-1-3), and cell death inducing chemotherapeutics are prepared and formulated in a ICell.

In one embodiment an immune checkpoint inhibitor, including but not limited to inhibitors of PD-1, PD-L1, CTLA-4, LAG3, and TIM-3 is HIP complexed and formulated in a ICell composition. In one embodiment an immune checkpoint inhibitor, including but not limited to inhibitors of PD-1, PD-L1, CTLA-4, LAG3, and TIM-3 is HIP complexed and co-formulated with a TLR7/8 agonist and / or TGFb inhibitor in a ICell composition. In one embodiment an immune checkpoint inhibitor, including but not limited to inhibitors of PD-1, PD-L1, CTLA-4, LAG3, and TIM-3 is HIP complexed and co-formulated with R848 and / or RepSox in a ICell composition. In one embodiment an immune checkpoint inhibitor, including but not limited to inhibitors of PD-1, PD-L1, CTLA-4, LAG3, and TIM-3 is HIP complexed and co-formulated with R848 and / or galunisertib in a ICell composition. '

In one embodiment a kinase or phosphatase inhibitor, including but not limited to inhibitors of SHP1, SHP2 and SHP1/2 is HIP complexed and formulated in a ICell composition. In one embodiment a kinase or phosphatase inhibitor, including but not limited to inhibitors of SHP1, SHP2 and SHP1/2 is HIP complexed and co-formulated with a TLR7/8 agonist and / or TGFb inhibitor in a ICell composition. In one embodiment a kinase or phosphatase inhibitor, including but not limited to inhibitors of SHP1, SHP2 and SHP1/2 is HIP complexed and co-formulated with R848 and / or RepSox in a ICell composition. In one embodiment a kinase or phosphatase inhibitor, including but not limited to inhibitors of SHP1, SHP2 and SHP1/2 is HIP complexed and co-formulated with R848 and / or galunisertib in a ICell composition.

In one embodiment an immunomodulator, including but not limited to inhibitors of beta-catenin, IDO, Arg1, A2AR, GSK3 and agonists for FLT3L, OX40L is HIP complexed and formulated in a ICell composition. In one embodiment an immunomodulator, including but not limited to inhibitors of beta-catenin, IDO, Arg1, A2AR, GSK3 and agonists for FLT3L, OX40L is HIP complexed and co-formulated with a TLR7/8 agonist and / or TGFb inhibitor in a ICell composition. In one embodiment an immunomodulator, including but not limited to inhibitors of beta-catenin, IDO, Arg1, A2AR, GSK3 and agonists for FLT3L, OX40L, CD40 is HIP complexed and co-formulated with R848 and / or RepSox in a ICell composition. In one embodiment an immunomodulator, including but not limited to inhibitors of beta-catenin, IDO, Arg1, A2AR, GSK3 and agonists for FLT3L, OX40L is HIP complexed and co-formulated with R848 and / or galunisertib in a ICell composition.

The possibility to provide tailored release by means of HIP complex and ICell optimizations allows for improved generation of optimal anticancer therapy. In the setting of cancer immunotherapy multiple steps are central for orchestrating an optimal immunological response, including i) antigen release and presentation, ii) innate and adaptive immune activation, iii) sustained reversal of immunosuppression and iv) inhibition of immune effector cell exhaustion. The possibility to tailor release kinetics of multiple APIs using HIP complexation and ICell compositional optimization can secure that all steps are supported from a single ICell. Importantly, through optimized release orchestration it can be assured that minimal spill-over from e.g., chemotherapeutic being released and having activity in the adaptive phases of the immune response, which would be problematic for rapidly dividing and sensitive cell e.g., lymphocytes.

In one embodiment an immunogenic cell death inducing API, e.g., a chemotherapeutic, is HIP complexed or the ICell composition is optimized for rapid, high dose, short term release, e.g., less than three days. This can be combined with an innate and adaptive immune activating API, e.g., a TLR, RIG-1, Sting or MDA-5 agonist with intermediate release period of, e.g., 14 days. This can be further combined with an immune modulation and immune polarizing agent, e.g., a TGFb -, IDO -, beta-catenin -, GSK-3 - , A2AR - or Arg1 - inhibitor with a release period of, e.g., 14-28 days, achieved by HIP complexation and ICell composition. Finally, all combinations or parts hereof can have an inhibitor of T cell exhaustion included in the ICell, e.g., PD-L1, PD-1, CTLA-4 inhibitor with a long release period of e.g., 4-8 weeks achieved by HIP complexation and ICell optimization.

In one embodiment a chemotherapeutic, including but not limited to doxorubicin, mitoxantron, danorubicin, epirubicin, cisplatin, cyclophophamide, is HIP complexed and co-formulated with a TLR7/8 agonist and / or TGFb inhibitor in a ICell composition.

In one embodiment a chemotherapeutic, including but not limited to doxorubicin, mitoxantron, danorubicin, epirubicin, cisplatin, cyclophophamide, is HIP complexed and co-formulated with a R848 and RepSox in a ICell composition.

In one embodiment a chemotherapeutic, including but not limited to doxorubicin, mitoxantron, danorubicin, epirubicin, cisplatin, cyclophophamide, is HIP complexed and co-formulated with a R848 and galunisertib in a ICell composition.

In one embodiment a doxorubicin is HIP complexed and co-formulated with a R848 and RepSox in a ICell composition.

In one embodiment a mitoxantrone is HIP complexed and co-formulated with a R848 and RepSox in a ICell composition.

In one embodiment a chemotherapeutic, including but not limited to doxorubicin, mitoxantron, danorubicin, epirubicin, cisplatin, cyclophophamide, is combined with an immune checkpoint inhibitor, including but not limited to inhibitors of PD-L1, PD-1, CTLA-4, TIM-3, LAG-3, and co-formulated with a TLR7/8 agonist and / or TGFb inhibitor in a ICell where release kinetics are optimized for all or selected APIs though HIP complexation and / or optimization of ICell composition.

In one embodiment a chemotherapeutic, including but not limited to doxorubicin, mitoxantron, danorubicin, epirubicin, cisplatin, cyclophophamide, is combined with an immune checkpoint inhibitor, including but not limited to inhibitors of PD-L1, PD-1, CTLA-4, TIM-3, LAG-3, and co-formulated with R848 and RepSox in a ICell where release kinetics are optimized for all or selected APIs though HIP complexation and / or optimization of ICell composition.

In one embodiment a chemotherapeutic, including but not limited to doxorubicin, mitoxantron, danorubicin, epirubicin, cisplatin, cyclophophamide, is combined with an immune checkpoint inhibitor, including but not limited to inhibitors of PD-L1, PD-1, CTLA-4, TIM-3, LAG-3, and co-formulated with R848 and galunisertib in a ICell where release kinetics are optimized for all or selected APIs though HIP complexation and / or optimization of ICell composition.

In one embodiment doxorubicin and an immune checkpoint inhibitor, including but not limited to inhibitors of atezolizumab, Ipilimumab, atezolizumab, avelumab, durvalumab, pembrolizumab, nivolumab, cemiplimab, spartalizumab, CN105705489A, BMS1166, and co-formulated with R848 and Repox in a ICell where release kinetics are optimized for all or selected APIs though HIP complexation and / or optimization of ICell composition.

In one embodiment doxorubicin and an immune checkpoint inhibitor, including but not limited to inhibitors of atezolizumab, Ipilimumab, atezolizumab, avelumab, durvalumab, pembrolizumab, nivolumab, cemiplimab, spartalizumab, CN105705489A, BMS1166, are co-formulated with R848 and galunisertib in a ICell where release kinetics are optimized for all or selected APIs though HIP complexation and / or optimization of ICell composition

In one embodiment doxorubicin and BMS1166 or CN105705489A iare co-formulated with R848 and RepSox in a ICell where release kinetics are optimized for all or selected APIs though HIP complexation and / or optimization of ICell composition.

In additional embodiments, antibody-based therapeutics including monoclonal antibodies as well as derivatives or analogues thereof, including variable domain (Fv) fragments, single chain Fv (scFv) fragments, Fab' fragments, F(ab')2 fragments, single domain antibodies; antibody fragments, humanized antibodies, and antibody fragments; multivalent versions of the foregoing prepared in HIP complexes formulated in a ICell compositions antibodies. Non-limiting examples include atezolizumab, Ipilimumab, atezolizumab, avelumab, durvalumab, pembrolizumab, nivolumab, cemiplimab, spartalizumab, herceptin, erbitux, avastin, rituxan, simulect, dostarlimab, enbrel, adalimumab, and remicade.

In one embodiment an antibody-based therapeutic, including but not limited to atezolizumab, Ipilimumab, atezolizumab, avelumab, durvalumab, pembrolizumab, nivolumab, cemiplimab, spartalizumab, herceptin, erbitux, avastin, rituxan, simulect, dostarlimab, enbrel, adalimumab, and remicade is HIP complexed and formulated in a ICell composition. In one embodiment an antibody-based therapeutic, including but not limited to atezolizumab, Ipilimumab, atezolizumab, avelumab, durvalumab, pembrolizumab, nivolumab, cemiplimab, spartalizumab, herceptin, erbitux, avastin, rituxan, simulect, dostarlimab, enbrel, adalimumab, and remicade is HIP complexed and co-formulated with a TLR7/8 agonist and / or TGFb inhibitor in a ICell composition. In one embodiment an antibody-based therapeutic, including but not limited to atezolizumab, Ipilimumab, atezolizumab, avelumab, durvalumab, pembrolizumab, nivolumab, cemiplimab, spartalizumab, herceptin, erbitux, avastin, rituxan, simulect, dostarlimab, enbrel, adalimumab, and remicade is HIP complexed and co-formulated with R848 and / or RepSox in a ICell composition. In one embodiment an antibody-based therapeutic, including but not limited to atezolizumab, Ipilimumab, atezolizumab, avelumab, durvalumab, pembrolizumab, nivolumab, cemiplimab, spartalizumab, herceptin, erbitux, avastin, rituxan, simulect, dostarlimab, enbrel, adalimumab, and remicade is HIP complexed and co-formulated with R848 and / or galunisertib in a ICell composition.

In one embodiments of the disclosure, HIP complexes formulated in a ICell composition may contain disease-associated antigens, such as one or more cancer associated antigen, one or more cancer associated neo-antigens, one or more pathogen-associated antigen or one or more degenerative-disorder-associated antigen. The term "disease-associated antigens" can relate to proteins produced in disease associated cells that have an abnormal structure and/or an abnormal expression pattern compared to non-disease associated cells. Abnormal proteins are also produced by cells infected with oncoviruses, e.g. EBV and HPV.

In one embodiments of the disclosure, HIP complexes may contain one or more disease-associated antigenic peptides and be co-formulated in ICell compositions also containing and releasing one or several immune activating agents such this invention provides a highly immunogenic vaccine based on the co-delivery of HIP complexes and native immune activating agents form the ICell composition capable of presenting an antigen and adjuvant. The combined delivery of adjuvants for improved antigen presentation represents a promising strategy for therapeutic vaccines to elicit an innate immune response by exploiting the major properties of the two components: (1) the strong adjuvanticity provided by immune activation HIP complex or native drug in the ICell composition; and (2) the specific adaptive immune response against antigen(s) HIP complexed in the ICell composition and associated with the targeted disease. For example, HIP complexed antigen may present any disease-associated antigen, such as one or more cancer associated antigen for cancer therapy, one or more pathogenic antigen for treatment of infection, or any other antigen or combination of antigens associated with other diseases, for immune-compromised conditions and/or where strong potentiation of immunity is needed (e.g., in the elderly). In addition, the invention provides a strong immune response important for vaccines such as those against cancer, hepatitis, flu, malaria and HIV. The invention is also useful for any therapy where the presentation of a combination of antigens to the immune system of a patient may be beneficial.

In one embodiment of the disclosure, HIP complexes of single or multiple vaccine peptides and glycopeptides such as, but not limited to, cancer cell antigens; carcinoembryonal antigen, alpha-1-fetoprotein, isoferritin, and fetal sulphoglycoprotein, cc2-H- ferroprotein and γ-fetoprotein, p53, ART-4, BAGE, beta-catenin/m, Bcr-abL CAMEL, CAP-1 , CASP-8, CDC27/m, CD 4/m, CEA, the cell surface proteins of the claudin family, such as CLAUDIN-6, CLAUDIN-18.2 and CLAUDIN-12, c-MYC, CT, Cyp-B, DAM, ELF2M, ETV6-AML1 , G250, GAGE, GnT-V, Gapl OO, HAGE, HER-2/neu, HPV-E7, HPV-E6, HAST-2, hTERT (or hTRT), LAGE, LDLR/FUT, MAGE- A, preferably MAGE-A1 , MAGE-A2, MAGE- A3, MAGE-A4, MAGE- A5, MAGE-A6, MAGE-A7, MAGE-A8, MAGE-A9, MAGE-A10, MAGE-A1 1 , or MAGE- A12, MAGE-B, MAGE-C, MART- 1 /Melan-A, MC1 R, Myosin/m, MUC1 , MUM-1 , -2, -3, NA88-A, NF1 , NY-ESO-1 , NY-BR-1 , pl 90 minor BCR-abL, Pm I/RARa, PRAME, proteinase 3, PSA, PSM, RAGE, RUI or RU2, SAGE, SART-1 or SART-3, SCGB3A2, SCP 1 , SCP2, SCP3, SSX, SURVrVIN, TEL/AMLI , TPI/m, TRP-1 , TRP-2, TRP-2/1 NT2, TPTE and WT, preferably WT-1 and antigens for peptide and glycopeptide vaccines for infectious diseases including, but not limited to, circumsporozoite protein (CSP), apical membrane antigen 1 (AMA-1), UK39, IC41 (IPEP 83, 84, 87, 89, and 1426), gp41, gp120, gp100, and antigens for vaccines against neurodegenerative, storage and autoimmune diseases, including but not limited to, Tau, Aβ(1-42).

The vaccine peptides HIP complexes may be prepared and formulated in ICell compositions and delivered as single peptides or multiple disease or cancer associated antigenic peptides.

In one embodiment of the invention personalized peptide(s) for neoantigen vaccines, synthesized based on patient tissue samples analyses, sequencing and cancer and/or neoantigen identification, are HIP complexed and formulated in a ICell composition. The HIP complexed or native peptides, or a combination of these may be formulated as single or multipeptide combinations in the ICell and may include immune activating adjuvant either as native compounds the ICell composition or complexed using HIP and formulated in the ICell composition, examples of adjuvants include, but are not limited to Poly(I:C), Poly ICLC, STING agonists, MDA5, TLR agonists (e.g., TLR3, TLR4, TLR7, TLR8, and TLR9), RIG-1 agonists, interleukins, including but not limited to IL-1, IL-2, IL-15, IL-15 sushi, IL-6, IL-12 single chain IL-12p35, IL-12p40, IL-12p70, IL-12, cytokines and chemokines, including but not limited to CCR7, CCL20, CCR7, CXCL9, CXCL10, CXCL11, FLT3L, OX40L, TNFs and IFNs, immune modulators, including but not limited to inhibitors of beta-catenin, IDO, Arg1, A2AR, GSK3, native or HIP complexed APIs including but not limited to 1018 ISS, aluminium salts, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, JuvImmune, LipoVac, MF59, monophosphoryl lipid A, Montanide IMS 1312 VG, Montanide ISA 206 VG, Montanide ISA 50 V2, Montanide ISA 51 VG, OK-432, OM-174, OM-197-MP-EC, ISA-TLR2 agonist, ONTAK, PepTel@ vector system, PLG microparticles, resiquimod, SRL172, virosomes and other virus-like particles, YF-17D, VEGF trap, R848, beta-glucan, Pam3Cys, Pam3CSK4, acrylic or methacrylic polymers, copolymers of maleic anhydride, and QS21 stimulon. a co-stimulatory ligand, a TNF ligand, an Ig superfamily ligand, CD28, CD80, CD86, ICOS, CD40L, OX40, CD27, GITR, CD30, DR3, CD69, or 4-1BB. In one embodiment a single or combination of native or HIP complexed vaccine epitopes are co-delivered with an TLR agonist and a TGFb inhibitor. In one embodiment a single or combination of native or HIP complexed vaccine epitopes are co-delivered with an TLR7/8 agonist and an immune checkpoint inhibitor, including but not limited to HIP complexed or native inhibitors of PD-1, PD-L1, CTLA-4, LAG3, and TIM-3.

In one embodiment HIP complexed or native peptides or proteins as single or multipeptide vaccine combinations may be co-formulated with an TLR7/8 agonist and a TGFb inhibitor in a ICell composition.

In one embodiment HIP complexed or native peptides or proteins as single or multipeptide vaccine combinations may be co-formulated with an TLR7/8 agonist and an immune checkpoint inhibitor, including but not limited to inhibitors of PD-1, PD-L1, CTLA-4, and TIM-3 in a ICell composition.

In one embodiment HIP complexed or native peptides or proteins as single or multipeptide vaccine combinations may be co-formulated with R848 and RepSox in a ICell composition

In one embodiment HIP complexed or native peptides or proteins as single or multipeptide combinations may be co-formulated with a R848 agonist and galunisertib in a ICell composition.

In one embodiment of the invention, therapeutic agents that acts as agonist or antagonists for cytokines, chemokines, and growth factors can be also used for HIP complexing and formulation in a ICell composition. Non-limiting examples of such cytokines are lymphokines, monokines, chemokines, leukotrins and hormones. Examples include agents that are active agonists or antagonist for such as but not limited to growth factors including vascular endothelial growth factor (VEGFs), hepatic growth factor; fibroblast growth factor (FGFs); integrin; thrombopoietin (TPO); nerve growth factors such as NGF-β; platelet growth factor; transforming growth factors (TGFs, BMPs); insulin-like growth factor-I and -II (IGFs); erythropoietin (EPO); osteoinductive factors; interferons, such as interferon-a, -β and -γ; colony stimulating factors (CSFs), such as macrophage-CSF (M-CSF), granulocyte-macrophage-CSF (GM-CSF), granulocyte-CSF (GCSF) and the like; interleukins (ILs); tumor necrosis factors, such as TNF-α or TNF-β; and other polypeptide factors, including LIF and kit ligand (KL). As used herein, the term cytokine includes proteins from natural sources or from recombinant sources (e.g., from T-cell cultures and biologically active equivalents of the native sequence cytokines). In one embodiment of the invention single or multiple growth factors are used for HIP complexing and formulation in a ICell composition in combination with a single or combination of multiple antibiotics for the treatment of diseases, inclduing but not limited to non-healing bone fractures, vascular defects, degenerative diseases, chronic infected wounds, diabetic ulcers.

In one embodiment of the invention, hormones or therapeutic synthetic hormone agonist or antagonists can be also used for HIP complexing and formulation in a ICell composition. Non-limiting examples of hormones for and therapeutically active agents that are active agonists or antagonist for hormones are peptide hormones and peptide hormone analogues: human growth hormone; parathyroid hormone (PTH); thyroxine; insulin; proinsulin; relaxin; prorelaxin; glycoprotein hormones; prolactin; placental lactogen; tumor necrosis factor-a and -β; mullerian-inhibiting substance; gonadotropin-associated peptide; inhibin; activin; adrenocorticotropic hormone (ACTH), amylin, angiotensin, atrial natriuretic peptide (ANP), calcitonin, cholecystokinin (CCK), gastrin, ghrelin, glucagon, follicle-stimulating hormone (FSH), insulin, Glucagon-like peptide-1 (GLP-1), leptin, luteinizing hormone (LH), melanocyte-stimulating hormone (MSH), oxytocin, parathyroid hormone (PTH), prolactin, renin, somatostatin, thyroid-stimulating hormone (TSH) thyrotropin-releasing hormone (TRH), vasopressin (arginine vasopressin (AVP) or anti-diuretic hormone (ADH)), vasoactive intestinal peptide (VIP), and steroid hormones and analogues: Glucocorticoids: alclometasone, prednisone, dexamethasone, triamcinolone, cortisone, Mineralocorticoid: fludrocortisone Vitamin D: dihydrotachysterol, Androgens: oxandrolone, oxabolone, nandrolone (also known as anabolic-androgenic steroids or simply anabolic steroids), Oestrogens: diethylstilbestrol (DES) and ethinyl estradiol (EE), Progestins: norethisterone, medroxyprogesterone acetate, hydroxyprogesterone caproate, and steroid hormone antagonists and analogues: cyproterone acetate, mifepristone, and gestrinone.

In one embodiment of the invention, therapeutic peptides, glycopeptides, glycoproteins, lipidated peptides, radionuclide-peptide conjugates and drug-peptide conjugates can be also used for HIP complexing and formulation in a ICell composition. Non-limiting examples include,
CXCR4 receptor antagonist LY-2510924, Romiplostim, DOTATATE, DOTATOC, Zoptarelin, NA-1 (HIV-TAT peptide), Peginesatide, silengitide, Liraglutide, GLP-1 receptor agonists derived from the exendin-4 structures, Dopastatin (BIM-23A760), etelcalcetide, Mibenratide, Balixafortide, Etelcalcetide, Abaloparatide, Plecanatide, Virginiamycin M1, Dalfopristin, Quinupristin, Ramoplanin, Telavancin, Dalbavancin, Etelcalcetide, dulaglutide, Plecanatide, Larazotide, carfilzomib, albiglutide receptor agonist romiplostim (AMG531), abaloparatide, Pegfilgrastim, nerinetide, Sermorelin, Anakinra, Gramicidin D, Cetrorelix, Abarelix, Romidepsin, Spirapril, Lanreotide, leuprotide, lanreotide, linaclotide, afamelanotide, derivatives of the endogenous peptides apelin, adrenomedullin, and neuromedin U, elcatonin, Thymalfasin, eledoisin, enalapril, bivalirudin, cemadotin, exenatide, pexiganan, ziconotide, chlorotoxin I-135 conjugate, Elisidepsin, dalazatide, SOR-C13, corticotropin, lypressin, vasopressin, Eledoisin, somatostatin, calcitonin, ornipressin, desmopressin, terlipressin, ambamustine, tetracosactide, elcatonin, felypressin, saralasin, cargutocin, buserelin, thiazolidine, ligdunine, leuprorelin, thymopentin, triptorelin, Sprifermin, Corticorelin, Leptin, goserelin, lisinopril, romurtide, glucagon, deslorelin, Plitidepsin, gonadorelin, ghrelin, motilin, conjugates of somatostatin-like peptides, macimorelin, nafarelin, histrelin, lanreotide, semaglutide, Tigapotide, Aclerastide, Albusomatropin, Anamorelin, G17DT, Insulin peglispro, Lenomorelin, Selepressin, Somapacitan, Cibinetide, Taspoglutide, Thymosin beta-4, Tirzepatide, Ularitide, Vapreotide, Lenograstim, Vosoritide, Zoptarelin doxorubicin, Angiotensin 1-7, Bombesin, Cenderitide, Deslorelin, Gastric inhibitory polypeptide, MK-3207, Olcegepant, Zilucoplan, Pancreatic Polypeptide, Peptide YY (3-36), Pirnabine, Somatoprim, Somatropin pegol, Thyrotropin, TT-232, BPI-3016, NBI-6024, NN344, TERT572Y, NP-12, PL120131, carperitide, Teverelix, glatiramer, eptifibatide, cetrorelix, ganirelix, P-15, atosiban, taltirelin, aviptadil, nesiritide, ovokinin, novokinin, bivalirudin, carbetocin, teriparatide, abarelix, enfuvirtide, ziconotide, abaloparatide, Ramucirumab, pazopanib, CBO-P11, Nesfatin-1, Polaprezinc, pramlintide, exenatide, romiplostim, Avexitide, Calcitonin gene-related peptide, Parathyroid Hormone-Related Protein 1-36, Peginterferon alfa-2a, degarelix, icatibant, Tiplimotide, mifamurtide, FOL-005, liraglutide, tesamorelin, peginesatide, Beinaglutide, lucinactant, pasireotide, teduglutide, linaclotide, lixisenatide, pazopanib, cabozantinib, Elobixibat, Rapastinel, lenvatinib, axitinib, vandetanib, Cediranib, Ipafricept, axitinib, Recombinant CD40-ligand, Aspartyl-alanyl-diketopiperazine, cabozantinib, albiglutide, dulaglutide, etelcalcetide, Plecanatide, bortezomib, ixazomib, carfilzomib, Forigerimod, sunitinib, vemurafenib, dabrafenib, encorafenib, trametinib, binimetinib, sorafenib, pazopanib, Setmelanotide, Plecanatide, Menotropins, Davunetide, Oprozomib, Pegdinetanib, Nagrestipen, Etelcalcetide, Macimorelin, Etelcalcetide, Murepavadin, Edratide, Pegcetacoplan, Erythropoietin, Pasireotide, PepGen P-15, Bremelanotide, nintedanib, Afamelanotide, Angiotensin II, Cyclosporin A, Eptifibatide, Difelikalin, idelalisib, Pegbelfermin, Voclosporin, Desmopressin, Pegvisomant, Zoptarelin Doxorubicin, (AN-152, AEZS-108), ANG1005, G202 (Mipsagargin), BIM-23A760, ⁶⁸Ga gozetotide, Lu177-dotatate, [111In-DTPA-D- Phe1]-octreotide, Zoptarelin Doxorubicin, AN-152, AEZS-108, GRN1005, Paclitaxel poliglumex CT2103, EP-100, BIM-23A760, Becaplermin, CGC 1072, KAI-1455 KAI-1678, KAI-9803, PTH(1-84), XG-102, DTS-108, DTS-201, BT-1718, 177Lu- PSMA-617, GLP-1-estrogen conjugate, GLP-1-estradiol conjugate, Pentetreotide, Glucagon-T3 Conjugate, RGD-doxorubicin conjugate, and EETI-2.5Z-Val-Ala-PABC-gemcitabine.

In one specific embodiment of the invention a parathyroid hormone-related protein (PTHrP) analog can be also used for HIP complexing and formulated on a ICell composition or formulated without complexation in a ICell composition.

In one embodiment a parathyroid hormone-related protein (PTHrP) analog, including but not limited to abaloparatide, teriparatide or PTH(1-84) is HIP complexed and formulated in a ICell composition.

In one embodiment abaloparatide is HIP complexed with SDS, *1-decane sulphonate* and the like, and formulated in a ICell composition.

In some embodiments of the present invention, antibiotic agent(s) belonging to the following groups may be HIP complexed and formulated in the ICell composition according to the present invention. One of the advantages of incorporating one or more antimicrobial agents in the ICell composition according to the present invention is that it can provide a localized antimicrobial agent(s) release depot with a much higher local concentration of the agent or agents than would be possible by systemic treatment with the same. The flexible and precise thin needle injection technology can further allow surgeon to accurately decorate the infected region(s)/lesion(s) to be treated.

In one embodiment of the invention, HIP complexes of antimicrobial agents such as, without limitation: Aminoglycosides, Amikacin, Gentamicin, Kanamycin, Neomycin, Netilmicin, Tobramycin, Paromomycin, Streptomycin, Spectinomycin(Bs), Ansamycins, Geldanamycin, Herbimycin, Rifaximin, Carbacephem, Loracarbef, Carbapenems, Ertapenem, Doripenem, Meropenem, Cefadroxil, Cefazolin, Cephradine, Cephapirin, Cephalothin, Cefalexin, Cefaclor, Cefoxitin, Cefotetan, Cefamandole, Cefmetazole, Cefonicid, Loracarbef, Cefprozil, Cefuroxime, Cefdinir, Cefditoren, Cefotaxime, Cefpodoxime, Ceftibuten, Ceftizoxime, Moxalactam, Ceftriaxone, Cefepime, Ceftaroline fosamil, Ceftobiprole, Glycopeptides, Teicoplanin, Vancomycin, Telavancin, Dalbavancin, Oritavancin, Lincosamides(Bs), Clindamycin, Lincomycin, Lipopeptide, Daptomycin, Macrolides(Bs), Azithromycin, Clarithromycin, Erythromycin, Roxithromycin, Telithromycin, Spiramycin, Fidaxomicin, Monobactams, Aztreonam, Nitrofurans, Furazolidone, Nitrofurantoin(Bs), Oxazolidinones(Bs), Linezolid, Posizolid, Radezolid, Torezolid, Penicillins, Amoxicillin, Ampicillin, Azlocillin, Dicloxacillin, Flucloxacillin, Mezlocillin, Methicillin,Nafcillin,Oxacillin,Penicillin G,Penicillin V,Piperacillin, Temocillin, Ticarcillin, Amoxicillin/clavulanate, Ampicillin/sulbactam, Piperacillin/tazobactam, Ticarcillin/clavulanate, Polypeptides, Bacitracin, Colistin, Polymyxin B, Polymyxin E, Ciprofloxacin, Enoxacin, Gatifloxacin, Gemifloxacin, Levofloxacin, Lomefloxacin, Enrofloxacin, Moxifloxacin, Nadifloxacin, Nalidixic acid, Norfloxacin, Ofloxacin, Trovafloxacin, Grepafloxacin, Sparfloxacin, Temafloxacin, Mafenide, Sulfacetamide, Sulfadiazine, Silver sulfadiazine, Sulfadimethoxine, Sulfamethizole, Sulfamethoxazole, Sulfanilimide (archaic), Sulfasalazine, Sulfisoxazole, Sulfonamidochrysoidine (archaic), Tetracyclines(Bs), Demeclocycline, Doxycycline, Metacycline, Minocycline, Oxytetracycline,Tetracycline, Clofazimine, Dapsone, Capreomycin, Cycloserine, Ethambutol(Bs), Ethionamide, Isoniazid, Pyrazinamide, Rifampicin, Rifabutin, Rifapentine, Streptomycin, Arsphenamine, Chloramphenicol(Bs), Fosfomycin, Fusidic acid, Metronidazole, Mupirocin, Platensimycin, Quinupristin/Dalfopristin, Thiamphenicol, Tigecycline(Bs), Tinidazole, Trimethoprim(Bs), Vancomycin, Doxycyline, Ceftobiprole[, Ceftaroline, Dalbavancin, Fusidic acid, Mupirocin, Omadacycline, Oritavancin, Tedizolid, Telavancin, Tigecycline, Ceftolozane/tazobactam, etambutol, isoniazid, pyrazinamide, aciclovir, Valaciclovir, efavirenz, emtricitabin, tenofovirdisoproxil, Rilpivirine, penicillin, Trimethoprim-sulfamethoxazole, rifampicin, etambutol, isoniazid, pyrazinamide, voriconazole, amphotericin B, caspofungin, flucytosine, itraconazole, and the like, but not limited to those are prepared and formulated in a ICell composition.

In some embodiments of the present invention, the HIP complexes of bioactive agents in a ICell composition may be used for treating or preventing a bacterial, fungal, viral, parasite infection in a subject or an organism. In this context, the microbial infection may be caused by a Gram negative or a Gram positive bacterium, fungus, virus or parasite (infestation, e.g., helminths, protozoa). The subject affected by the bacterial infection may be a mammal, such as a human being. The acute and chronic diseases include, but is not limited to endocarditis, abscesses, phlegmons, respiratory and pulmonary infections, central nervous system infections, otitis interna, media and externa, eye infections, bone and joint infections, urinary tract infections, gastrointestinal infections and skin and soft tissue infections including wound infections, pyoderma and dermatitis, chronic and acute wound infections, diabetic ulcers, diabetic foot ulcers, chronic ulcers, implant associated osteomyelitis, and pyoderma.

In one embodiments of the disclosure, antibiotic are HIP complexed with but not limited to: antimicrobial peptides; cecropins, andropin, moricin, ceratotoxin and melittin, magainin, dermaseptin, bombinin, brevinin-1, esculentins and buforin II, CAP18, LL37, abaecin, drosocin, apidaecin, diptericin, attacin, prophenin, indolicidin, Maximin H5, dermicidin, defensins, protegrin, tachyplesins, and brevinins or short chain unsaturated fatty acids, including but not limited to DSF, cis-2-decenoic acid, hexanoic acid, heptanoic acid, octanoic acid, nonanoic acid, decanoic acid, undecanoic acid, dodecanoic acid, tridecanoic acid, tetradecanoic acid, pentanoic acid, hexadecanoic acid, heptadecanoic acid, octadecanoic acid, and nonadecanoic acid, such as alkenoic acids include 2-hexenoic acid, 2-heptenoic acid, 2-octenoic acid, 2-nonenoic acid, 2-tuidecenoic acid, 2-dodecenoic acid, 2-tridecenoic acid, 2-tetradecenoic acid, 2-pentadecenoic acid, 2-hexadecenoic acid, 2-heptadecenoic acid, 2-octadecenoic acid, and 2-nonadecenoic acid. These may be cis or trans isomers.

In one embodiment HIP complexes may contain one or more antimicrobial peptides co-formulated in ICell compositions also containing and releasing one or several antimicrobial agents as native APIs or as HIP complexed APIs in a ICell composition.

In one embodiment combinations of r rifampicin, rifampin, gentamycin, levofloxacin, ciprofloxacin, linezolid, colistin, dalbavancin, tobramycin, amoxicilin, Cefepime, Imipenem, Meropenem, Ertapenem, Aztreonam, Amikacin, Moxifloxacin, Minocycline, Doxycycline, Daptomycin, Fosfomycin, Metronidazole, clindamycin or vancomycin are formulated in a ICell formulation as HIP complexes, with anionic counterions including but not limited to Sodium hexanoate, Sodium decanoate, Sodium myristate, Sodium oleate, Pamoic acid disodium salt, Sodium deoxycholate, Benezenesulfonic acid monohydrate, Sodium 2-naphthalenesulfonate, Camphorsulfonic acid, Sodium 1,2-ethanesulfonate, Sodium 1-heptanesulfonate, Sodium 1-octane-sulfonate monohydrate, Sodium 1-decanesulfonate, Sodium dodecyl sulfate (SDS), Sodium dodecyl-benzenesulfonate (DBS), Linoleic acid, Cis-2-decenoic acid, Sodium docusate, Curcumin or incorporated as native APIs in any combination hereof in a ICell composition.

In one embodiment combinations of rifampicin, rifampin, gentamycin, levofloxacin, ciprofloxacin, linezolid, colistin, dalbavancin, tobramycin, amoxicilin, Cefepime, Imipenem, Meropenem, Ertapenem, Aztreonam, Amikacin, Moxifloxacin, Minocycline, Doxycycline, Daptomycin, Fosfomycin, Metronidazole, clindamycin or vancomycin are formulated in a ICell formulation as HIP complexes, with cationic counterions including but not limited to Tetraethyl ammonium bromide, Benzyl trimethyl ammonium chloride, Cetyl trimethyl ammonium bromide (CTAB), N-benzyl-2-phenylethanamine (benethamine), Dodecylamine, Tetrahexyl ammonium bromide (THAB), Didodecyl dimethyl ammonium bromide (DDAB), Octadecylamine, Tetraoctyl ammonium bromide (TOAB), Tridodecyl methyl ammonium chloride (TDMAC) or incorporated as native APIs in any combination hereof in a ICell composition.

In one embodiment clindamycin is HIP complexed in a ICell formulation. In one embodiment HIP complexed or native clindamycin is formulated with HIP complexed vancomycin in a ICell formulation.

In one embodiment HIP complexed vancomycin and HIP complexed gentamycin are formulated in a ICell composition. In one embodiment the combination further includes the addition of native clindamycin or linezolid to ICell composition.

In one embodiment gentamycin is combined in a HIP complex with a counterion including but not limited to SDS, DSF, docusate or cis-2-decenoic acid and formulated in a ICell composition.

In one embodiment of the invention gentamycin is combined in a HIP complex with a counterion including but not limited to SDS, docusate, DSF or cis-2-decenoic acid and co-formulated with native clindamycin or linezolid in a ICell composition.

In one embodiment gentamycin is HIP complexed with a counterion including but not limited to SDS, DSF, docusate or cis-2-decenoic acid and vancomycin is HIP complexed with a counterion including but not limited to SDS, DSF, docusate or cis-2-decenoic acid and co-formulated in a ICell composition.

In one embodiment vancomycin is HIP complexed with a counterion including but not limited to SDS, DSF, docusate or cis-2-decenoic acid and formulated in a ICell composition.

In one embodiment gentamycin is HIP complexed with a counterion including but not limited to SDS, DSF, docusate or cis-2-decenoic acid and vancomycin is HIP complexed with a counterion including but not limited to SDS, DSF, docusate or cis-2-decenoic acid and co-formulated with native clindamycin or linezolid in a ICell composition.

In one embodiment rifampicin or rifampin is HIP complexed with a counterion including but not limited to SDS, DSF, docusate or cis-2-decenoic acid and vancomycin is HIP complexed with a counterion including but not limited to SDS, DSF, docusate or cis-2-decenoic acid and co-formulated in a ICell composition.

In one embodiment rifampicin or rifampin is HIP complexed with a counterion including but not limited to SDS, DSF, docusate or cis-2-decenoic acid and vancomycin is HIP complexed with a counterion including but not limited to SDS, DSF, docusate or cis-2-decenoic acid and co-formulated with native clindamycin or linezolid in a ICell composition.

In one embodiment rifampicin or rifampin is HIP complexed with a counterion including but not limited to SDS, DSF, docusate or cis-2-decenoic acid and gentamycin is HIP complexed with a counterion including but not limited to SDS, DSF, docusate or cis-2-decenoic acid and co-formulated in a ICell composition.

In one embodiment rifampicin or rifampin is HIP complexed with a counterion including but not limited to SDS, DSF, docusate or cis-2-decenoic acid and gentamycin is HIP complexed with a counterion including but not limited to SDS, DSF, docusate or cis-2-decenoic acid and co-formulated with native clindamycin or linezolid in a ICell composition.

In one embodiment the HIP complexed bioactive agents and/or imaging agent in the ICell composition provides an injectable liquid ready for use in the treatment by injection in tissues, including soft tissue, organs, and bones for e.g. as part of a surgical treatment, post-surgical treatment or radiation therapy, in order to treat disorders of supportive tissue in a human or animal by regenerating and healing of bone tissue, soft tissue, soft tissue infections and/or treating bone infections. Such disorders may be, but not limited to bone loss, peritendinous adhesion formation, excessive granulation, chronic wounds, scar tissue, fibrosis, bone fracture, bone non-union, cartilage regeneration, bone trauma, cartilage regeneration, osteoarthritis, musculoskeletal diseases and disorder, soft tissue infections and/or osteomyelitis and bioactive agents include, but are not limited to antioxidants, vitamins, hormones, antibiotics, cytostatics (e.g., 5-FU), bisphosphonates, or growth factors and analogues of these, including but not limited to BMPs, VEGFs (VEGF-A, VEGF-B, VEGF-C, VEGF-D, and placental growth factor (PIGF)), FGFs, TGFb1-3, BMPs, BMP-4, M-CSF, G-CSF, EGFs, EPO, HGFs, IGFs, OGFs, CGRPs,NRGs, PDGF, KGF, ILs, GDF9, GDNF, Calcitonin gene-related peptides (CGRP), Thrombin Peptide 508 (Chrysalin), and β-tricalcium phosphate, Teriparatide, BMPs belonging to the transforming growth factor β (TGFβ) superfamily, including but not limited to TGFβs, activins/inhibins, Mullerian-inhibiting substance (MIS) and glial cell line-derived neurotrophic factor, GFOGER (glycine-phenylalanine-hydroxyproline-glycine-glutamate-arginine), SVVYGLR peptide, amide derivatives of stilbene, Bergenin, P15, RADA16-I, RANKL-binding peptide, core-binding factor A1 (CBFA1) Runt-related transcription factor-2 (RUNX-2), AC-100, mechano growth factor E, Osteoactivin and B2A2-K-NS (B2A) collagen-mimetic peptide HIP complexed and formulated in a ICell composition.

In one embodiment of the invention a bioactive agent is combined with a native antimicrobial agent or a HIP complexed anyimicrobial agent, or a combination hereof in a ICell composition. In one embodiment of the invention a growth factor for supporting bone formation, including but not limited to TGFb, BMPs (BMP-4), FGF agonists and analogues, and a native antimicrobial agent or a HIP complexed antimicrobial agent, or a combination hereof in a ICell composition.

In one embodiment solid support material is included in the ICell composition, solid support material, examples include but are not limited to calcium sulphate, calcium phosphate, hydroxyapatite, silver nanoparticles, silver microparticles, organophosphates, whitlockite (WH), octacalcium phosphate, tetracalcium phosphate, beta-TCP, zirconia phosphate, silver nitrate (AgNO3), chitosan, PLGA, PEG, polycaprolactone (PCL), poly-I-lactic acid, monocalcium phosphate monohydrate, bioglass, polytetrafluoroethylene, polyethylene-oxide-terephtalane, polybuthylene-terephtalate-polymer, titanium, titanium salts, polypropylenefumarate (PPF), poly-methylmethaacrylate (PMMA), tricalcium phosphate, hydroxyapatite.

In one embodiments of the invention the HIP complexed bioactive agents and/or imaging agent in the ICell composition provides an injectable liquid ready for use in clinical treatment of infections or inflammation or immune reactivity and autoimmunity associated with, but not limited to catheters, stent, grafts, pins, screws, needles, plates, artificial joints, passive and active biomaterials, artificial organs, allografts, orthografts, autografts, xenografts, bioactive devices and implants. The ICell composition provides a flexible technology to both inject in around the device or material and in the surrounding tissues affect by disease e.g., infections, pain, inflammation, allergic foreign body responses. Possible HIP complexed bioactive agents can further provide local thromboresistance and thereby inhibit: (i) protein and cell adsorption, (ii) thrombin and fibrin formation, and (iii) platelet activation and aggregation to inhibit implant-induced coagulation.

In some embodiments, the HIP complexes of bioactive agents in the ICell of the present invention may be used for targeting an inflamed site in a subject. Therefore, in such embodiments, the technology of the present invention may be used for treating, preventing, monitoring and/or diagnosing a condition or disease associated with an inflammation. Representative conditions include, without limitation: allergies; asthma; neurodegenerative disease; diabetes; hormonal imbalances; autoimmune diseases, such as rheumatoid arthritis and psoriasis; osteoarthritis; osteoporosis; atherosclerosis, including coronary artery disease; vasculitis; chronic inflammatory conditions, such as obesity; ulcers; respiratory inflammations; inflammations caused by viruses; cardiac inflammatory disease, cardiovascular disease and inflammation, vascular disease and inflammation, pelvic inflammatory disease; ovarian epithelia inflammation; gastritis; chronic pancreatitis; chronic cholecystitis and inflammatory bowel disease; inflammation-associated cancers,

In one embodiment of the invention, HIP complexes of bioactive agents are formulated in a ICell composition to elicit sustained immune suppression capabilities as for example desired in the context of autoimmune diseases, infections, allergies and transplantation to avoid detrimental activation and/or overreaction of a subjects' immune system. In one embodiment HIP complexes formulated in ICell composition may inhibit immune reaction developing as result of autogenic/allogeneic/xenogeneic cell transplant, or allogeneic/xenogeneic organ transplant,

In one embodiment the invention of the present disclosure includes the controlled release of multiple HIPs or HIPs in combination with non-hydrophobic ion-paired therapeutics in the injected lesion/region. The flexibility of the injectable liquid ICell gel-forming technology provides optimal inclusion of multiple drug or HIPs for which the release kinetics of the individual HIPs may be controlled to achieve the optimal stimulation from a therapeutic point of view. The present invention provides optimized HIPs of drugs for combinatorial treatment with multiple drugs and matching of their release profile from the gel-forming drug delivery formulation

In one embodiment of the present invention a broader range of drugs, imaging agents and excipients are made available for formulation as HIPs in ICell, which can be combined with surgery, chemotherapy, radiotherapy, immunotherapy and/or ACT in the treatment of disease such as cancer, infectious disease or autoimmune disease in humans or animals.

### Examples and description of figures

### Example 1: Description of co-ions, their logP and pKa for hydrophobic ion pairing.

**Aim:** To list commonly used co-ions for hydrophobic ion-pairing (HIP) and their relevant physicochemical properties.

### Materials and methods:

The pKa and logP values of each molecule were calculated using ChemAxon^{®} software. pKa values are predicted based on the molecule's partial charge distribution of atoms (1,2). Log P is calculated mainly based on the molecule's structure and extent of ionization (3).

### Results and discussion:

Hydrophobic ion pairing involves the ionic interaction between a charged hydrophilic molecule and a co-ion with an opposite charge. In addition, the co-ion needs to have a hydrophobic domain to increase the hydrophobicity of the complex. Tables 1 and 2 show example molecules suitable as anionic or cationic co-ions, respectively.

**Table 1. Log P and pKa values of anionic co-ions for hydrophobic ion pairing.**

| **Compound structure** | **Compound name** | **LogP value** | **pKa value** |
|---|---|---|---|
| 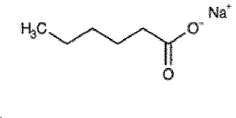 | Sodium hexanoate | 2.01 | 5.1 |
| 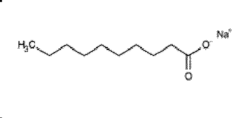 | Sodium decanoate | 4.03 | 5.0 |
| 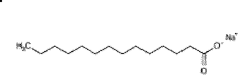 | Sodium myristate | 6.05 | 5.0 |
| 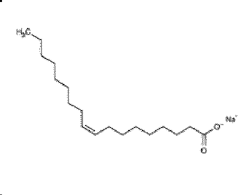 | Sodium oleate | 7.58 | 5.0 |
| 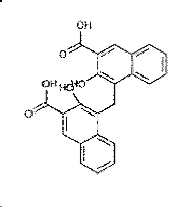 | Pamoic acid disodium salt | 5.52 | 2.7 |
| 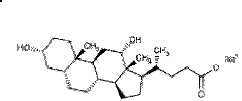 | Sodium deoxycholate | 4.25 | 4.65 |
| 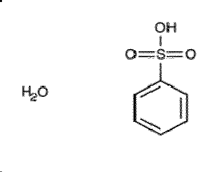 | Benezenesulfonic acid monohydrate | -1.07 | -2.4 |
| 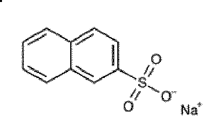 | Sodium 2-naphthalenesulfon ate | 0.11 | -1.8 |
| 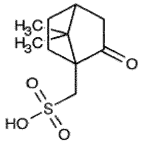 | Camphorsulfonic acid | -1.3 | -0.8 |
| 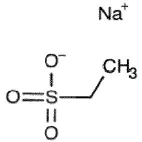 | Sodium 1,2-ethanesulfonate | -4.92 | -1.9 |
| 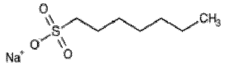 | Sodium 1-heptanesulfonate | 0.35 | -0.5 |
| 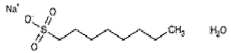 | Sodium 1-octane-sulfonate monohydrate | 0.85 | -0.4 |
| 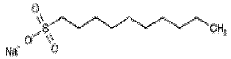 | Sodium 1-decanesulfonate | 1.86 | -0.6 |
| 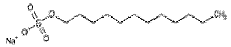 | Sodium dodecyl sulfate (SDS) | 2.83 | -1.5 |
| 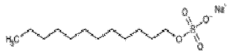 | Sodium dodecyl-benzenesulfonate (DBS) | 4.83 | -1.8 |
| 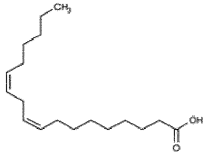 | Linoleic acid | 6.4 | 4.99 |
| 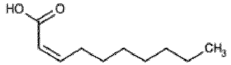 | Cis-2-decenoic acid | 3.59 | 4.83 |
| 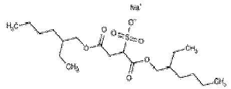 | Sodium docusate | 5.24 | -0.75 |
| 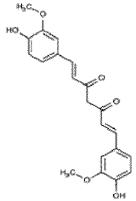 | Curcumin | 4.13 | 8.79 |

**Table 2. Log P and pKa values of cationic co-ions for hydrophobic ion pairing.**

| **Compound structure** | **Compound name** | **LogP value** | **pKa value** |
|---|---|---|---|
| 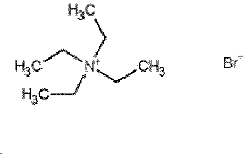 | Tetraethyl ammonium bromide | -2.55 | N/A |
| 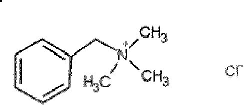 | Benzyl trimethyl ammonium chloride | -2.25 | N/A |
| 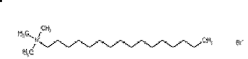 | Cetyl trimethyl ammonium bromide (CTAB) | 2.69 | N/A |
| 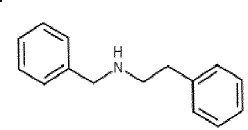 | N-benzyl-2-phenylethanamine (benethamine) | 3.55 | 9.59 |
| 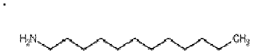 | Dodecylamine | 4.26 | 10.21 |
| 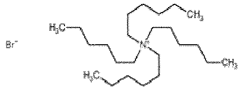 | Tetrahexyl ammonium bromide (THAB) | 4.88 | N/A |
| 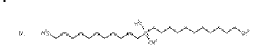 | Didodecyl dimethyl ammonium bromide (DDAB) | 5.79 | N/A |
| 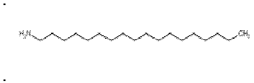 | Octadecylamine | 6.92 | 10.21 |
| 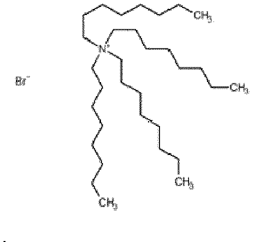 | Tetraoctyl ammonium bromide (TOAB) | 8.44 | N/A |
| 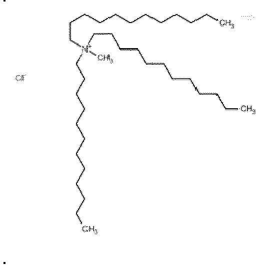 | Tridodecyl methyl ammonium chloride (TDMAC) | 10.67 | N/A |

### Conclusions:

The listed molecules possess a charge and a hydrophobic domain, which make them suitable as co-ions to form hydrophobic complexes with positively or negatively charged hydrophilic molecules.

### References:

1. Prediction of dissociation constant using microconstants, J. Szegezdi and F. Csizmadia, 27th ACS National Meeting, Anaheim, California, March 28-April 1, 2004
2. A method for calculating the pKa values of small and large molecules, J. Szegezdi and F. Csizmadia, American Chemical Society Spring meeting, March 25-29th, 2007
3. Viswanadhan, V. N.; Ghose, A. K.; Revankar, G. R. and Robins, R. K., J.Chem.lnf.Comput.Sci. , 1989 , 29 , 3, 163-172; doi

### Example 2: Methods for HIP complexation

**Aim:** To investigate different methods to form hydrophobic complexes between co-ions and a charged hydrophilic molecule (using the dyes direct black 36 (DB36) and indocyanine green (ICG) as models).

### Materials and methods:

*HIP method 1 (organic solvent-free):* DB36 (5 mg/mL) was dissolved in water. Cetyl trimethyl ammonium (CTAB) was dissolved in water in a 2:1 molar ratio to DB36. 2 mL of DB36 solution was added to a Falcon tube and an equal volume of CTAB solution was added dropwise. The resulting solution was shaken for 2 h at 400 rpm and 25°C.

Then the solution was centrifuged for 10 min at 12500 rpm and 25°C. The supernatant was removed and the formed HIP complex was washed with water. The centrifugation step was repeated and the formed precipitate is the HIP complex.

*HIP method 2 (biphasic metathesis reaction):* DB36 (5 mg/mL) was dissolved in water. CTAB, tetrahexyl ammonium bromide (THAB), tetraoctyl ammonium bromide (TOAB) and tridodecyl methyl ammonium bromide (TDMAC) were used as co-ions. Each co-ion was dissolved in chloroform in a 2:1 molar ratio to DB36. Equal volumes of DB36 and the co-ion solution were added to an Eppendorf tube and shaken for 3.5 h at 700 rpm and 25°C. The resulting HIP complex is present in the lower organic phase.

*HIP method 3 (Bligh-Dyer method):* ICG (5 mg/mL) was dissolved in a 0.01 M HCI solution. Benethamine, dodecylamine, CTAB and tetraethyl ammonium bromide were used as co-ions. Each co-ion was dissolved in methanol in a 1:1 molar ratio to ICG. In an 8-mL glass vial, 1 mL of chloroform, 2 mL of co-ion solution (methanol), and 1 mL of ICG solution (water) were added in that order (methanol without co-ions was used as a control). The vials was gently shaken (i.e., slowly turned over 2-3 times) and incubated for 45 min at room temperature in the dark. Then 1 mL of water was added followed by 1 mL of chloroform. The vial was gently shaken again and incubated for 45 min at room temperature in the dark. The resulting HIP complex is present in the lower organic phase.

### Results and discussion:

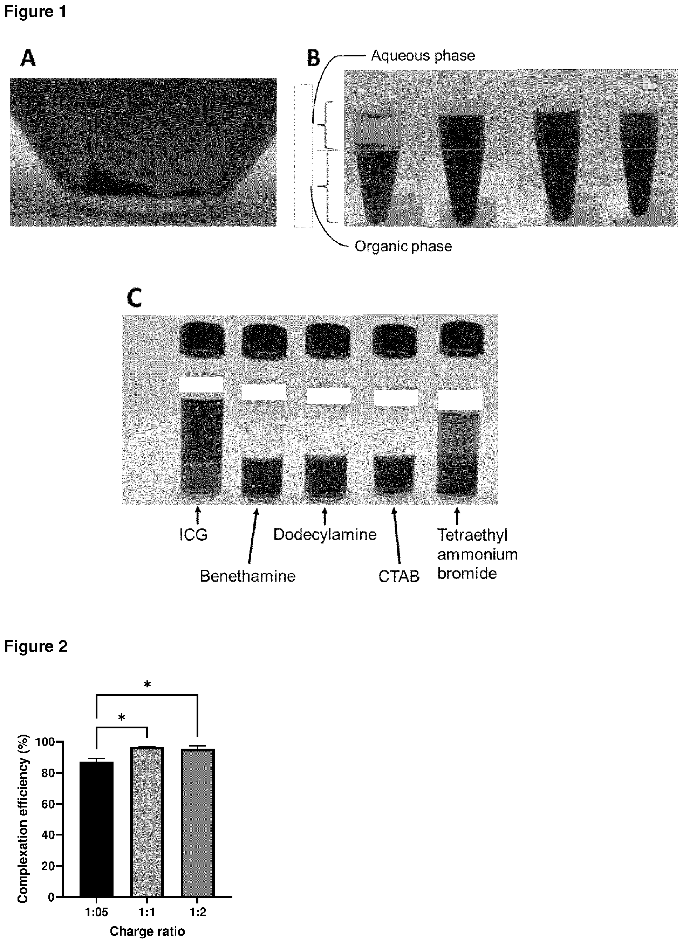

DB36 and ICG are negatively charged molecules, which are very soluble in water, but not in organic solvents such as chloroform. All the used co-ions have a positive charge and they could form hydrophobic ion pairing (HIP) complexes with either DB36 or ICG. When solubilized, DB36 and ICG form dark blue or green solutions, respectively. Thus, successful HIP complexation was assessed by either observing precipitates (for method 1) or a change in coloration in the chloroform phase (for methods 2 and 3). Method 1 was only suitable for water-soluble co-ions such as CTAB, which could form a HIP complex with DB36 visible as a precipitate (Figure 1A). Method 2 could be used for both water-soluble and water-insoluble co-ions. All tested co-ions with this method could form complexes with DB36; the organic phase had a dark blue color indicating DB36 was present (Figure 1B). In the case of some co-ions (e.g., THAB, TOAB), DB36 was also present in the aqueous phase, which also had a dark blue color.

HIP complexes of ICG with different co-ions could be formed using method 3. When using CTAB, benethamine or dodecylamine as co-ions, near 100% of the ICG could be complexed; the methanol:water phase was almost colorless, while only the chloroform phase was green (Figure 1C). In the case of the control vial, a small amount of ICG transferred to the chloroform. Due to the addition of HCl, a portion of ICG likely de-salted, making it more hydrophobic and soluble in the organic phase.

**Figure 1****: Methods for HIP complexation.** (A) Precipitates of DB36:CTAB complexes in water obtained after method 1. (B) Eppendorf tubes from method 2 showing the resulting organic and aqueous phases when complexing DB36 with the co-ions CTAB, THAB, TOAB and TDMAC (from left to right); DB36 as a complex is soluble in the organic phase and turns the solution dark blue. (C) Resulting vials from method 3 when using methanol with no co-ions, benethamine, dodecylamine, CTAB and tetraethyl ammonium bromide (from left to right) to form HIP complexes; a near total transfer of ICG to the chloroform can be observed when using benethamine, dodecylamine or CTAB.

### Conclusion:

Hydrophobic complexes between several cationic co-ions and DB36/ICG could be formed using three different methods. Method 1 is only useful for water-soluble co-ions and the most efficient complexation was obtained when using method 3.

### References

Wibel, R. et al. (2020) 'Hydrophobic ion pairing (HIP) of (poly)peptide drugs: Benefits and drawbacks of different preparation methods', European Journal of Pharmaceutics and Biopharmaceutics, 151, pp. 73-80.
doi:10.1016/j.ejpb.2020.04.004.

### Example 3: Complexation efficiency of ICG-HIP

**Aim:** To investigate the complexation efficiency as a function of charge ratio.

### Materials and methods:

*ICG HIP complexation:* HIP complexes of ICG with benethamine were formed as described in method 3 of example 2. The initial concentration of ICG was 2.5 mg/mL and three different concentrations of benethamine were used accordingly. The ratio of the molar concentrations of ICG:benethamine were 1:1, 1:2 and 1:4, which corresponded to charge ratios of 1:0.5, 1:1 and 1:2, respectively. After HIP complexation, the chloroform phase was isolated and transferred to a new glass vial. Chloroform was dried under a gentle flow of nitrogen. The dried HIP complexes were re-suspended in ethanol and the ICG concentration was quantified by UHPLC. *UHPLC:* Samples were analyzed on a Shimadzu Nexera-X HPLC. Samples were injected (10 µL) onto a Waters Terra XBridge BEH C8 column (2.5 µm, 4.6x75mm, temperature 25 °C) at a flow rate of 1 mL/min. The solvent system consisted of mobile phase A (5% MeCN, 0.1% TFA in water) and mobile phase B (0.1% TFA in MeCN). The gradient was 0% B for 1 min, 0 to 100% B in 5 min, 100% B for 4 min, 100% B to 0% B in 0.5 min, 0% B for 1 min. UV detection at 780 nm was used to measure the AUC of ICG.

### Results and discussion:

The concentration of ICG in the organic phase was determined by UHPLC and the percentage of complexed ICG was calculated. At charge ratios of 1:1 and 1:2 (ICG:benethamine), the HIP complexation efficiency was around 95%. Complexation efficiency dropped to approximately 87% at a charge ratio of 1:0.5 (Fig. 2). At a charge ratio of 1:0.5, there are less molecules of benethamine to form ionic interactions with ICG, thus, less ICG is complexed. Moreover, the complexation efficiency remained constant at a charge ratio of 1:2 despite the excess of benethamine molecules.

**Figure 2****: Complexation efficiency of ICG at different charge ratios.** ICG was complexed with benethamine at charge ratios of 1:0.5, 1:1 and 1:2 (ICG:benethamine). Data is presented as mean ± SEM (n=3); the statistical analysis corresponds to a one-way ANOVA with Tukey multiple comparisons (* p ≤ 0.05).

### Conclusion:

The ICG-HIP complexation efficiency was higher (95%) at charge ratios of 1:1 and 1:2.

### Example 4. Preparation of ICell formulations

**Aim:** To describe the preparation procedure of ICell formulations and solubilization of active pharmaceutical compounds (APIs) or HIPs in these.

### Materials and Methods:

ICell formulations are prepared by mixing carbohydrate esters e.g., SuBen, LacBen, RaBen etc. with solvents and co-solvents e.g. EtOH, GTO, DMSO, PC etc. Compositions are given in weight percent (or weight ratio) and the corresponding amount of compound is weighed into one vial. The mixture is placed in an ultrasonication bath at 70-80°C for 1-2 hours and occasionally vortexed to generate homogenous solutions (ICell) that are subsequently stored at 4 °C until further use. All APIs or HIP complexes are incorporated into the formulation by proportionally adding ICell on top of freeze-dried APIs, HIPs or combinations of freeze-dried APIs or HIPs. ICell is subjected to magnetic stirring at 40-50 °C until the HIP or combination of HIPs is completely dissolved. This final ICell formulation is transferred and stored in sealed vials at room temperature or at 4 °C. Certain formulations may be stored at -20 °C.

### Example 5: Formulation of ICG HIP complexes in ICells.

**Aim:** To show the solubility of different ICG HIP complexes in ICells.

### Materials and methods:

*ICG HIP complexation:* Complexes of ICG with benethamine, dodecylamine, CTAB and tetraethyl ammonium bromide were formed as described in method 3 of example 2 but using 2.5 mg/mL as the initial ICG concentration. For each co-ion, the chloroform phase was isolated and transferred to new glass vials. Chloroform was dried under a gentle flow of nitrogen.

*ICell formulation:* A SuBen:GTO:EtOH (60:25:15) ICell was prepared as described in example 4. 1 g of ICell was added to each vial containing the previously isolated ICG HIP complexes. In addition, 1 g of ICell was added to a vial containing 2.5 mg of native ICG.

### Results and discussion:

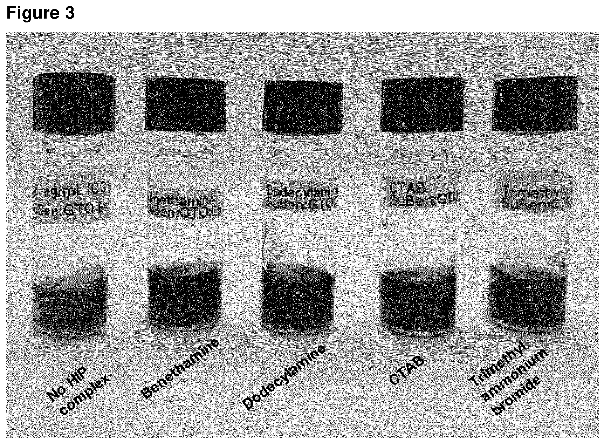

The hydrophobic complexes of ICG with benethamine, dodecylamine, CTAB and tetraethyl ammonium bromide were easily solubilized in ICell. All the formed solutions were green, translucent, and free of particles (Figure 3). The powder of ICG in its native form, as a hydrophilic compound, was not soluble in ICell since the resulting solution was opaque/cloudy and it contained visible particles.

**Figure 3****: Native ICG (no HIP complex) and ICG complexed with benethamine, dodecylamine**, CTAB and trimethyl ammonium bromide formulated in SuBen:GTO:EtOH (60:25:15) ICells. ICG HIP complexes in ICell formed green, translucent and particle free solutions.

### Conclusion:

The solubility of ICG in ICell was successfully improved by forming hydrophobic complexes with all the tested co-ions.

### Example 6: Stability of ICG HIP complexes in ICell

**Aim:** To investigate the stability of ICG complexed with different co-ions when formulated in ICell.

### Materials and methods:

*ICG HIP complexation:* Complexes of ICG with benethamine, dodecylamine, CTAB and tetraethyl ammonium bromide were formed as described in method 3 of example 2. For each co-ion, the chloroform phase was isolated and transferred to new glass vials. Chloroform was dried under a gentle flow of nitrogen.

*ICell formulation:* A SuBen:GTO:EtOH (60:25:15) ICell was prepared as described in example 4. 1 g of ICell was added to each vial containing the previously isolated ICG HIP complexes. In addition, 1 g of ICell was added to a vial containing 2.5 mg of native ICG.

*Stability study:* After preparation, ICells were stored at room temperature and protected from light. After 7 weeks, approximately 25 µL of each formulation were taken and dissolved in 500 µL of acetonitrile. The samples were then analyzed by HPLC. *HPLC analysis:* Samples were analyzed on a Shimadzu Nexera-X HPLC. Samples were injected (5 µL) onto a Waters Terra XBridge BEH C18 column (2.5 µm, 4.6x75mm, temperature 25 °C) at a flow rate of 1 mL/min. The solvent system consisted of mobile phase A (5% MeCN, 0.1% TFA in water) and mobile phase B (0.1% TFA in MeCN). The gradient was 0% B for 1 min, 0 to 100% B in 5 min, 100% B for 4 min, 100% B to 0% B in 0.5 min, 0% B for 1 min. UV detection at 280 nm was used to measure the AUC of ICG and its related degradation products.

### Results and discussion:

The total AUC of ICG plus its related degradation products at 280 nm was measured. The stability of ICG was evaluated by calculating the percentage AUC corresponding to ICG. After 7 weeks at 25 °C in the dark, more than 95% of intact ICG was present in all the ICell formulations regardless of the co-ion used for complexation (Table 3). It is expected that stability can be further improved by storing the formulations at lower temperatures.

**Table 3: Stability of ICG complexed with different co-ions in a SuBen:GTO:EtOH (60:25:15) ICell after 7 weeks in the dark at room temperature. Stability is reported as the percentage AUC corresponding to ICG at 280 nm.**

| **Co-ion complex** | **ICG % AUC** |
|---|---|
| Benethamine | 96.9 |
| CTAB | 97.6 |
| Dodecylamine | 95.3 |
| Tetraethyl ammonium bromide | 96.3 |

### Conclusion:

ICG HIP complexes showed good stability after storage in the dark for 7 weeks at room temperature.

### Example 7: Stability of ICG HIP complexes in different solvents

**Aim:** To investigate the stability of ICG HIP complexes (using benethamine as model co-ion) in different solvents.

### Materials and methods:

*ICG HIP complexation:* Complexes of ICG with benethamine were formed as described in method 3 of example 2, but using 2.5 mg/mL as the initial ICG concentration. For each co-ion, the chloroform phase was isolated and aliquots were prepared in new glass vials. Chloroform was dried under a gentle flow of nitrogen.

*Stability study:* 1 mL of acetone, ethanol, propylene carbonate (PC) or benzyl alcohol were added to individual vials containing isolated ICG HIP complexes. The resulting solutions were divided by half in Eppendorf tubes, which were incubated at either 25 °C or 37 °C and protected from light. Native ICG dissolved in water and stored at 25°C in the dark was included as a control. At selected time points, 50 µL of sample were taken and analyzed by HPLC.

*HPLC analysis:* Samples were analyzed on a Shimadzu Nexera-X HPLC. Samples were injected (10 µL) onto a Waters Terra XBridge BEH C18 column (2.5 µm, 4.6x75mm, temperature 25 °C) at a flow rate of 1 mL/min. The solvent system consisted of mobile phase A (5% MeCN, 0.1% TFA in water) and mobile phase B (0.1% TFA in MeCN). The gradient was 0% B for 1 min, 0 to 100% B in 5 min, 100% B for 4 min, 100% B to 0% B in 0.5 min, 0% B for 1 min. UV detection at 280 nm was used to measure the AUC of ICG and its related degradation products.

### Results and discussion:

The ICG:benethamine complex was very soluble in ethanol, PC and benzyl alcohol, but it had limited solubility in acetone. The stability of the resulting solutions was evaluated by looking for new peaks in the chromatograms (which might indicate the presence of new molecular entities) and monitoring the AUC of ICG, which should remain constant.

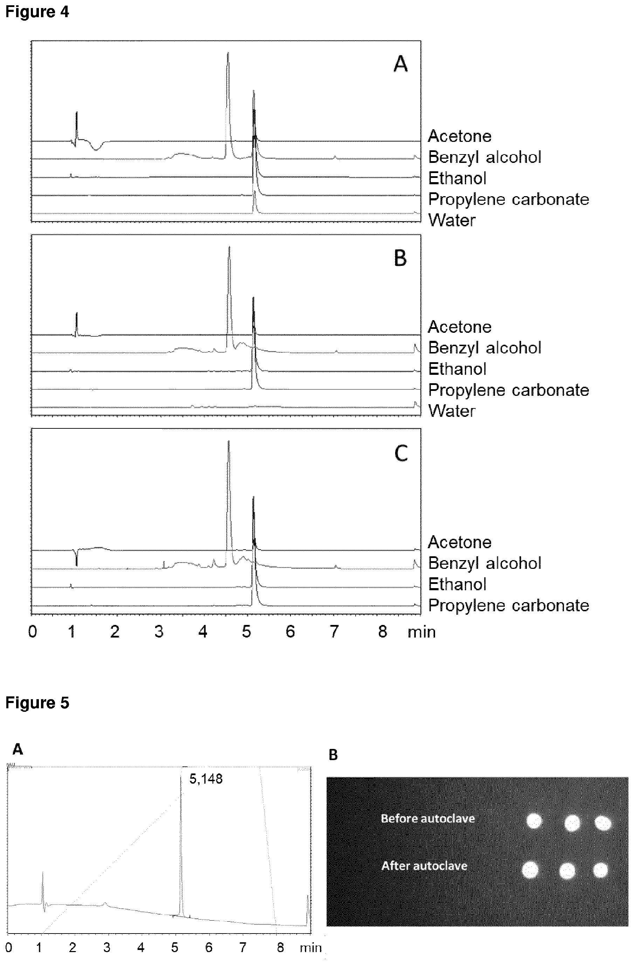

When complexed with benethamine, ICG showed good stability in ethanol, PC and acetone since no degradation products were detected and the AUC of ICG remained constant after 5 days at both 25 and 37°C (Figure 4). In contrast, regardless of the temperature, the ICG HIP complex showed lower stability in benzyl alcohol; after 5 days, the ICG peak could not be easily identified and several other peaks were visible in the chromatogram (very likely corresponding to ICG degradation products). Furthermore, native non-complexed ICG in water showed the worst stability as ICG was no longer visible in the chromatogram after 5 days at 25°C.

**Figure 4****: Comparison of individual chromatograms of the ICG:benethamine complex dissolved in acetone, benzyl alcohol, ethanol and propylene carbonate at time zero (A) and after 5 days of incubation in the dark at 25°C (B) and 37°C (C).** Native ICG dissolved in water was included as a control for incubation at 25°C. ICG had a retention time of 5.2 min; the peak observed at 4.5 min in the benzyl alcohol samples correspond to the used solvent. The detection wavelength was 280 nm.

### Conclusion:

The ICG:benethamine complex showed great stability in ethanol, PC and acetone at 25 and 37°C during the tested period. The stability of complexed ICG was better than the native form.

### Example 8: Stability of an ICG HIP complex after autoclaving

**Aim:** To investigate the effect of autoclaving on an ICG-benethamine complex formulated in ICell.

### Materials and methods:

*ICG HIP complexation:* A complex of ICG with benethamine was formed as described in method 3 of example 2, but using 2.5 mg/mL ICG as the initial concentration. For each co-ion, the chloroform phase was isolated and transferred to new glass vials. Chloroform was dried under a gentle flow of nitrogen.

*ICell formulation:* A SuBen:CLA-8:GTH:DMSO (50:10:20:20) ICell was prepared as described in example 4. 1 g of ICell was added to the vial containing the previously isolated ICG HIP complex. Around 10 µL of ICell was taken and dissolved in acetonitrile. Then the sample was analyzed by HPLC and the concentration of ICG in the ICell was determined. A ICell formulation containing 1% (1 mg/g) ICG was prepared by proportionally adding empty ICell to reach the desired concentration.

*Stability study:* Around 150 µL of ICell were placed in a glass ampoule, which was then sealed. The ampoule was subjected to autoclaving. Before and after the autoclave, a 10-µL sample was taken, dissolved in acetonitrile and then analyzed by HPLC to evaluate the stability of ICG. ICells were also imaged using an in-house assembled NIR imaging lamp and camera, which were connected to an HP Windows computer (no special software needed).

*HPLC:* Samples were analyzed on a Shimadzu Nexera-X HPLC. Samples were injected (10 µL) onto a Waters Terra XBridge BEH C8 column (2.5 µm, 4.6x75mm, temperature 25 °C) at a flow rate of 1 mL/min. The solvent system consisted of mobile phase A (5% MeCN, 0.1% TFA in water) and mobile phase B (0.1% TFA in MeCN).

The gradient was 0% B for 1 min, 0 to 100% B in 5 min, 100% B for 4 min, 100% B to 0% B in 0.5 min, 0% B for 1 min. UV detection at 785 nm was used to measure the AUC of ICG.

### Results and discussion:

The HPLC chromatogram after autoclaving did not show signs of ICG chemical degradation. Degradation products were not detected and ICG retained its integrity, shown by the single peak observed at 5.1 min at 785 nm (Figure 5A). In addition, the signal from the ICells before and after autoclaving was considered equal when imaged with a NIR camera (ex 750-820nm / em >840nm) .

**Figure 5****:** (A) HPLC chromatogram (785 nm) of the ICell containing 1% ICG complexed with benethamine after autoclaving; only ICG is visible with a retention time of 5.1 min. (B) **Conclusion:**

ICG complexed with benethamine was chemically stable after autoclaving and its visibility using a NIR imaging system was not affected.

### Example 9: Fluorescence of ICG HIP complexes.

**Aim:** To investigate the effect of HIP complexation with several co-ions on the fluorescence of ICG.

### Materials and methods:

*ICG HIP complexation:* Complexes of ICG with benethamine (BENA), dodecylamine (DDA), cetyl trimethyl ammonium bromide (CTAB), and tetraoctyl ammonium bromide (TOAB) were formed as described in method 3 of example 2. For each co-ion, the chloroform phase was isolated and transferred to new glass vials. Chloroform was dried under a gentle flow of nitrogen. The dried complexes were re-dissolved in 2 mL of ethanol. The solutions were analyzed by HPLC to determine the ICG concentration. *HPLC analysis:* Samples were analyzed on a Shimadzu Nexera-X HPLC. Samples were injected (10 µL) onto a Waters Terra XBridge BEH C8 column (2.5 µm, 4.6x75mm, temperature 25 °C) at a flow rate of 1 mL/min. The solvent system consisted of mobile phase A (5% MeCN, 0.1% TFA in water) and mobile phase B (0.1% TFA in MeCN). The gradient was 0% B for 1 min, 0 to 100% B in 5 min, 100% B for 4 min, 100% B to 0% B in 0.5 min, 0% B for 1 min. UV detection at 785 nm was used to measure the AUC of ICG.

*Fluorescence spectroscopy:* Solutions of the different ICG-HIP complexes were prepared in ethanol in a concentration range of 0.0006 - 1 mg/mL. Solutions of native ICG in ethanol were included as control. 100 µL of each sample and PBS (in duplicate) were placed in a 96-well black NUNC plate covered with a lid. Fluorescence emission and excitation spectra were recorded using a TECAN Spark multimode microplate reader with the following settings: excitation wavelength=790nm (for emission scan), emission wavelength=822 nm (for excitation scan), top reading, bandwidth=5 nm.

### Results and discussion:

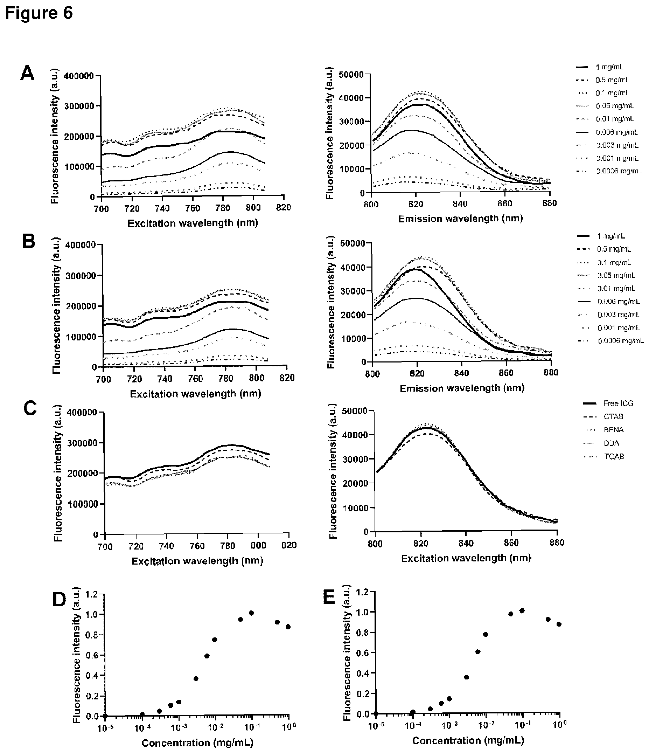

The fluorescence intensity of ICG, both as a free molecule or in a HIP complex, gradually increased and reached a maximum emission intensity at a concentration of 0.1 mg/mL. The excitation maxima was 785 nm, whereas the emission maxima was 822 nm. Above 0.1 mg/mL, the fluorescence intensity gradually decreased due to ICG self-quenching (Fig. 6A,B). Moreover, free ICG and the different ICG-HIP complexes displayed the same excitation and emission spectra at the tested concentrations as displayed in Figure 6C. There was no apparent effect of HIP complexation in the fluorescence intensity of ICG as a function of concentration as exemplified by the comparison between free ICG and ICG-BENA in Fig. 6D and Fig. 6E, respectively.

**Figure 6****: Fluorescence of ICG in its native form and in HIP complexes.** (A-B) Fluorescence excitation and emission spectra of (A) free ICG and (B) ICG-BENA at varying concentrations. (C) Fluorescence excitation and emission spectra of ICG as a free molecule and in a HIP complex with various co-ions at a concentration of 0.1 mg/mL. (D-E) Fluorescence intensity of (D) free ICG and (E) ICG-BENA as a function of concentration. All solutions had ethanol as solvent

### Conclusion:

ICG in a HIP complex showed the same fluorescence excitation and emission properties as ICG in its free form.

### Example 10: Fluorescence of ICG-HIP in ICell

**Aim:** To evaluate the fluorescence properties of ICG-HIP when formulated in ICell.

### Materials and methods:

*ICG HIP complexation:* ICG complexed with benethamine was formed as described in method 3 of example 2, but using 2.5 mg/mL ICG as the initial concentration. To isolate the ICG-HIP complexes, the chloroform phase was isolated and transferred to new glass vials. Chloroform was dried under a gentle flow of nitrogen.

*ICell formulation:* A SuBen:GTO:EtOH (60:25:15) ICell was prepared as described in example 4. ICell was added to the vial containing the previously isolated ICG HIP complex to obtain a concentration of 1 mg/g. Serial dilutions were done to prepare ICells in the ICG concentration range of 0.0001 - 1 mg/g. Approximately 75 µL of each ICell (in duplicate) were placed in a 96-well black NUNC plate and fluorescence was measured. ICells were also imaged using an in-house assembled NIR imaging lamp and camera, which were connected to an HP Windows computer (no special software needed).

*Fluorescence measurement:* Fluorescence emission and excitation spectra were recorded using a TECAN Spark multimode microplate reader with the following settings: excitation wavelength=790nm (for emission scan), emission wavelength=822 nm (for excitation scan), top reading, bandwidth= 7.5nm.

### Results and discussion:

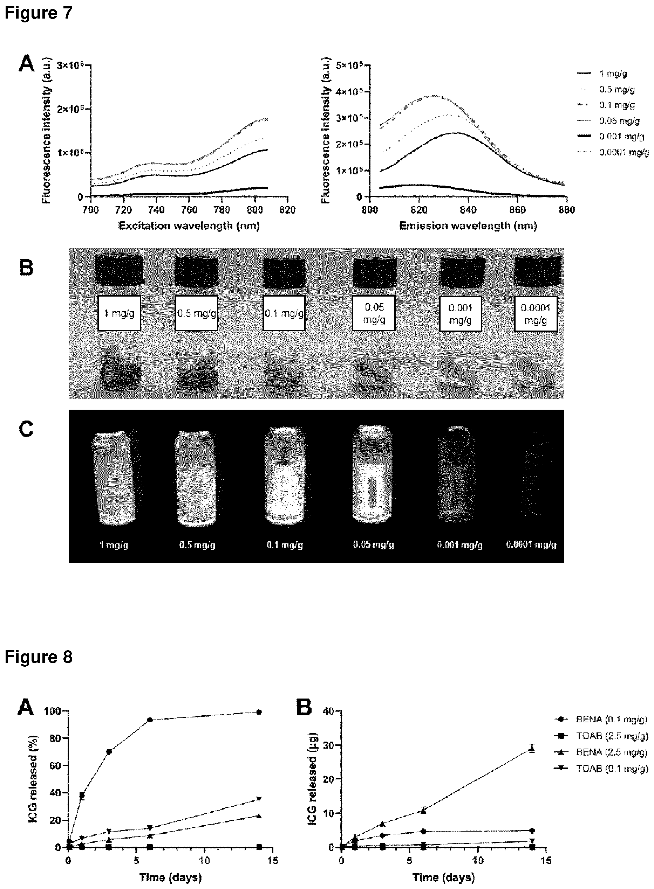

Self-quenching of ICG occurred at the concentrations above 0.1 mg/g. The highest fluorescence intensity was obtained at 0.05 and 0.1 mg/g ICG-HIP (Fig. 7A). At these concentrations, the emission maxima was obtained at 825 nm. Accordingly, the ICells containing 0.05 and 0.1 mg/g ICG-HIP displayed the best visibility through NIR imaging. A reduced fluorescence intensity was observed in the 0.5 and 0.1 mg/g ICG-BENA formulations, whereas the lowest concentrations showed a very low or non-existent signal (Fig. 7B, C).

**Figure 7****: Fluorescence of ICG-HIP in a ICell.** (A) Fluorescence and emission spectra of ICG-HIP in a ICell at varying concentrations. (B) Color and (C) NIR fluorescence images of ICG-HIP at various concentrations in the ICell when contained in glass vials.

### Conclusion:

ICG retained its fluorescence properties in the ICell and the highest fluorescence intensity of ICG-HIP was obtained at an ICG concentration of 0.05 and 0.1 mg/g.

### Example 11: In vitro release of ICG-HIP complexes in ICell.

**Aim:** To investigate the in-vitro release profile of ICG when complexed with different co-ions.

### Materials and methods:

*ICG HIP complexation:* Complexes of ICG with benethamine (BENA) and TOAB were formed as described in method 3 of example 2, where the initial concentration of ICG was 2.5 mg/mL, unless otherwise stated. For each co-ion, the chloroform phase was isolated and transferred to new glass vials. Chloroform was dried under a gentle flow of nitrogen.

*ICell formulation:* Four different ICells were prepared as described in example 4. The compositions of the ICells were LOIB:GTO:EtOH (82.5:7.5:10), SuBen:GTH:DMSO (60:20:20), SuBen:GTO:EtOH (60:25:15) and SuBen:Et-Pal:EtOH (62.5:22.5:15). ICell was added to each vial containing the previously isolated ICG HIP complexes to obtain a final ICG concentration of either 0.1 or 2.5 mg/g.

*In vitro initial release:* 50 µL of ICells containing either ICG complexed with BENA or TOAB were injected in glass vials containing 2 mL PBS and they were incubated at 37°C protected from light. After 2.5 h, the release media (PBS) was retrieved and the concentration of ICG was measured by UHPLC. The ICG concentration in all formulations was 0.1 mg/g.

*In vitro long-term release (14 days):* ICG was complexed with BENA or TOAB and then incorporated into a SuBen:GTH:DMSO (60:20:20) ICell to obtain an ICG final concentration of 0.1 or 2.5 mg/g. The ICG HIP complexes were prepared using either an ICG initial concentration of 2.5 or 5 mg/mL. The tested formulations are shown in Table 4. 50 µL of each ICells were injected in glass vials containing 2 mL PBS and they were incubated at 37°C protected from light. At specified time points, PBS was carefully removed from the vials and the ICell depot was dissolved in DMSO and vortexed until dissolution. As reference, 10 µL of each ICell (non-injected) were dissolved in DMSO (n=4). All samples were analyzed by UHPLC. The ICG release percentage was determined by ratiometric analysis. The AUC values of ICG (780 nm) and SuBen (280 nm) were measured to calculate the AUC ratio of ICG/SuBen in all samples. The percentage of released ICG was calculated by comparing the AUC ratios in the collected ICells versus the corresponding ratios in the reference (non-injected) ICell.

**Table 4. Experiment design to investigate the in vitro long-term release kinetics of ICG-HIP complexes. ICG was complexed with either BENA or TOAB. All HIP complexes were formulated in SuBen:GTH:DMSO (60:20:20) ICells.**

| **ICG conc. for complexation** | **Co-ion** | **ICG conc. in ICell** |
|---|---|---|
| 5 mg/mL | BENA | 0.1 mg/mL |
| 5 mg/mL | TOAB | 2.5 mg/mL |
| 2.5 mg/mL | BENA | 2.5 mg/mL |
| 2.5 mg/mL | TOAB | 0.1 mg/mL |

*UHPLC analysis:* Samples were analyzed on a Shimadzu Nexera-X HPLC. Samples were injected (5 µL) onto a Waters Terra XBridge BEH C8 column (2.5 µm, 4.6x75mm, temperature 25 °C) at a flow rate of 1 mL/min. The solvent system consisted of mobile phase A (5% MeCN, 0.1% TFA in water) and mobile phase B (0.1% TFA in MeCN). The gradient was 0% B for 1 min, 0 to 100% B in 5 min, 100% B for 4 min, 100% B to 0% B in 0.5 min, 0% B for 1 min. UV detection at 780 nm was used to measure the AUC of ICG.

### Results and discussion:

It was observed that the initial release of ICG was influenced by both the ICell formulation and the co-ion used for the HIP complexation (Table 5). ICG was not detected in the release media from neither SuBen:Et-Pal:EtOH (62.5:22.5:15) ICells. Additionally, it appeared that slightly more ICG was initially released when using BENA as co-ion than TOAB. Moreover, ICG complexed with BENA could not be fully solubilized in the LOIB:GTO:EtOH (82.5:7.5:10) ICell and, in contrast, ICG-TOAB was easily dissolved in uch ICell.

**Table 5. In vitro initial release of ICG-HIP complexes. Percentage of ICG released 2.5 h post-injection in PBS of ICG-BENA and ICG-TOAB from different ICell formulations. Data is presented as mean ± SD (n=3). If ICG was not detected in the release media, this was reported as 0.0. The concentration of ICG in the ICells was 0.1 mg/g.**

| **ICell composition (w/w%)** | **ICG released (%)** | |
|---|---|---|
| | *BENA* | *TOAB* |
| LOIB:GTO:EtOH (82.5:7.5:10) | - | 10.4 ± 0.4 |
| SuBen:GTH:DMSO (60:20:20) | 5.9 ± 0.6 | 3.0 ± 1.0 |
| SuBen:GTO:EtOH (60:25:15) | 3.3 ± 0.5 | 2.7 ± 0.3 |
| SuBen:Et-Pal:EtOH (62.5:22.5:15) | 0.0 | 0.0 |

To investigate the effect of the co-ions and the concentration of ICG-HIP in the ICell on the ICG release profile over 14 days, ICG was complexed with BENA or TOAB and then incorporated into a SuBen:GTH:DMSO (60:20:20) ICell with a final ICG concentration of 0.1 or 2.5 mg/g. It was observed that both the co-ion and the ICG-HIP concentration influenced the release kinetics of ICG (Fig. 8). Overall, more ICG was released when complexed with BENA rather than with TOAB. Additionally, it was found that the release of ICG was reduced for both types of co-ions when increasing the concentration of ICG-HIP in the ICell. For instance, the percentage of ICG released from ICells with ICG-BENA decreased by around 4-fold when increasing the ICG concentration from 0.1 to 2.5 mg/g. At 2.5 mg/g, the release of ICG was completely abolished from ICells containing ICG-TOAB. Moreover, the chromatograms did not show signs of ICG degradation in the injected depots, suggesting that the ICell protects ICG and enhances its stability. In addition, there was no apparent effect caused by the different initial concentrations of ICG used during the complexation method.

**Figure 8****. In vitro release of ICG when complexed with BENA or TOAB.** (A) Percentage and (B) total amount of ICG released from SuBen:GTH:DMSO (60:20:20) ICells after injection in PBS and incubation at 37 °C. ICG was complexed with BENA or TOAB and formulated in the ICells at a final concentration of 0.1 or 2.5 mg/g.

### Conclusion:

The in vitro release profile of ICG from ICell could be modified depending on the ICell formulation, the co-ion used for HIP complexation and the final ICG-HIP concentration in the ICell.

### Example 12: Correlation between co-ions and ICell surface tension

**Aim:** To investigate the effect of different co-ions on the surface tension of ICell.

### Materials and methods:

*ICell formulation:* A SuBen:GTH:DMSO (60:20:20), SuBen:GTH:DMSO (60:20:15) and a SuBen:GTO:EtOH (60:25:10) ICell were prepared as described in example 4. *Effect on surface tension without ICG:* TOAB was dissolved in DMSO and EtOH to produce solutions with a concentration of 40 nM and 200 nM. CTAB was dissolved in EtOH to produce solutions with a concentration of 40 nM and 200 nM. 0.03 g of each co-ion solution were individually added to 0.57 g of either a SuBen:GTH:DMSO (60:20:15) or a SuBen:GTO:EtOH (60:25:10) ICell. Thus, the final co-ion concentration in each ICell was 2mM or 10 mM. 50 µL of the previously prepared ICells were injected in glass vials containing 2 mL PBS. The vials were incubated at 37°C during 7 days and the visual appearance of the injected depots was evaluated.

*ICG HIP complexation:* Complexes of ICG with benethamine (BENA) and TOAB were formed as described in method 3 of example 2, where the initial concentration of ICG was 2.5 mg/mL or 5 mg/mL as described in Table 4. For each co-ion, the chloroform phase was isolated and transferred to new glass vials. Chloroform was dried under a gentle flow of nitrogen.

*Effect on surface tension with ICG:* SuBen:GTH:DMSO (60:20:20) ICell was proportionally added to the previously isolated ICG HIP complexes to obtain formulations with a final ICG concentration of either 0.1 or 2.5 mg/g as described in Table 4. 50 µL of these ICells were injected in glass vials containing 2 mL PBS. The vials were incubated at 37°C during 3 days and the visual appearance of the injected depots was evaluated.

### Results and discussion:

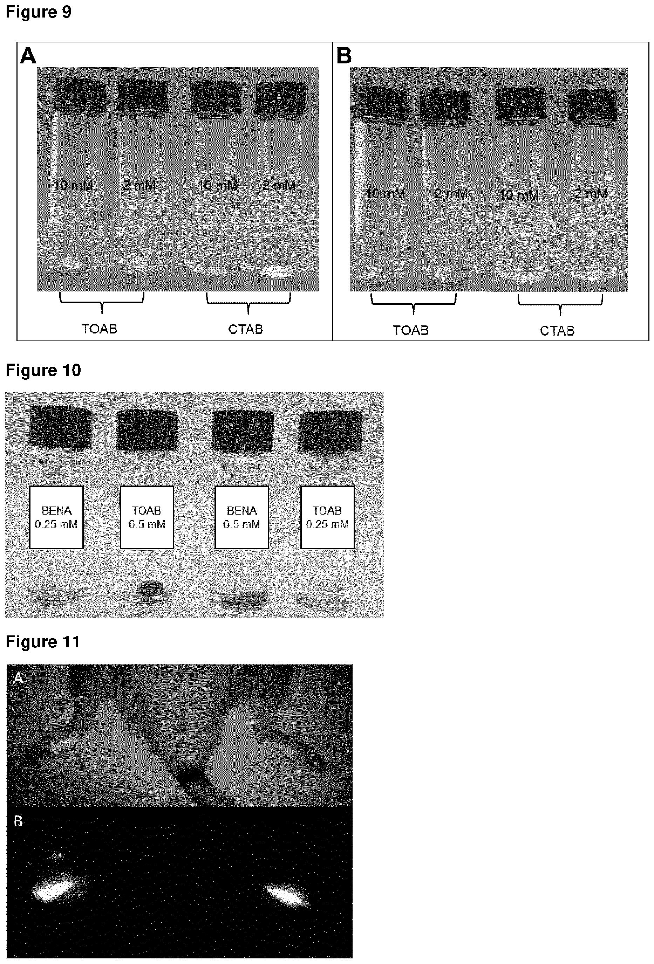

The co-ions CTAB and TOAB were incorporated into ICells to evaluate their effect on the surface tension of the formulations after injection. After 7 days at 37°C, it was observed that the ICells depots containing TOAB had retained their spherical shape, while the ones containing CTAB had flatten (Fig. 9). The flattening of the ICells was more pronounced when the CTAB concentration was 10 mM compared to 2 mM. Regarding the molecular structure of the co-ions, CTAB presents a linear structure, while TOAB is a branched and bulkier molecule. Thus, the molecular structure of CTAB facilitates its arrangement in the surface of the injected ICell, where the hydrophilic group interacts with the surrounding water and the hydrophobic domain interacts with the ICell. Such arrangement diminishes the surface tension of the ICell depot and causes its flattening.

**Figure 9****: Effect of different co-ions on the surface tension of ICell.** Injected depots of (A) SuBen:GTH:DMSO (60:20:20) and (B) SuBen:GTO:EtOH (60:25:15) ICells containing 2 mM and 10 mM of either TOAB or CTAB. The injected ICells had been incubated at 37°C for 7 days.

Next, ICG was complexed with either BENA or TOAB and formulated in a SuBen:GTH:DMSO ICell (60:20:20). The ICells had a final ICG concentration of 0.1 mg/g and 2.5 mg/g, which corresponded to a co-ion concentration of 0.25 mM and 6.5 mM, respectively. As expected, all ICell depots containing TOAB retained its shape after injection (Fig. 10). Regarding the formulations containing BENA, flattening of the depots was observed at a co-ion concentration of 6.5 mM, but not at 0.25 mM. The size/bulkiness of BENA is lower than that of TOAB, which again promotes the arrangement of BENA in the surface of the ICell depot to reduce the surface tension. Moreover, at a low concentration, the small amount of BENA molecules is not enough to visibly affect the surface tension of the ICell depot.

**Figure 10****: Effect of co-ion concentration on the surface tension of ICell.** Injected depots of SuBen:GTH:DMSO (60:20:20) containing 0.1 mg/g ICG-BENA, 2.5 mg/g ICG-TOAB, 2.5 mg/g ICG-BENA and 0.1 mg/g ICG-TOAB (from left to right). The corresponding co-ion concentration in the ICell is indicated on each vial. The injected ICells had been incubated at 37°C for 3 days.

### Conclusion:

The surface tension of the ICell was lowered by co-ions with a linear structure and by increasing their concentration.

### Example 13: In vivo evaluation of ICG-HIP complexes using fluorescence imaging

**Aim:** To demonstrate in vivo fluorescence properties of ICG-HIP complexes at the injection site and in regional draining lymph nodes after ICG release from the injection site depot material.

### Materials and methods:

*ICG HIP complexation:* A complex of ICG with benethamine was formed as described in method 3 of example 2, but using 2.5 mg/mL ICG as the initial concentration. For each co-ion, the chloroform phase was isolated and transferred to new glass vials. Chloroform was dried under a gentle flow of nitrogen.

*ICell formulation:* Two ICell formulations were prepared. The first one consisted of SuBen:CLA-8:GTH:DMSO (50:10:20:20) and the ICell was prepared as described in example 4. 1 g of ICell was added to the vial containing the previously isolated ICG HIP complex. Around 10 µL of ICell was taken and dissolved in acetonitrile. Then the sample was analyzed by HPLC and the concentration of ICG in the ICell was determined. The final concentration of ICG in the ICell was 1 mg/g, which was obtained by proportionally adding empty ICell until reaching the desired concentration. The second ICell formulation consisted of SuBen:Et-Pal:EtOH (62.5:22.5:15), which was prepared as described in example 4. ICell was added to a previously isolated ICG HIP complex to obtain a final concentration of 0.1 mg/g ICG.

*In-vivo evaluation:* 50 µL of SuBen:CLA-8:GTH:DMSO (50:10:20:20) ICell containing ICG-HIP complexes were injected sub cutaneous on the plantar aspect (midway between the calcaneus and proximal toe joint) of the hindlimbs of a rat (450 g). Injected regions were images immediately and 1 hour after injection. Next, 30 µL of SuBen:Et-Pal:EtOH (62.5:22.5:15) ICell containing ICG-HIP complexes were injected subcutaneously on the plantar aspect of the right hind limb of a separate rat, which also received a 100-µL injection deep in the thigh. Injected regions were images immediately and 20 hour after injection. In all cases, the ICell ICG-HIP was injected using a 23 Ga needle and 1 ml syringe.

Injected depots and regions were imaged using an in-house assembled NIR imaging lamp and camera. Images were acquired and processed using Quick Time Viewer on a Macintosh computer. Excitation was performed at 750-820 nm using a LED lamp, emission was recorded using a NIR camera and a 840nm LP emission filter. To evaluate the accumulation of fluorophore in the regional lymph node after release from the injected depot, the popliteal lymph node was carefully dissected, and imaging performed at multiple tissue depths during the procedure.

### Results and discussion:

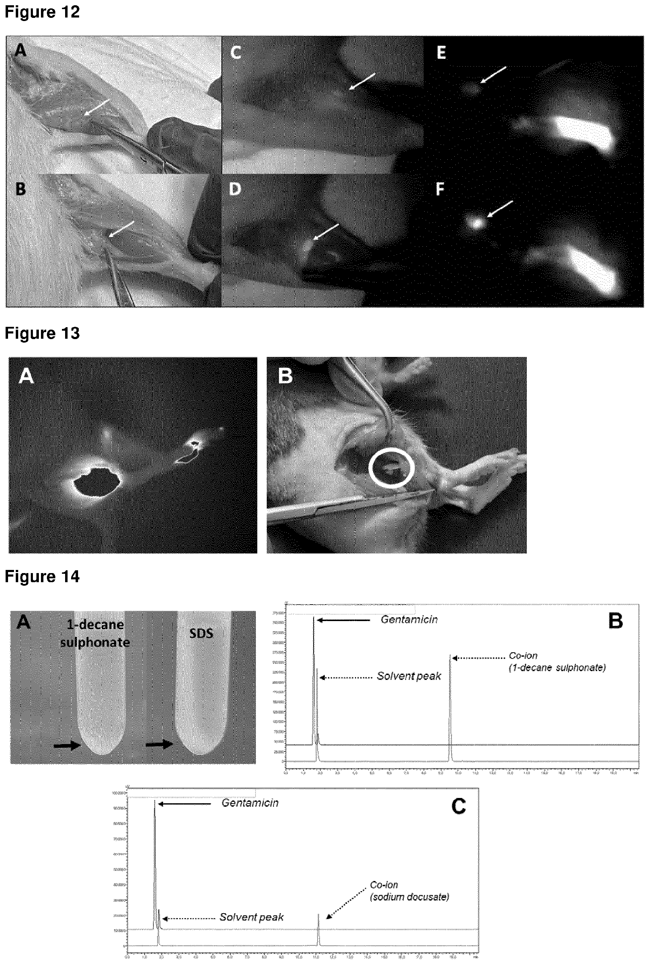

Injected depots of SuBen:CLA-8:GTH:DMSO (50:10:20:20) ICell were clearly visible immediately after injection (Fig. 11). The depots could be visualized both using the X-Nite850 filter and 840nm longpass filter. The X-Nite850nm filter provided additional black and white visual guidance (Figure 11A) whereas the 840nm longpass filter improved signal from the injected depots (Figure 11B). After one hour the popliteal lymph node displayed NIR emission both through the thigh musculature (Figure 12A, C, E, white arrow) and after retracting the overlaying musculature (Figure 12B, D, F). In the case of SuBen:Et-Pal:EtOH (62.5:22.5:15) ICell, it was found that ICG was retained in the injected depot. ICG was not visualized in the local draining lymph nodes 20 h post-injection, not even after retracting the overlaying muscles. Instead, a high fluorescence intensity was only observed at the sites were the ICell was injected (Fig. 13A). In addition, it was observed that the ICell depot had solidified and was easily palpable (Fig. 13B).

**Figure 11****. ICell ICG-HIP depots injected in the right and left hind limb of a rat.** Images of SuBen:CLA-8:GTH:DMSO (50:10:20:20) ICell depots containing ICG-HIP, where image A was acquired using a X-Nite850 emission filter and image B imaged using an 840nm longpass filter.

**Figure 12****. Visualization of fluorophore accumulation in the local draining lymph node.** Imaging of ICG in the local draining lymph node after skin incision (A) and after retracting overlaying muscle (B). NIR images after skin incision (C and E) and after retraction of overlaying muscle (D and F). White arrows indicate the position of the popliteal lymph node, image C and D acquired using a X-Nite850 emission filter, images E and F using a 840nm longpass filter.

**Figure 13****. Visualization of ICell depots retaining ICG-HIP.** (A) NIR fluorescence image (acquired using a X-Nite850 emission filter) of the sites where SuBen:Et-Pal:EtOH (62.5:22.5:15) ICell containing ICG-HIP was injected (right thigh and left hind limb) taken 20 h post-injection; ICG was retained at the injection site and was not visible in the local draining lymph nodes. (D) Picture of the solidified depot of SuBen:Et-Pal:EtOH (62.5:22.5:15) ICell containing ICG-HIP, shown inside the circle.

### Conclusions:

The injected ICell ICG-HIP depot can be easily identified using a NIR imaging system. ICG-HIP can be formulated in the ICells to either release ICG or retain ICG after injection. The release of ICG from the ICell depot provides a clear signal from the regional draining lymph node when imaged one hour after injection. ICG retained in the ICell depot provides a clear signal when imaged 20 h post-injection.

### Example 14: HIP complexation of gentamicin

**Aim:** To form a HIP complex of gentamicin with different co-ions (SDS, 1-decane sulphonate, and sodium docusate).

### Materials and methods:

*HIP complexation via organic-solvent free method:* Gentamicin sulphate was complexed with SDS and sodium 1-decanesulfonate following method 1 described in example 2. Gentamicin (10 mg/mL) was dissolved in 0.01 M HCl. SDS and 1-decane sulphonate were dissolved in 0.01 M HCI in a 5:1 molar ratio (co-ion:gentamicin), which equals a 1:1 charge ratio. The gentamicin and each co-ion solution were mixed in equal volumes, followed by shaking and centrifugation steps as described in method 1 of example 2. A white precipitate was obtained, which corresponded to the HIP complex. The supernatant was removed and its gentamicin content was evaluated by HPLC. After removing the supernatant, the complex was dissolved in methanol, transferred to a new glass vial and the solvent was dried under a gentle nitrogen flow.

*HIP complexation via Bligh-Dyer method:* Gentamicin sulphate was complexed with sodium docusate following method 2 described in example 2. Gentamicin (5 mg/mL) was dissolved in 0.01 M HCl, whereas sodium docusate was dissolved in methanol in a 7.5:1 molar ratio (co-ion:gentamicin) to achieve a 1:1 charge ratio. A sample of the aqueous phase was taken to evaluate its gentamicin content by HPLC.

*HPLC method*: Samples were analyzed on a Shimadzu Nexera-i with an evaporative light scattering detector (ELSD). Samples were injected (5 µL) onto a Waters Terra XBridge BEH C8 column (5 µm, 4.6x150mm, temperature 25 °C) at a flow rate of 1 mL/min. The solvent system consisted of mobile phase A (5% MeCN, 0.1% TFA in water) and mobile phase B (0.1% TFA in MeCN). The gradient was 1% B for 4 min, 1 to 100% B in 10 min, 100% B for 3 min, 100% B to 0% B in 0.5 min, 0% B for 2.5 min. Gentamicin was detected by ELSD.

### Results and discussion:

Regarding HIP complexation via the organic-solvent free method, a cloudy solution was immediately formed after mixing the gentamicin and co-ion solutions. Subsequently, a white precipitate was obtained following the centrifugation step, indicating the formation of a HIP complex (Fig. 14A). Such precipitate was freely soluble in methanol. Additionally, gentamicin was not detected in none of the supernatants as exemplified in Fig. 14B, which indicates that 100% of gentamicin was complexed with the corresponding co-ion. Regarding HIP complexation via the Bligh-Dyer method, gentamycin was not detected in the aqueous phase, which indicated that gentamicin is present in the organic phase, thus 100% gentamicin was complexed with sodium docusate (Fig. 14C). In both methods, even though gentamicin was not detected in the samples, co-ions were detected. Gentamicin presents five positive charges and it is possible that not all of them need to be interacting with a co-ion to form a HIP complex.

**Figure 14****. HIP complexation of gentamicin via organic solvent-free method and Bligh-Dyer method.** (A) Precipitates (pointed by an arrow) resulting from the organic solvent-free method indicating the formation of HIP complexes of gentamicin with 1-decane sulphonate and SDS. (B) Comparison between the chromatogram of a gentamicin standard (top line) and the supernatant from the complexation of gentamicin with 1-decane sulphonate (bottom line). (C) Comparison between the chromatogram of a gentamicin standard (top line) and the aqueous phase from the complexation of gentamicin with sodium docusate (bottom line).

### Conclusion:

HIP complexes of gentamicin with SDS and 1-decane sulphonate were successfully formed via the organic-solvent free method, whereas a HIP complex of gentamicin with sodium docusate was formed via the Bligh-Dyer method.

### Example 15: Gentamicin-HIP formulation in ICell.

**Aim:** To show the solubility of different gentamicin-HIP complexes in ICell.

### Materials and methods:

*Gentamicin-HIP complexation:* Gentamicin sulphate was complexed with SDS and sodium 1-decane sulphonate as described in example 14 (organic free-solvent method). After removing the supernatant, the complex was dissolved in methanol, transferred to a new glass vial and the solvent was dried under a gentle nitrogen flow. Additionally, gentamicin sulphate was complexed with sodium docusate as described in example 14 (Bligh-Dyer method). The chloroform phase was isolated, transferred to a new vial and subsequently chloroform was dried under a gentle flow of nitrogen. *ICell formulation:* A SuBen:GTH:DMSO (60:20:20) ICell was prepared as described in example 4. ICell was proportionally added to a vial containing native gentamicin sulphate to achieve a final gentamicin concentration of 20 mg/g. Gentamicin-HIP complexes were pre-dissolved in DMSO (corresponding to 5% weight of the final formulation) followed by the proportional addition of ICell to obtain a final gentamicin concentration of 20 mg/g.

### Results and discussion:

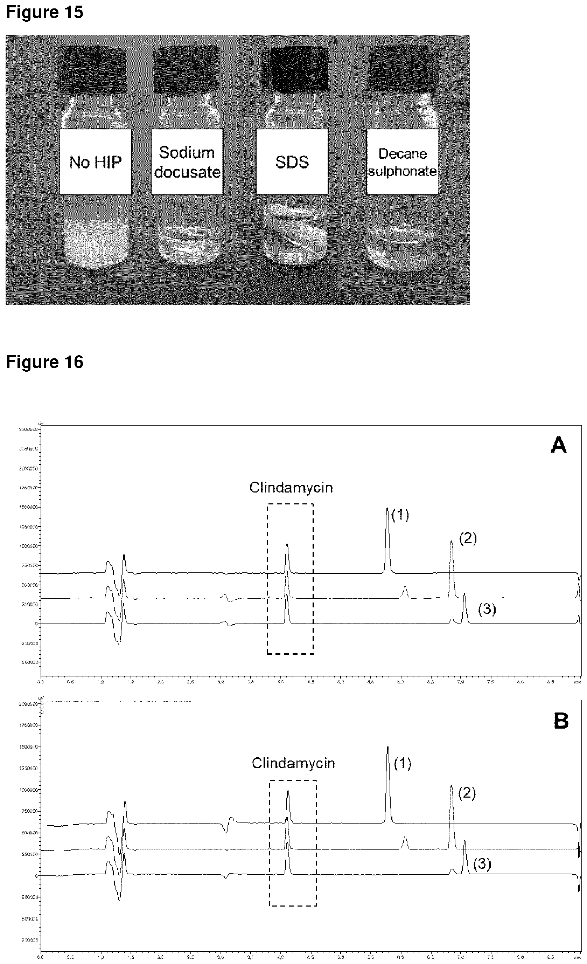

The hydrophobic complexes of gentamicin with SDS, sodium 1-decane sulphonate and sodium docusate were easily solubilized in a SuBen:GTH:DMSO (60:20:20) ICell (Fig. 15). The resulting formulations were clear, translucent and free of particles. In contrast, native gentamicin was insoluble in the ICell, in which a cloudy solution with precipitates was observed. Thus, HIP complexation of gentamicin noticeably improved the solubility of gentamicin in the ICell.

**Figure 15****. Gentamicin-HIP complexes in ICell.** (From left to right) Native gentamicin (no HIP complex) and gentamicin complexed with sodium docusate, SDS and sodium 1-decane sulphonate formulated in SuBen:GTH:DMSO (60:20:20) ICells. In all formulations, the gentamicin concentration was 20 mg/g.

### Conclusion:

The solubility of gentamicin in ICell was successfully improved by forming hydrophobic complexes with sodium docusate, SDS and sodium 1-decane sulphonate as co-ions.

### Example 16: HIP complexation of clindamycin

**Aim:** To form a HIP complex of clindamycin with different co-ions (C2DA, linoleic acid and sodium oleate).

### Materials and methods:

*HIP complexation via Bligh-Dyer method:* Clindamycin monohydrate HCI was complexed with C2DA, linoleic acid and sodium oleate as co-ions according to method 2 (example 2). Clindamycin (5 mg/mL) was dissolved in phosphate buffer pH 6, whereas the co-ions were dissolved in methanol in a 1:2 molar ratio (co-ion:clindamycin) to achieve a 1:1 charge ratio. Moreover, chloroform and methanol were mixed before the addition of the aqueous clindamycin solution. For each co-ion, the chloroform phase was isolated and transferred to new glass vials. 0.5 mL of the organic phase were taken and chloroform was dried under a gentle flow of nitrogen. The dried complex was re-suspended in methanol and analyzed by UHPLC to determine the amount of clindamycin.

*HIP complexation via modified Bligh*-*Dyer method:* Clindamycin monohydrate HCI was complexed with C2DA, linoleic acid and sodium oleate as co-ions according to method 2 (example 2) with the following modifications. Clindamycin (5 mg/mL) was dissolved in phosphate buffer pH 6, whereas the co-ions were dissolved in methanol in a 1:2 molar ratio (co-ion:clindamycin) to achieve a 1:1 charge ratio. 4 mL of antibiotic solution and 8 mL of the corresponding co-ion solution (methanol) were mixed and gently shaken for 10 min. Next, 4 mL of chloroform were added and the resulting monophase was shaken at 400 rpm at room temperature for 30 min. 4 mL of chloroform followed by 4 mL of water were added to form a biphasic system. Isolation and analysis of the dried complex was done as described beforehand for the Bligh-Dyer method. *UHPLC method*: Samples were analyzed on a Shimadzu Nexera-X with a PDA detector. Samples were injected (5 µL) onto a Waters Terra XBridge BEH C8 column (2.5 µm, 4.6x75mm, temperature 25 °C) at a flow rate of 1 mL/min. The solvent system consisted of mobile phase A (5% MeCN, 0.1% TFA in water) and mobile phase B (0.1% TFA in MeCN). The gradient was 0% B for 1 min, 0 to 100% B in 5 min, 100% B for 4 min, 100% B to 0% B in 0.5 min, 0% B for 1 min. UV detection of clindamycin was done at 202 nm.

### Results and discussion:

After isolating the organic phase from each complexation method, the amount of clindamycin was determined by HPLC. As exemplified in Fig. 16, clindamycin was detected in the organic phase of all the experiments, indicating that clindamycin had been successfully complexed with the corresponding co-ion. Additionally, the clindamycin complexation efficacy for each co-ion and each complexation method was calculated. In average, all tested co-ions showed around 70% of complexation efficacy (Table 6).

**Figure 16****: Identification of clindamycin following HIP complexation.** Individual chromatograms of the organic phase from the HIP complexation of clindamycin with C2DA, linoleic acid and sodium oleate via (A) the Bligh-Dyer method and (B) the modified Bligh-Dyer method.

**Table 6: HIP complexation efficacy of clindamycin. Percentage of clindamycin complexed with C2DA, linoleic acid and sodium oleate following complexation via Bligh-Dyer method or the modified Bligh-Dyer method.**

| **Co-ion** | **Complexation efficacy** | |
|---|---|---|
| | *Bligh-Dyer method* | *Modified Bligh-Dyer method* |
| C2DA | 80% | 72% |
| Linoleic acid | 64% | 80% |
| Sodium oleate | 72% | 72% |

### Conclusion:

HIP complexes of clindamycin with C2DA, linoleic acid and sodium oleate were successfully formed via the Bligh-Dyer method and the modified Bligh-Dyer method.

### Example 17: Clindamycin-HIP formulation in ICell.

**Aim:** To show the solubility of different clindamycin-HIP complexes in ICell.

### Materials and methods:

*Clindamycin-HIP complexation:* Clindamycin monohydrate HCI was complexed with C2DA, linoleic acid and sodium oleate as described in example 16 (Bligh-Dyer method). The chloroform phase was isolated, transferred to a new vial and subsequently chloroform was dried under a gentle flow of nitrogen.

*ICell formulation:* A SuBen:GTH:EtOH (60:20:20) ICell was prepared as described in example 4. ICell was proportionally added to a vial containing native clindamycin monohydrate HCI to achieve a final clindamycin concentration of 20 mg/g. Clindamycin-HIP complexes were pre-dissolved in EtOH (corresponding to 5% weight of the final formulation) followed by the proportional addition of ICell to obtain a final clindamycin concentration of 20 mg/g.

### Results and discussion:

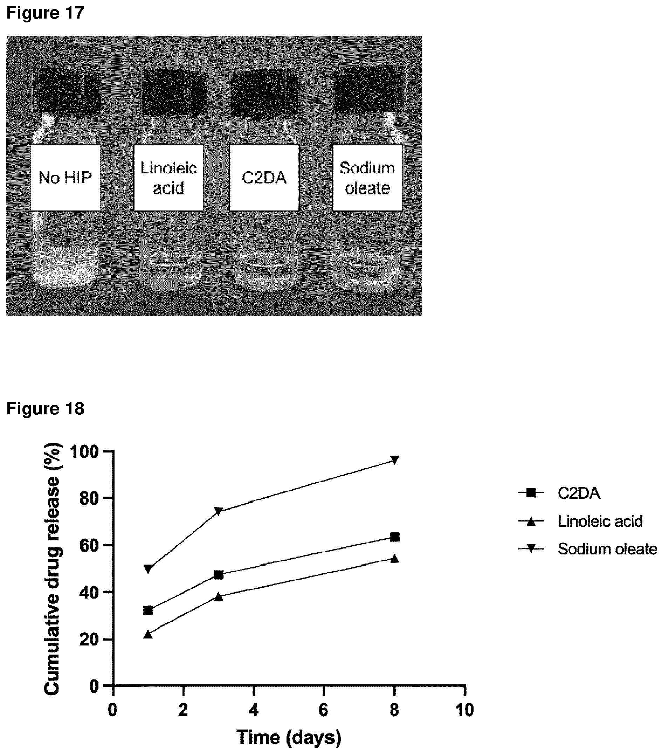

The hydrophobic complexes of clindamycin with C2DA, linoleic acid and sodium oleate were easily solubilized in a SuBen:GTH:EtOH (60:20:20) ICell (Fig. 17). The resulting formulations were clear, translucent and free of particles. Contrastingly, native clindamycin was insoluble in the ICell, in which a cloudy solution was obtained. Thus, HIP complexation of clindamycin greatly improved the solubility of clindamycin in the ICell.

**Figure 17****. Clindamycin-HIP complexes in ICell.** (From left to right) Native clindamycin (no HIP complex) and clindamycin complexed with linoleic acid, C2DA and sodium oleate formulated in SuBen:GTH:EtOH (60:20:20) ICells. In all formulations, the clindamycin concentration was 20 mg/g.

### Conclusion:

The solubility of clindamycin in ICell was successfully improved by forming hydrophobic complexes with linoleic acid, C2DA and sodium oleate as co-ions.

### Example 18: In vitro release of clindamycin-HIP complexes

**Aim:** To evaluate the in vitro release kinetics of different clindamycin-HIP complexes from ICell.

### Materials and methods:

*Clindamycin-HIP complexation:* Clindamycin monohydrate HCI was complexed with C2DA, linoleic acid and sodium oleate as described in example 16 (Bligh-Dyer method). The chloroform phase was isolated, transferred to a new vial and subsequently chloroform was dried under a gentle flow of nitrogen.

*ICell formulation:* A SuBen:GTH:EtOH (60:20:20) ICell was prepared as described in example 4. Clindamycin-HIP complexes were pre-dissolved in EtOH (corresponding to 5% weight of the final formulation) followed by the proportional addition of ICell to obtain a final clindamycin concentration of 20 mg/g.

*In vitro release:* 50 µL of each ICell were individually injected in 8-mL glass vials containing 2 mL PBS, for which the exact amount of injected ICell was weighed. The vials were incubated at 37 °C and at each sampling point 1 mL of PBS was taken. 1 mL of fresh buffer was added immediately to the vial to replace the taken sample. The samples were then analyzed by HPLC and the concentration of drug in the release media was calculated.

*UHPLC method:* HPLC analyses were done using a Shimadzu Nexera-X UHPLC instrument with a PDA detector. The samples were injected (40 µL) onto a Waters Terra BEH C8 column (2.5µm, 4.6x75mm, temperature 25°C) at a flow rate of 0.8 mL/min. The solvent system consisted of mobile phase A (5% MeCN, 0.1% TFA in water) and mobile phase B (0.1% TFA in MeCN). Chromatographic separation was achieved using a gradient of 0 to 100% phase B in 5 min followed by 2.5 min at 100% B. UV detection at 210 nm was used to identify clindamycin.

### Results and discussion:

Overall, the release of clindamycin from the ICell was higher when clindamycin was formulated as a HIP complex. As displayed in Fig. 18, after 8 days non-complexed clindamycin showed the slowest release (44%), followed by clindamycin complexed with linoleic acid (54%), C2DA (64%) and sodium oleate (96%).

**Figure 18****. In vitro release profiles of clindamycin-HIP complexes.** In vitro release profile of clindamycin and different clindamycin-HIP complexes from SuBen:GTH:EtOH (60:20:20) ICells. The clindamycin concentration in all formulations was 20 mg/g. Data points are presented as mean (n=2).

### Conclusion:

The release kinetics of clindamycin were modified by using different co-ions for HIP complexation.

### Example 19: HIP complexation of gentamicin

**Aim:** To complex gentamicin with a hydrophobic co-ion

### Materials & Methods

### The Modified Bligh-Dryer Method (Wibel et al., 2020)

Gentamicin-SO₄ was dissolved in an aqueous buffer (10 mM sodium acetate, 10 mM potassium chloride, 10 mM calcium chloride at pH 5.0). Then, a 1:5 molar ratio of sodium docusate was dissolved in methanol of twice the volume as gentamicin in buffer, after which the same volume of chloroform as gentamicin in buffer was added. For example, if a volume of 10 ml gentamicin in buffer is used, then 10 ml of docusate in methanol would be added, and 5 ml of chloroform. The monophasic solution was shaken and stirred at 600 rpm with a magnet stirrer at 37 °C for 30 minutes. Then, 1 volume of aqueous buffer and chloroform was added, in comparison to the used volume of gentamicin in buffer, and the mixture was shaken and left at room temperature for 30 minutes, until it became biphasic. For instance, if 5 ml of gentamicin in buffer was used in the first step, then 5 ml of aqueous buffer and 5 ml of chloroform is to be added in this second step. The organic- and aqueous phases were then separated, and the organic phase was evaporated in a fumehood using nitrogen flow. The resulting product was dissolved in dimethyl sulfoxide.

### Results & Discussion

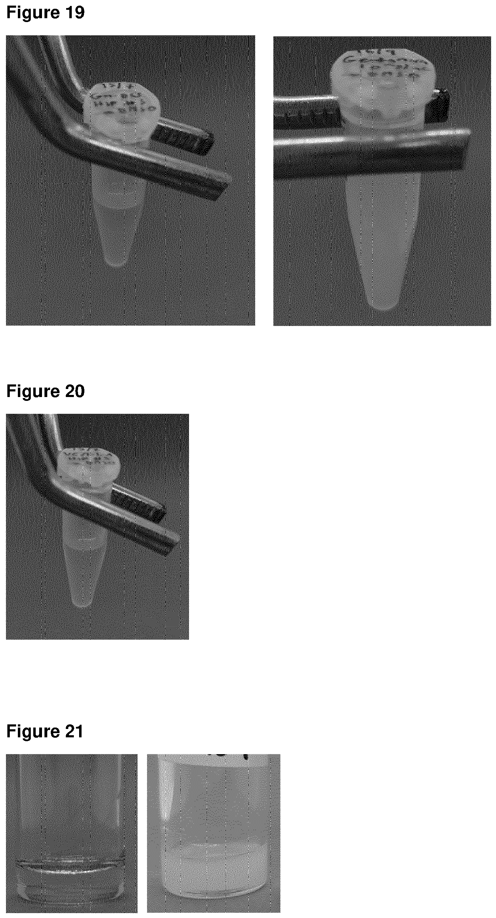

Following the Modified Bligh-Dryer Method, a precipitate was formed which was dissolvable in dimethyl sulfoxide, indicating that the hydrophobicity had increased, as gentamicin sulphate by itself is otherwise immiscible with dimethyl sulfoxide (Figure 19).

**Figure 19****.** Left-most picture: Dissolved gentamicin-docusate in DMSO following HIP complexation according to Example 19. The DMSO solution was transparent, indicating that the resulting product was dissolvable in DMSO. Right-most picture: Gentamicin sulphate by itself is not dissolvable in DMSO, evaluated by the cloudy appearance of the solution. This indicates that indeed the complexation must have led to a new compound, which is dissolvable in DMSO.

### Conclusion

A HIP complex comprising gentamicin and docusate was successfully formed.

### References

Wibel, R. et al. (2020) 'Hydrophobic ion pairing (HIP) of (poly)peptide drugs: Benefits and drawbacks of different preparation methods', European Journal of Pharmaceutics and Biopharmaceutics, 151, pp. 73-80.
doi:10.1016/j.ejpb.2020.04.004.

### Example 20: HIP complexation of vancomycin

**Aim:** To form a HIP complex of vancomycin and a hydrophobic counterion

### Materials & Methods

### The Modified Bligh-Dryer Method (Wibel et al., 2020)

Vancomycin-HCl was dissolved in an aqueous buffer (10 mM sodium acetate, 10 mM potassium chloride, 10 mM calcium chloride at pH 5.0). Then, a 1:1 molar ratio of linoleic acid was added to methanol in twice the volume of vancomycin in buffer, after which chloroform was added in the same volume as gentamicin in buffer. For example, if a volume of 5 ml vancomycin in buffer is used, then 10 ml of linoleic acid in methanol would be added, and 5 ml of chloroform. The monophasic solution was shaken and stirred at 600 rpm with a magnet stirrer at 37 °C for 30 minutes. Then, 1 volume of aqueous buffer and chloroform was added, in comparison to the used volume of vancomycin in buffer, and the mixture was shaken and left at room temperature for 30 minutes, until it became biphasic. For instance, if 5 ml of vancomycin in buffer was used in the first step, then 5 ml of aqueous buffer and 5 ml of chloroform is to be added in this second step. The organic- and aqueous phases were then separated, and the organic phase was evaporated in a fumehood using nitrogen flow. The resulting product was dissolved in dimethyl sulfoxide.

### Results & Discussion

Following the Modified Bligh-Dryer Method, a precipitate was formed which was dissolvable in dimethyl sulfoxide (Figure 20).

**Figure 20****. Dissolved vancomycin-linoleic acid in DMSO following HIP complexation.** The DMSO solution was transparent, indicating that the resulting HIP product was dissolvable in DMSO, which the corresponding amounts of gentamicin-sulphate is not.

### Conclusion

A HIP complex was successfully formed by Vancomycin and linoleic acid.

### References

Wibel, R. et al. (2020) 'Hydrophobic ion pairing (HIP) of (poly)peptide drugs: Benefits and drawbacks of different preparation methods', European Journal of Pharmaceutics and Biopharmaceutics, 151, pp. 73-80.
doi:10.1016/j.ejpb.2020.04.004.

### Example 21: Formulation of Gentamicin-Docusate complexes in ICells

**Aim:** show enhanced solubility of Gentamicin HIP complex in ICell compared to solubility of the native API.

### Materials & Methods

### ICell formulation

In this experiment, a SuBen: GTH: DMSO (60:15:25) ICell composition was prepared according to example 3 and utilized to dissolve the Gentamicin-Docusate HIP complex, which was prepared as described in Example 19.

### Results & Discussion

ICell formulation with the Gentamicin-Docusate HIP complex was successfully prepared as transparent, homogenous solution, which free gentamicin sulphate was not (Figure 21).

Figure 21. Left-most picture: The Gentamicin-Docusate HIP complex was dissolvable in a SuBen:GTH:DMSO (60:15:25) ICell. Right-most picture: Gentamicin sulphate by itself is not dissolvable in ICells as seen by the cloudy appearance of the gel.

### Conclusion

Gentamicin-Docusate HIP complex prepared by the Modified Bligh-Dryer Method described in Example 19 was successfully prepared as transparent, homogenous solution, in a ICell. A Gentamicin HIP complex with increased hydrophobicity was formulated in ICell.

### Example 22: Formulation of Vancomycin-Linoleic Acid complexes in ICells

**Aim:** show enhanced solubility of Vancomycin HIP complex in ICell compared to solubility of the native API.

### Materials & Methods

### ICell formulation

In this experiment, a SuBen:GTH:DMSO (60:15:25) ICell composition prepared according to example 3 was utilized to dissolve the Vancomycin-Linoleic Acid HIP complex.

### Results & Discussion

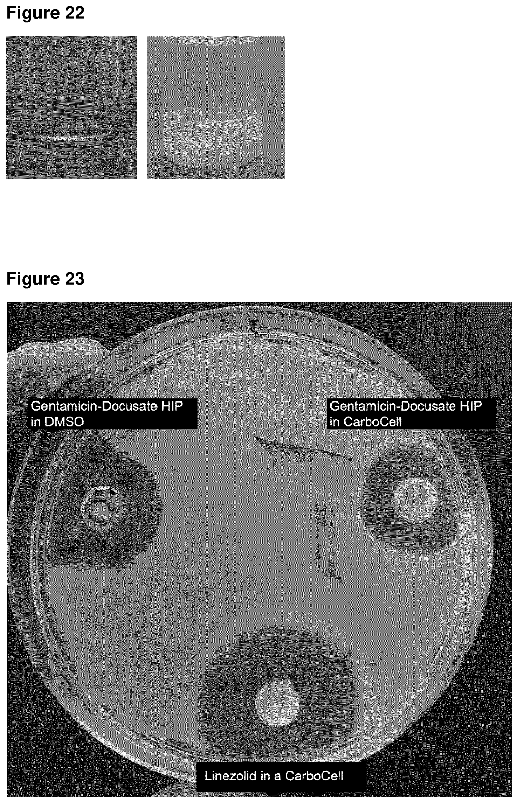

ICell formulation with the Vancomycin-Linoleic Acid HIP complex was successfully prepared as clear, homogenous solution, which vancomycin hydrochloride by itself is not (Figure 22).

**Figure 22**. Left-most picture: The vancomycin-linoleic acid HIP complex was dissolvable in a SuBen:GTH:DMSO (60:15:25) ICell. Right-most picture: Vancomycin hydrochloride by itself is not dissolvable in the ICells, as seen by the cloudy appearance of the gel.

### Conclusion

Vancomycin-Linoleic Acid HIP complex prepared by the Modified Bligh-Dryer Method described in Example 20 was successfully prepared as transparent, homogenous solution in a ICell. A Vancomycin HIP complex with increased hydrophobicity was formulated in ICell.

### Example 23: In vitro efficacy evaluation of gentamicin-docusate HIP complexes

**Aim:** The aim is to demonstrate potency of the gentamicin HIP complex against *Staphylococcus aureus* bacteria, also after release from a ICell

### Materials & Methods

### Microbiological assay, "Disc Diffusion Assay"

A 1.5%-agar LB plate was plated out with 100 µl 0.4 OD₆₀₀ S. *aureus* strain S54F9; an isolate from porcine osteomyelitis. Then, 3 wells were carved out with the use of the broad side of an autoclaved 1 ml pipette tip. In one well, 7.5 mg gentamicin-docusate HIP complex was injected in 90 µl autoclaved phosphate buffered saline water. In a second well 50 µl 15 mg/g Linezolid was injected into phosphate buffered saline water, dissolved in a ICell consisting of SuBen:GTO:EtOH (60:25:15), and ICell. In a third well, gentamicin-docusate was injected as 50 µl 20 mg/g, in a ICell consisting of SuBen:GTH:DMSO (55:25:20).

This setup was repeated for three experimental replicates. Each plate was incubated for 24 hours at 37 °C, before inspection.

### Results & Discussion

The gentamicin-docusate complex had retained its biological antimicrobial activity throughout the complexation procedure; exerting its effect either complexed with docusate or by dissociating the complex followed by diffusion of pure gentamicin into the agar phase (Figure 23).

**Figure 23****. Testing the *in vitro* efficacy of gentamicin-docusate HIP complexes.** The experiment was repeated three times, and this is a representative picture of one of such experiments. Three wells had either gentamicin-docusate HIP in DMSO, in a SuBen:GTO:EtOH (60:25:15), or linezolid (15 mg/ml) in a SuBen:GTO:EtOH (60:25:15) ICell injected. Each well had a clearing zone of S. *aureus* bacteria, as evaluated by the appearance of a transparent circle around the hole, or well, in the plate, caused by the compound diffusing out of the well and killing the bacteria. It is clear from this Figure that the gentamicin-docusate HIP complex generated by the Modified Bligh-Dryer Method (see Example 2) retained its antimicrobial efficacy after complexation, and is also released from ICells over time, after which it also retained its antimicrobial efficacy.

### Conclusion

The gentamicin-docusate generated in Example 19 can be released from ICells and whether released or as a free complex, the complex or dissociated complex retains the antimicrobial activity of the gentamicin antibiotic used to generate the complex, also after being released from a ICell.

### Example 24: In vitro efficacy evaluation of vancomycin-linoleic acid HIP complexes

**Aim:** The aim is to demonstrate potency of the vancomycin HIP complex against *Staphylococcus aureus* bacteria

### Materials & Methods

### Microbiological assay, "Disc Diffusion Assay"

A 1.5%-agar LB plate was plated out with 100 µl 0.4 OD₆₀₀ S. *aureus* strain S54F9; an isolate from porcine osteomyelitis. Then, 3 wells were carved out with the use of the broad side of an autoclaved 1 ml pipette tip. In one well, 0.5 mg linoleic acid was injected as 90 µl 0.22 µm-sterile-filtered dimethyl sulfoxide. In a second well, 90 µl autoclaved phosphate-buffered saline with 0.5 mg vancomycin hydrochloride was injected. In a third well, 45 µl vancomycin-linoleic acid HIP complex was added to 45 µl autoclaved, phosphate-buffered saline. The plate was incubated for 24 hours at 37 °C, before inspection.

### Results & Discussion

The vancomycin-linoleic acid complex had retained its biological antimicrobial activity throughout the complexation procedure; vancomycin exerting its effect either complexed with linoleic acid or by dissociating the complex followed by diffusion of pure vancomycin into the agar phase. Linoleic acid by itself also has an antimicrobial effect, but to an extent lesser than vancomycin, indicating that it is the vancomycin from the complex which acts from the vancomycin-linoleic acid HIP complex.

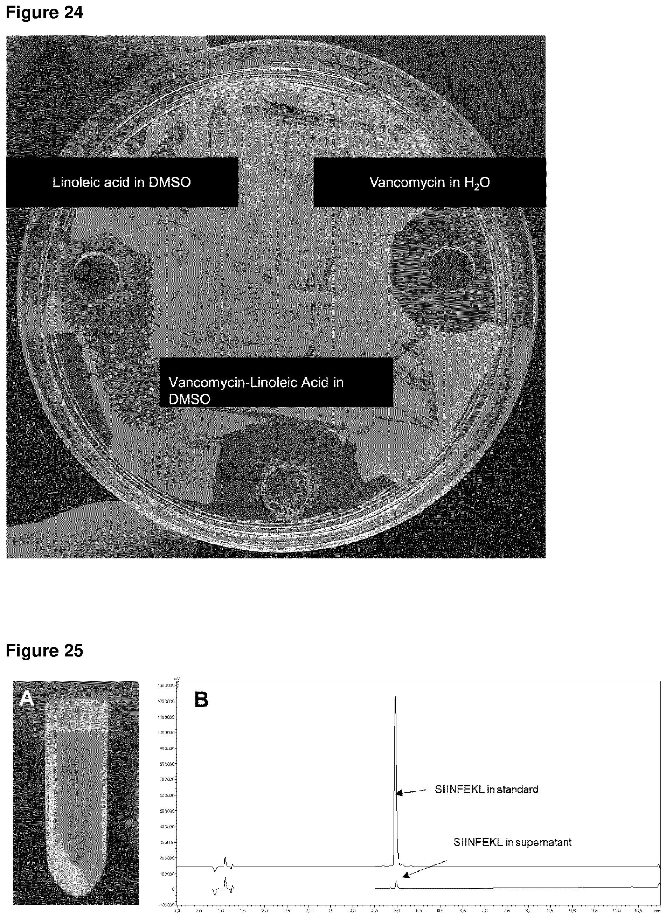

**Figure 24****. Testing the *in vitro* efficacy of vancomycin-linoleic HIP complexes.** The three circles show clearance of S. *aureus* bacteria, as evaluated by the appearance of a transparent circle around the hole, or well, in the plate, caused by the compound diffusing out of the well. The left-most well was added linoleic acid in DMSO. In the middle well, the vancomycin-linoleic acid HIP complex was injected into phosphate-buffered saline. In the right well, free vancomycin hydrochloride was added in sterile water. It is clear from this Figure that the vancomycin-linoleic acid HIP complex generated by the Modified Bligh-Dryer Method (see Example 20) retained its antimicrobial efficacy after complexation, to an extent comparable to pure vancomycin.

### Conclusion

The vancomycin-linoleic acid HIP complex generated in Example 20 can exert the antimicrobial effect of vancomycin whether as complexed with linoleic acid or dissociated from the linoleic acid complex.

### Example 25: HIP complexation of SIINFEKL

**Aim:** To form a HIP complex of the SIINFEKL peptide with SDS as a co-ion.

### Materials and methods:

*HIP complexation via organic-solvent free method:* SIINFEKL peptide was complexed with SDS following method 1 described in example 2. SIINFEKL (1.5 mg/mL) was dissolved in 0.01 M HCI. SDS was dissolved in 0.01 M HCI in a 2:1 molar ratio (co-ion:peptide), which equals a 1:1 charge ratio. The SIINFEKL and co-ion solution were mixed in equal volumes, followed by shaking and centrifugation steps as described in method 1 of example 2. The supernatant was removed the content of SIINFEKL was evaluated by HPLC. After removing the supernatant, the complex was dissolved in methanol, transferred to a new glass vial and the solvent was dried under a gentle nitrogen flow.

*HPLC method:* Samples were analyzed on a Shimadzu Nexera-X with PDA. Samples were injected (40 µL) onto a Waters Terra XBridge BEH C8 column (2.5 µm, 4.6x150mm, temperature 25 °C) at a flow rate of 1 mL/min. The solvent system consisted of mobile phase A (5% MeCN, 0.1% TFA in water) and mobile phase B (0.1% TFA in MeCN). The gradient was 0% B for 1.5 min, 0 to 100% B in 7 min, 100% B for 1.5 min, 100% B to 0% B in 0.5 min, 0% B for 1.5 min. SIINFEKL was quantified by UV at 220 nm.

### Results and discussion:

When conducting the HIP complexation, a cloudy solution was immediately formed after mixing the peptide and the co-ion solution. Subsequently, a white precipitate was obtained following the centrifugation step, which indicates the formation of a HIP complex (Fig. 25A). Such precipitate was soluble in methanol, whereas native SIINFEKL is insoluble in such solvent. Moreover, only 2% of the total SIINFEKL was detected in the resulting supernatant (Fig. 25B), indicating that 98% of SIINFEKL was complexed with SDS (n=3).

**Figure 25****. HIP complexation of SIINFEKL.** (A) Precipitate resulting from the organic solvent-free method indicating the formation of a HIP complex between SIINFEKL and SDS. (B) Comparison between the chromatogram of a SIINFEKL standard and the supernatant from the complexation of SIINFEKL with SDS.

### Conclusion:

A HIP complex of SIINFEKL with SDS was successfully formed via the organic-solvent free method with a 98% complexation efficacy.

### Example 26: HIP complexation of abaloparatide

**Aim:** To form a HIP complex of the abaloparatide peptide with different co-ions (SDS and 1-decane sulphonate).

### Materials and methods:

*HIP complexation via organic-solvent free method:* Abaloparatide was complexed with SDS and sodium 1-decane sulphonate following method 1 described in example 2. Abaloparatide (1 mg/mL) was dissolved in 0.01 M HCl. SDS and 1-decane sulphonate were dissolved in 0.01 M HCI in a 11:1 molar ratio (co-ion:peptide), which equals a 1:1 charge ratio. Additionally, the co-ions were dissolved in water in a 5:1 molar ratio (co-ion:peptide to obtain a 0.45:1 charge ratio. The peptide and each co-ion solution were mixed in equal volumes, followed by shaking and centrifugation steps as described in method 1 of example 2. The supernatant was removed and the content of abaloparatide was evaluated by HPLC.

*HPLC method:* Samples were analyzed on a Shimadzu Nexera-i with PDA. Samples were injected (30 µL) onto a Waters Terra XBridge BEH C8 column (5 µm, 4.6x150mm, temperature 25 °C) at a flow rate of 1 mL/min. The solvent system consisted of mobile phase A (5% MeCN, 0.1% TFA in water) and mobile phase B (0.1% TFA in MeCN). The gradient was 0% B for 2 min, 0 to 100% B in 10 min, 100% B for 4 min, 100% B to 0% B in 0.5 min, 0% B for 2 min. Abaloparatide was quantified by UV detection at 220 nm.

### Results and discussion:

As part of the complexation method, a cloudy solution was immediately formed after mixing the abaloparatide and the co-ion solutions at a 1:1 (co-ion:peptide) charge ratio. At a charge ratio of 0.45:1, a cloudy solution was only observed when using SDS as co-ion, while the solution remained clear when using 1-decane sulphonate as co-ion. The concentration of abaloparatide in the resulting supernatant (i.e., non-complexed peptide) was measured by HPLC and the complexation efficacy was calculated. As shown in Table 7, at a charge ratio of 1:1, both co-ions displayed a complexation efficacy of 90-100%. When reducing the charge ratio to 0.45:1, the complexation efficacy with SDS decreased to 42%, while abaloparatide was not complexed with 1-decane sulphonate as the totality of the peptide was detected in the supernatant. The higher hydrophobicity of SDS compared to 1-decane sulphonate, as indicated by their log P values (2.83 and 1.86, respectively), might be beneficial for a more efficient complexation.

**Table 7: HIP complexation efficacy of abaloparatide. Average complexation efficacy of abaloparatide with SDS and 1-decane sulphonate at different charge ratios following HIP complexation via the organic solvent-free method (n=2).**

| **Charge ratio (co-ion: abaloparatide)** | **Complexation efficacy** | |
|---|---|---|
| | *SDS* | *1-decane sulphonate* |
| 1:1 | 100 % | 90% |
| 0.45:1 | 42% | 0% |

### Conclusion:

HIP complexes of abaloparatide with SDS and 1-decane sulphonate were successfully formed via the organic solvent-free method, with a 90-100% complexation efficacy at a charge ratio of 1:1.

### Example 27: Abaloparatide-HIP formulation in ICell.

**Aim:** To show the solubility of different abaloparatide-HIP complexes in ICell.

### Materials and methods:

Abaloparatide-HIP *complexation:* Abaloparatide (Tymlos) was complexed with SDS and sodium 1-decane sulphonate as described in example 2 (organic free-solvent method). After removing the supernatant, the complex was dissolved in methanol, transferred to a new glass vial and the solvent was dried under a gentle nitrogen flow. *ICell formulation:* A SuBen:GTH:DMSO (60:20:20) ICell was prepared as described in example 4. ICell was proportionally added to a vial containing Abaloparatide in its native form to achieve a final Abaloparatide concentration of 1 mg/g. Abaloparatide-HIP complexes were pre-dissolved in DMSO (corresponding to 5% weight of the final formulation), followed by the proportional addition of ICell to obtain a final Abaloparatide concentration of 1 mg/g.

### Results and discussion:

The hydrophobic complexes of Abaloparatide with SDS and sodium 1-decane sulphonate were easily solubilized in a SuBen:GTH:DMSO (60:20:20) ICell (Fig. 22). The resulting formulations were clear, translucent and free of particles. In contrast, at an equal concentration, Abaloparatide in its native form (non-complexed) was insoluble in the ICell, in which a cloudy solution was observed. Thus, HIP complexation of Abaloparatide noticeably improved the solubility of Abaloparatide in the ICell.

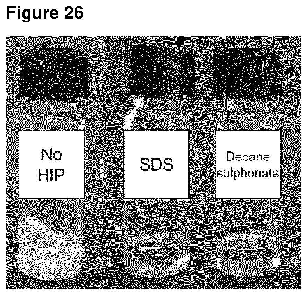

**Figure 26****. Abaloparatide-HIP complexes in ICell.** (From left to right) Native Abaloparatide (no HIP complex) and Abaloparatide complexed with SDS and and sodium 1-decane sulphonate formulated in SuBen:GTH:DMSO (60:20:20) ICells. In all formulations, the abaloparatide concentration was 1 mg/g.

### Conclusion:

The solubility of abaloparatide in ICell was successfully improved by forming hydrophobic complexes with SDS and sodium 1-decane sulphonate as co-ions.

### Clauses - a second aspect of the present invention

1. A composition forming hydrophobic ion pairing (HIP) comprising:
   a. a pharmaceutical agent with a zwitterionic, cationic or anionic charge;
   b. a counterion being a hydrophobic or amphiphilic acid or base, or salt thereof;
   c. a non-water soluble carbohydrate;
   d. a non-aqueous solvent

   wherein at least 30% of the non-water soluble carbohydrates are carbohydrates selected from derivatives of disaccharides or trisaccharides selected from sucrose, lactose, maltose, trehalose, raffinose, or derivatives of disaccharides or trisaccharides, or mixtures thereof;
   wherein the composition is a liquid before administration into the human or animal body and increases in viscosity by more than 10,000 centipoise (cP) after administration to transform into a ICell;
   and wherein the hydrophobic or amphiphilic acid or base or salt thereof enhances the solubility of the pharmaceutical agent in the ICell by forming a hydrophobic ion-pair or multiple hydrophobic ion-pairs with the pharmaceutical agent.
2. The composition according to claim 1, wherein the hydrophobic or amphiphilic acid or base or salt thereof reduces or enhances the release rate of the pharmaceutical agent from the ICell compared to a ICell composition without the hydrophobic or amphiphilic acid or base or salt thereof.
3. The composition according to claim 1 or 2, wherein the composition further comprises a co-solvent selected from one or more lipids, monoglycerides, diglycerides or triglycerides.
4. The composition according to any of claims 1-3, wherein the composition further comprises one or more polymers or copolymers.
5. The composition according to any of claims 1-4, wherein the pharmaceutical agent is a small molecule drug, peptide, or protein.
6. The composition according to claim 5, wherein the small molecule drug is one or more anticancer agents, immunotherapeutic, chemotherapeutics, antimicrobials (antibiotics, antivirals, antifungals, antiprotozoals, and antihelminthics), antiinflammatory agents, anticoagulant, antidepressant, antiepileptic, antipsychotic, sedatives, antidiabetic, cardiovascular, and analgesic agents; wherein the peptide is one or more therapeutic peptides, agonistic and antagonistic receptor ligands, peptides with regulatory or enzymatic activity, hormones, peptides with special targeting activities, vaccines, antigens, therapeutic peptides, diagnostic peptides, steroids; wherein the protein is one or more immunotherapeutic agonists and antagonists, growth factors, cytokines, chemokines, hormones, glycoprotein hormones and antibodies, affibodies, peptide and nanobodies.
7. The composition according to any of claims 1-6, wherein the pharmaceutical agent and the hydrophobic or amphiphilic acid or base, or salt thereof have different charge ratios.
8. The composition according to any of claims 1-7, wherein the hydrophobic or amphiphilic acid or base, or salt thereof is selected from any of the class of ionic detergents, ionic lipids and lysolipids, fatty acids and ionic polymers.
9. The composition according to any of claims 1-8, wherein the hydrophobic or amphiphilic acid or base, or salt thereof is selected from one or multiple ionizable groups belonging to the group of one or more amines (primary, secondary, tertiary or quaternary), alcohols, carboxylic acids, thiols, sulphonic, sulphenic or sulfinic acids, tetraalkyl phosphonium, phosphonic, phosphenic, or phosphinic acids, and conjugated systems with delocalized charges.
10. The composition according to any of claims 1-9, wherein the composition further comprises an imaging agent.
11. A method for forming one or more hydrophobic ion pairing (HIP) complexes, wherein the method comprises the steps of:
   a) dissolving a pharmaceutical agent in a first liquid;
   b) dissolving a hydrophobic counterion in a second liquid;
   c) mixing the solution of step a) and b);
   d) allowing the HIP formation;
   e) resuspending the HIPs in an organic solvent, oil or ICell composition; and
   f) removal of at least aqueous solvent/phase.
12. The method according to claim 11, wherein the HIP is formulated in an ICell and/or as a powder.
13. The method according to claim 11 or 12, wherein the first liquid is water; wherein the hydrophobic counterion is water soluble and the second liquid is water; or wherein the hydrophobic counterion is weakly water-soluble and the second liquid is chosen from methanol, ethanol, chloroform, DCM, DMSO, PC, iso-propanol, BnOH, or an organic solvent being mixable with water.
14. The method according to any of claims 11-13, wherein the method has an additional step a') adjusting the pH of the solution such that the charge of the imaging agent or pharmaceutical agent and the hydrophobic counterion is non-zero and have opposing charge, wherein a') is performed between step a) and b).
15. The method according to any of claims 11-14, wherein the mixing of step c) is performed in the presence of chloroform or DCM and wherein the formation of step d) is performed through HIP partition into an organic phase.

## Claims

1. A composition forming a hydrophobic ion par (HIP), comprising:
a. an imaging agent with zwitterionic, cationic or anionic charge;
b. a counterion being a hydrophobic or amphiphilic acid or base, or salt thereof;
c. a non-water soluble carbohydrate;
d. a non-aqueous solvent;
wherein at least 50% of the non-water soluble carbohydrates are carbohydrates selected from derivatives of disaccharides or trisaccharides selected from sucrose, lactose, maltose, trehalose, raffinose, or derivatives of disaccharides or trisaccharides, or mixtures thereof;
wherein the composition is a liquid before administration into the human or animal body and increases in viscosity by more than 10,000 centipoise (cP) after administration to transform into a ICell;
and wherein the hydrophobic or amphiphilic acid or base or salt thereof enhances the solubility of the imaging agent in the ICell by forming a hydrophobic ion-pair or multiple hydrophobic ion-pairs with the imaging agent.

2. The composition according to claim 1, wherein the composition further comprises a co-solvent selected from one or more lipids, monoglycerides, diglycerides or triglycerides.

3. The composition according to claim 1 or 2, wherein the composition further comprises one or more polymers or copolymers.

4. The composition according to any of claims 1-3, wherein the counterion reduces or enhances the release rate of the imaging agent from the ICell compared to a ICell composition without the counterion.

5. The composition according to any of claims 1-4, wherein the imaging agent is a fluorescent imaging agent.

6. The composition according to any of claims 1-4, wherein the imaging agent is a fluorescent imaging agent or an imaging agent, which has one or more ionizable groups or moieties, preferably the ionized group is one or more amines (primary, secondary, tertiary or quaternary), alcohols, carboxylic acids, thiols, sulphonic, sulphenic or sulfinic acids, tetraalkyl phosphonium, phosphonic, phosphenic, or phosphinic acids.

7. The composition according to claim 5 or 6, wherein the fluorescent imaging agent is methylene blue (MB), indocyanine green (ICG), or wherein the imaging agent is an MRI imaging agent, preferably being Gadoteric acid, gadopentetate, gadobenate or gadoxentate.

8. The composition according to claim 5 or 6, wherein the fluorescent imaging agent is methylene blue (MB), indocyanine green (ICG) and wherein the counterion is selected from Tetraethyl ammonium bromide, Dodecylamine, Cetyl trimethyl ammonium bromide (CTAB), Tetraoctyl ammonium bromide (TOAB), N-benzyl-2-phenylethanamine (benethamine, BENA).

9. The composition according to any of claims 1-8, wherein the imaging agent and the hydrophobic or amphiphilic acid or base, or salt thereof have different charge ratios.

10. The composition according to any of claims 1-9, wherein the counterion is selected from any of the class of ionic detergents, ionic lipids and lysolipids, fatty acids and ionic polymers; or wherein the counterion is selected from any of one or multiple ionizable groups belonging to the group of amines (primary, secondary, tertiary or quaternary), alcohols, carboxylic acids, thiols, sulphonic, sulphenic or sulfinic acids, tetraalkyl phosphonium, phosphonic, phosphenic, or phosphinic acids, and conjugated systems with delocalized charges.

11. A method for forming one or more hydrophobic ion pairing (HIP) complexes, wherein the method comprises the steps of:
a) dissolving an imaging agent in a first liquid;
b) dissolving a hydrophobic counterion in a second liquid;
c) mixing the solution of step a) and b);
d) allowing the HIP formation;
e) resuspending the HIPs in an organic solvent, oil or ICell composition; and
f) removal of at least aqueous solvent/phase.

12. The method according to claim 11, wherein the HIP is formulated in an ICell and/or as a powder.

13. The method according to claim 11 or 12, wherein the first liquid is water; wherein the hydrophobic counterion is water soluble and the second liquid is water; or wherein the hydrophobic counterion is weakly water-soluble and the second liquid is chosen from methanol, ethanol, chloroform, DCM, DMSO, PC, iso-propanol, BnOH, or an organic solvent being mixable with water.

14. The method according to any of claims 10-13, wherein the method has an additional step a') adjusting the pH of the solution such that the charge of the imaging agent or pharmaceutical agent and the hydrophobic counterion is non-zero and have opposing charge, wherein a') is performed between step a) and b).

15. The method according to any of claims 11-14, wherein the mixing of step c) is performed in the presence of chloroform or DCM and wherein the formation of step d) is performed through HIP partition into an organic phase.
